# EUROPEAN PATENT APPLICATION

(11) **EP 2 426 212 A1**
(43) Date of publication of application: **07.03.2012**
(21) Application number: 11179000.2
(22) Date of filing: 15.02.2007
(51) Int. Cl.: C12Q 1/26

(54) **Method for controlling NAD(P)/NAD(P)H ratio by oxidoreductase**

(30) Priority: 15.02.2006 KR 20060014520
(62) Divisional of application: 07708979.5
(71) Applicant: Md Bioalpha Co., Ltd., 302-120 Daejeon (KR)
(72) Inventor: Park, Myung-Gyu, 446-908 Gyeonggi-do (KR); Yoo, Sang-Ku, 427-040 Gyeonggi-do (KR); Jo, In Geun, 330-210 Chungcheongnam-do (KR); Kwak, Taehwan, 449-906 Gyeonggi-do (KR)
(74) Representative: HOFFMANN EITLE

(57) **Abstract**

The present invention provides a method capable of effectively treating various diseases associated with energy excess, such as obesity, diabetes, metabolic syndromes, degenerative diseases and mitochrondrial dysfunction-related diseases, via elevation of an NAD(P)⁺ZNAD(P)H ratio by increasing an NAD(P)⁺ concentration *in vivo* or *in vitro* through use of NAD(P)H as a substrate or coenzyme by oxidoreductase such as NAD(P)H:quinine oxidoreductase (NQO1), a method of screening a drug for the same and a therapeutic drug.

<110>    MD Bio Alpha

<120>    Method for Controlling NAD(P)+/NAD(P)H Ratio by Oxidoreductase

<130>    MDB 66(P)

<140>    10-2007-0015920
<141>    2007-02-15

<150>    KR10-2006-0014520
<151>    2006-02-15

<160>    64

<170>    KopatentIn 1.71

<210>    1
<211>    20
<212>    DNA
<213>    Artificial Sequence

<220>
<223>    mPGC1-alpha (PCR primer: forward)


<400>    1
cgatgtgtcg ccttcttgct                                                    20


<210>    2
<211>    23
<212>    DNA
<213>    Artificial Sequence

<220>
<223>    mAMPK alpha1 (PCR primer: forward)


<400>    2
ttccgaagta tctctttcct gag                                                23


<210>    3
<211>    23
<212>    DNA
<213>    Artificial Sequence

<220>
<223>    mAMPk alpha2 (PCR primer: forward)


<400>    3
aggaagtgtg tgagaaattc gag                                                23


<210>    4
<211>    23
<212>    DNA
<213>    Artificial Sequence

<220>
<223>    mGLUT2 (PCR primer: forward)


<400>    4

gctgtactga gttccttcca gtt                                              23

<210>  5
<211>  23
<212>  DNA
<213>  Artificial Sequence

<220>
<223>  mHK2 (PCR primer: forward)

<400>  5
cggatattga agacgataag gac                                              23

<210>  6
<211>  23
<212>  DNA
<213>  Artificial Sequence

<220>
<223>  mACC1 (PCR primer: forward)

<400>  6
tgctgagatt gaggtaatga aga                                              23

<210>  7
<211>  22
<212>  DNA
<213>  Artificial Sequence

<220>
<223>  mACC2 (PCR primer: forward)

<400>  7
cagctgtctg tcctacagag gt                                               22

<210>  8
<211>  23
<212>  DNA
<213>  Artificial Sequence

<220>
<223>  mFAS (PCR primer: forward)

<400>  8
cctgctatca tctgacttcc tct                                              23

<210>  9
<211>  23
<212>  DNA
<213>  Artificial Sequence

<220>
<223>  mSCD1 (PCR primer: forward)

<400>  9

agtacgtctg gaggaacatc att                                                23

<210>  10
<211>  23
<212>  DNA
<213>  Artificial Sequence

<220>
<223>  mHMG-CoA synthase (PCR primer: forward)

<400>  10
gtctaatttg atgcagctgt ttg                                                23

<210>  11
<211>  23
<212>  DNA
<213>  Artificial Sequence

<220>
<223>  mLPL (PCR primer: forward)

<400>  11
aggacacttg tcatctcatt cct                                                23

<210>  12
<211>  23
<212>  DNA
<213>  Artificial Sequence

<220>
<223>  mCPT1 (PCR primer: forward)

<400>  12
ttacttcaag gtctggctct acc                                                23

<210>  13
<211>  23
<212>  DNA
<213>  Artificial Sequence

<220>
<223>  mAOX (PCR primer: forward)

<400>  13
tatggtgtcg tacttgaatg acc                                                23

<210>  14
<211>  23
<212>  DNA
<213>  Artificial Sequence

<220>
<223>  mUCP1 (PCR primer: forward)

<400>  14

gggacctaca atgcttacag agt                                                23

<210>  15
<211>  23
<212>  DNA
<213>  Artificial Sequence

<220>
<223>  mUCP2 (PCR primer: forward)

<400>  15
agcctacaga tgtggtaaag gtc                                                23

<210>  16
<211>  23
<212>  DNA
<213>  Artificial Sequence

<220>
<223>  mtTFA (PCR primer: forward)

<400>  16
ataccttcga ttttccacag aac                                                23

<210>  17
<211>  20
<212>  DNA
<213>  Artificial Sequence

<220>
<223>  mPPAR gamma (PCR primer: forward)

<400>  17
atcttaactg ccggatccac                                                    20

<210>  18
<211>  20
<212>  DNA
<213>  Artificial Sequence

<220>
<223>  mPPAR alpha (PCR primer: forward)

<400>  18
tgcagacctc aaatctctgg                                                    20

<210>  19
<211>  20
<212>  DNA
<213>  Artificial Sequence

<220>
<223>  mGLUT4 (PCR primer: forward)

<400>  19

cagcgagtga ctggaacact                                                    20

<210>    20
<211>    20
<212>    DNA
<213>    Artificial Sequence

<220>
<223>    mNRF1 (PCR primer: forward)

<400>    20
tgatggagag gtggaacaaa                                                    20

<210>    21
<211>    20
<212>    DNA
<213>    Artificial Sequence

<220>
<223>    mCOX4i1 (PCR primer: forward)

<400>    21
gactacccct tgcctgatgt                                                    20

<210>    22
<211>    20
<212>    DNA
<213>    Artificial Sequence

<220>
<223>    mCOX7ah (PCR primer: forward)

<400>    22
gctctggtcc ggtcttttag                                                    20

<210>    23
<211>    20
<212>    DNA
<213>    Artificial Sequence

<220>
<223>    mSIRT1 (PCR primer: forward)

<400>    23
agtaagcggc ttgagggtaa                                                    20

<210>    24
<211>    20
<212>    DNA
<213>    Artificial Sequence

<220>
<223>    mSIRT2 (PCR primer: forward)

<400>    24

cttgccaagg agctctatcc                                                    20

<210>    25
<211>    20
<212>    DNA
<213>    Artificial Sequence

<220>
<223>    mSIRT3 (PCR primer: forward)

<400>    25
cactacaggc ccaatgtcac                                                    20

<210>    26
<211>    20
<212>    DNA
<213>    Artificial Sequence

<220>
<223>    mSIRT4 (PCR primer: forward)

<400>    26
cggttccatc tcggttatct                                                    20

<210>    27
<211>    20
<212>    DNA
<213>    Artificial Sequence

<220>
<223>    mSIRT5 (PCR primer: forward)

<400>    27
cgattcattt cccagttgtg                                                    20

<210>    28
<211>    20
<212>    DNA
<213>    Artificial Sequence

<220>
<223>    mSIRT6 (PCR primer: forward)

<400>    28
gggagctgag agacaccatt                                                    20

<210>    29
<211>    20
<212>    DNA
<213>    Artificial Sequence

<220>
<223>    mSIRT7 (PCR primer: forward)

<400>    29

acctcctgca tccctaacag                                                    20

<210>    30
<211>    21
<212>    DNA
<213>    Artificial Sequence

<220>
<223>    ATGL (PCR primer: forward)

<400>    30
cgccttgctg agaatcacca t                                                  21

<210>    31
<211>    24
<212>    DNA
<213>    Artificial Sequence

<220>
<223>    Leptin (PCR primer: forward)

<400>    31
ggtgtgaaag aacctgagct gagg                                               24

<210>    32
<211>    24
<212>    DNA
<213>    Artificial Sequence

<220>
<223>    Adiponectin (PCR primer: forward)

<400>    32
tctcctgttc ctcttaatcc tgcc                                               24

<210>    33
<211>    18
<212>    DNA
<213>    Artificial Sequence

<220>
<223>    mPGC1-alpha (PCR primer: Reverse)

<400>    33
cgagagcgca tcctttgg                                                      18

<210>    34
<211>    23
<212>    DNA
<213>    Artificial Sequence

<220>
<223>    mAMPK alpha1 (PCR primer: Reverse)

<400>    34

acgcaaataa taggggttta caa                                              23

<210>    35
<211>    23
<212>    DNA
<213>    Artificial Sequence

<220>
<223>    mAMPk alpha2(PCR primer: Reverse)

<400>    35
ccaggtaaag ctgtaagctc att                                              23

<210>    36
<211>    23
<212>    DNA
<213>    Artificial Sequence

<220>
<223>    mGLUT2(PCR primer: Reverse)

<400>    36
gtcctgaaat tagcccacaa tac                                              23

<210>    37
<211>    23
<212>    DNA
<213>    Artificial Sequence

<220>
<223>    mHK2 (PCR primer: Reverse)

<400>    37
ttcatccttc tcttaacctc cag                                              23

<210>    38
<211>    23
<212>    DNA
<213>    Artificial Sequence

<220>
<223>    mACC1(PCR primer: Reverse)

<400>    38
atacaggact gatgtgatgt tgc                                              23

<210>    39
<211>    23
<212>    DNA
<213>    Artificial Sequence

<220>
<223>    mACC2 (PCR primer: Reverse)

<400>    39

caggtccagt ttcttgtgtt ctc 23

<210> 40
<211> 23
<212> DNA
<213> Artificial Sequence

<220>
<223> mFAS (PCR primer: Reverse)

<400> 40
agggtggttg ttagaaagat caa 23

<210> 41
<211> 23
<212> DNA
<213> Artificial Sequence

<220>
<223> mSCD1 (PCR primer: Reverse)

<400> 41
gcttgtagta cctcctctgg aac 23

<210> 42
<211> 23
<212> DNA
<213> Artificial Sequence

<220>
<223> mHMG-CoA synthase (PCR primer: Reverse)

<400> 42
aaagatcatg aagccaaaat cat 23

<210> 43
<211> 23
<212> DNA
<213> Artificial Sequence

<220>
<223> mLPL (PCR primer: Reverse)

<400> 43
agacatctac aaaatcagcg tca 23

<210> 44
<211> 23
<212> DNA
<213> Artificial Sequence

<220>
<223> mCPT1 (PCR primer: Reverse)

<400> 44

ctcctttaca gtgtccatcc tct                                                23

<210>    45
<211>    23
<212>    DNA
<213>    Artificial Sequence

<220>
<223>    mAOX (PCR primer: Reverse)

<400>    45
gggtcacatc cttaaagtca aag                                                23

<210>    46
<211>    23
<212>    DNA
<213>    Artificial Sequence

<220>
<223>    mUCP1 (PCR primer: Reverse)

<400>    46
gtacaatcca ctgtctgtct gga                                                23

<210>    47
<211>    23
<212>    DNA
<213>    Artificial Sequence

<220>
<223>    mUCP2 (PCR primer: Reverse)

<400>    47
gctcataggt gacaaacatc act                                                23

<210>    48
<211>    23
<212>    DNA
<213>    Artificial Sequence

<220>
<223>    mtTFA (PCR primer: Reverse)

<400>    48
tcatttcatt gtcgtaacga atc                                                23

<210>    49
<211>    20
<212>    DNA
<213>    Artificial Sequence

<220>
<223>    mPPAR gamma (PCR primer: Reverse)

<400>    49

tggtgatttg tccgttgtct                                                    20

<210>    50
<211>    20
<212>    DNA
<213>    Artificial Sequence

<220>
<223>    mPPAR alpha (PCR primer: Reverse)

<400>    50
tagccttggc aaattctgtg                                                    20

<210>    51
<211>    20
<212>    DNA
<213>    Artificial Sequence

<220>
<223>    mGLUT4 (PCR primer: Reverse)

<400>    51
cagcatagcc cttttccttc                                                    20

<210>    52
<211>    20
<212>    DNA
<213>    Artificial Sequence

<220>
<223>    mNRF1 (PCR primer: Reverse)

<400>    52
ggtttcccca gacaggacta                                                    20

<210>    53
<211>    20
<212>    DNA
<213>    Artificial Sequence

<220>
<223>    mCOX4i1 (PCR primer: Reverse)

<400>    53
gcagtgaagc caatgaagaa                                                    20

<210>    54
<211>    19
<212>    DNA
<213>    Artificial Sequence

<220>
<223>    mCOX7ah (PCR primer: Reverse)

<400>    54

tcacttcttg tggggggaag                                                    19

<210>    55
<211>    20
<212>    DNA
<213>    Artificial Sequence

<220>
<223>    mSIRT1 (PCR primer: Reverse)

<400>    55
aaatccagat cctccagcac                                                    20

<210>    56
<211>    20
<212>    DNA
<213>    Artificial Sequence

<220>
<223>    mSIRT2 (PCR primer: Reverse)

<400>    56
acgatatcgg gctttaccac                                                    20

<210>    57
<211>    20
<212>    DNA
<213>    Artificial Sequence

<220>
<223>    mSIRT3 (PCR primer: Reverse)

<400>    57
tcacaacgcc agtacagaca                                                    20

<210>    58
<211>    20
<212>    DNA
<213>    Artificial Sequence

<220>
<223>    mSIRT4 (PCR primer: Reverse)

<400>    58
ccacaattct cacccaacag                                                    20

<210>    59
<211>    20
<212>    DNA
<213>    Artificial Sequence

<220>
<223>    mSIRT5 (PCR primer: Reverse)

<400>    59

ctccctccgg tagtggtaaa                                                    20

<210>   60
<211>   20
<212>   DNA
<213>   Artificial Sequence

<220>
<223>   mSIRT6 (PCR primer: Reverse)

<400>   60
gtctgcacat cacctcatcc                                                    20

<210>   61
<211>   20
<212>   DNA
<213>   Artificial Sequence

<220>
<223>   mSIRT7 (PCR primer: Reverse)

<400>   61
agaggcgggg atatttcttt                                                    20

<210>   62
<211>   21
<212>   DNA
<213>   Artificial Sequence

<220>
<223>   ATGL (PCR primer: Reverse)

<400>   62
agtgagtggc tggtgaaagg t                                                  21

<210>   63
<211>   22
<212>   DNA
<213>   Artificial Sequence

<220>
<223>   Leptin (PCR primer: Reverse)

<400>   63
cagtggatgc taatgtgccc tg                                                 22

<210>   64
<211>   25
<212>   DNA
<213>   Artificial Sequence

<220>
<223>   Adiponectin (PCR primer: Reverse)

<400>   64

catctccttt ctctcccttc tctcc 25

## Description

### FIELD OF THE INVENTION

The present invention relates to a method for controlling an NAD(P)⁺/NAD(P)H ratio by oxidoreductase, preferable NAD(P)H:quinone oxidoreductase (NQOI). More specifically, the present invention relates to a technique capable of solving problems associated with excessive energy intake or an abnormal redox state, e.g. problems associated with diseases that may occur due to a low NAD(P)⁺/NAD(P)H ratio, by inducement of a high ratio of NAD(P)⁺/NAD(P)H reflecting an energy level. For example, NQO1 elevates an *in vivo* or *in vitro* NAD(P)⁺ level using NAD(P)H as a substrate and consequently increases the NAD(P)⁺/NAD(P)H ratio. Therefore, it is possible to solve the problems associated with various diseases which may arise from energy excess, such as obesity, diabetes, metabolic syndromes, degenerative diseases and mitochondrial dysfunction-related diseases.

### BACKGROUND OF THE INVENTION

Obesity, a condition in which an amount of body fat is abnormally high compared to standard body weight, refers to a disease resulting from accumulation of surplus calories in adipose tissues of the body when calorie intake is greater than calorie expenditure. Complications caused from obesity include, for example, hypertension, myocardial infarction, varices, pulmomary embolism, coronary artery diseases, cerebral hemorrhage, senile dementia, Parkinson's disease, type 2 diabetes, hyperlipidemia, cerebral apoplexy, various cancers (such as uterine cancer, breast cancer, prostate cancer, colon cancer and the like), heart diseases, gall bladder diseases, sleep apnea syndrome, arthritis, infertility, venous ulcer, sudden death, fatty liver, hypertrophic cardiomyopathy (HCM), thromboembolism, esophagitis, abdominal wall hernia (Ventral Hernia), urinary incontinence, cardiovascular diseases, endocrine diseases and the like (Obesity Research Vol. 12 (8), 2004,1197-1211).

Diabetes is a systemic metabolic disorder resulting from multiple environmental and genetic factors, and refers to a condition characterized by abnormally elevated blood glucose levels due to absolute or relative deficiency of insulin in the body. Complications of diabetes include, for example, hypoglycemia, ketoacidosis, hyperosmolar coma, macrovascular complications, erectile dysfunction (impotence), diabetic retinopathy, diabetic neuropathy, diabetic nephropathy and the like.

Metabolic syndromes refer to syndromes accompanied by health risk factors such as hypertriglyceridemia, hypertension, glycometabolism disorders, blood coagulation disorders and obesity. According to the ATP III criteria of the National Cholesterol Education Program (NCEP) published in 2001, individuals are diagnosed with the metabolic syndrome by the presence of three or more of the following components: 1) A waistline of 40 inches (102 cm) or more for men and 35 inches (88 cm) or more for women (central obesity as measured by waist circumference), 2) A triglyceride level above 150 mg/dL, 3) A high density lipoprotein (HDL) level less than 40 mg/dL (men) or under 50 mg/dL (women), 4) A blood pressure of 130/85 mm Hg or higher and 5) A fasting blood glucose level greater than 110 mg/dL.

Insulin resistance refers to a phenomenon wherein, even though insulin is normally secreted in the body, "supply of glucose into cells" performed by insulin does not work properly. Therefore, glucose in the blood cannot enter cells, thus causing hyperglycemia, and further, cells themselves cannot perform normal functions thereof due to a shortage of glucose, leading to the manifestation of metabolic syndrome.

The degeneration is the term derived from pathological findings, thus meaning the condition which is accompanied by "decreases in consumption of oxygen", and refers to a degenerative disease wherein dysfunction of mitochondria, which is an organelle that generates energy using oxygen within the cell, is associated with the senescence. As examples of the degenerative disease, mention may be made of neurodegenerative diseases such as Alzheimer's disease, Parkinson's disease and Huntington's disease (Korean Society of Medical Biochemistry and Molecular Biology News, 2004, 11 (2), 16-22).

Diseases arising from mitochondrial dysfunction may include for example, mitochondrial swelling due to mitochondrial membrane potential malfunction, functional disorders due to oxidative stress such as by the action of active oxygen species or free radicals, functional disorders due to genetic factors, and diseases due to functional deficiency of oxidative phosphorylation mechanisms for energy production of mitochondria. Specific examples of diseases, developed by the above-mentioned pathological causes, may include multiple sclerosis, encephalomyelitis, cerebral radiculitis, peripheral neuropathy, Reye's syndrome, Friedrich's ataxia, Alpers syndrome, MELAS, migraine, psychosis, depression, seizure and dementia, paralytic episode, optic atrophy, optic neuropathy, retinitis pigmentosa, cataract, hyperaldosteronemia, hypoparathyroidism, myopathy, amyotrophy, myoglobinuria, muscular hypotonia, myalgia, the decrease of exercise tolerance, renal tubulopathy, renal failure, hepatic failure, liver function failure, hepatomegaly, red blood cell anemia (iron-deficiency anemia), neutropenia, thrombocytopenia, diarrhea, villous atrophy, multiple vomiting, dysphagia, constipation, sensorineural hearing loss (SNHL), epilepsy, mental retardation, Alzheimer's disease, Parkinson's disease and Huntington's disease (see, for example US Patent No. 6,183,948; Korean Patent Laid-open Publication No. 2004-7005109; Journal of Clinical Investigation 111, 303-312, 2003; Mitochondria 74, 1188-1199, 2003; and Biochimica et Biophysica Acta 1658 (2004) 80-88).

The above-mentioned obesity, diabetes, metabolic syndromes, degenerative diseases and mitochondrial dysfunction-related diseases will be collectively referred to as "disease syndrome" hereinafter.

At present, the most effective way to ameliorate or fight against the conditions associated with such disease syndromes is known to be exercising more and losing weight, and dietary control. All of the currently effective ways of fighting against "the disease syndrome" have in common the fact that they facilitate energy metabolism, thus resulting in maximized expenditure of surplus energy in the body leading to prevention of energy accumulation. Effective expenditure of such surplus energy is considered a method for treating the disease syndrome. Enhancing of a metabolic activity is essential for effective elimination of surplus energy. For this purpose, it is essential to achieve inhibition of lipogenesis, inhibition of gluconeogenesis, facilitation of glucose consumption, facilitation of fat oxidation, facilitation of biogenesis of mitochondria that is a central apparatus of energy metabolism and collective activation of factors involved in metabolic activation.

Yet little is known about targets to treat the disease syndromes, whereas numerous target proteins or genes are known only for treating individual diseases and therefore there have been proposed some methods for the prevention or treatment of such diseases via use of the above-mentioned corresponding target proteins or genes. However, there is still room for further significant improvement even in treatment of individual diseases such as metabolic syndromes including obesity, diabetes, and the like. In spite of the fact that a great deal of studies has been conducted on the treatment of diseases, there are yet no drugs available for the treatment of various diseases resulting from excess energy intake and aging.

Most of diseases including obesity, diabetes, metabolic syndromes, degenerative diseases and mitochondrial dysfunction-related diseases, *i.e.,* large numbers of diseases including "disease syndromes", stem from the imbalance of energy metabolism and oxidation-reduction state. It is believed that all of diseases arise from energy excess due to superfluity of NAD(P)H.

NADPH is an important factor implicated in fat synthesis, and the synthesis of palmitate requires 14 NADPH molecules. NADPH is a reducing agent and is used in biosynthetic processes including fat synthesis. On the other hand, NADH is used in energy-producing reactions. However, surplus NAD(P)H, remained after fat synthesis and energy production, is scavenged by an oxidative enzyme, called NAD(P)H oxidase, present on a plasma membrane, during which free radicals such as reactive oxygen species (ROS) are generated. A primary cause responsible for increased oxidative stress in obesity and diabetic diseases was found to be NAD(P)H oxidase (Free Radical Biology & Medicine. Vol. 37, No 1, 115-123, 2004). It was also found that free radicals such as reactive oxygen species (ROS) generated by NAD(P)H oxidase are primary factors responsible for pathogenesis of various diseases such as cancers, cardiovascular diseases, hypertension, arteriosclerosis, cardiac hypertrophy, ischemic heart diseases, septicemia, inflammatory conditions and diseases, thrombosis, cranial nerve diseases (such as cerebral apoplexy (stroke), Alzheimer's disease, and Parkinson's disease), senescence-acceleration (J. Pharm. Pharmacol, 2005, 57 (1):111-116).

Therefore, when NAD⁺/NADH and NADP⁺/NADPH ratios *in vivo* or *in vitro* decrease, thus leaving surplus NADH and NADPH molecules, they are utilized in a fat biosynthesis process. In addition, since NADH and NADPH are also used as main substrates causing generation of reactive oxygen species (ROS) when they are present in excessive amounts, NADH and NADPH may be a pathogenic factor for significant diseases including inflammatory conditions and diseases caused by ROS. For these reasons, it is believed that fat oxidation and various energy expenditure (metabolism) by NAD⁺ and NADP⁺ will be activated if an *in vivo* or *in vitro* environment can be established to ensure stable maintenance of NAD⁺/NADH and NADP⁺/NADPH ratios in an increased state. As a result, if it is possible to activate an action mechanism continuously maintaining the NAD(P)H concentration at a low level, it is considered that various diseases including obesity can be treated by inducement of full expenditure of such surplus energy.

There has recently been a great deal of attention devoted to NA(D)P⁺. NA(D)P⁺ functions as a substrate or a coenzyme for various enzymes involved in numerous metabolisms including fat oxidation. Specifically, NA(D)P⁺ is an *in vivo* substance implicated in numerous biological metabolic processes and is used as a coenzyme of various enzymes responsible for regulation of energy metabolism, DNA repair and transcription. On the other hand, NAD⁺ is used as a substrate or a coenzyme for various kinds of enzymes including NAD⁺-dependent DNA ligase, NAD⁺-dependent oxidoreductase, poly(ADP-ribose) polymerase (PARP), CD38, AMPK, CtBP and Sir2p family members. NAD⁺ was found to play a crucial role through the above-mentioned *in vivo* actions in transcriptional regulation, longevity, calories restriction-mediated life span extension and aging-related diseases. NAD⁺ affects longevity and transcriptional silencing via regulation of Sir2p family members of putative NAD⁺-dependent deacetylases.

Meanwhile, the NAD(P)⁺/NAD(P)H ratio, a key regulator of an intracellular redox state, is often regarded as an indicator reflecting the metabolic state of organisms. The NAD(P)⁺/NAD(P)H ratio varies with changes in the metabolic process, *Inter alia,* it is known that NAD⁺ functions as a metabolic regulator. A variety of aging-related diseases are directly or indirectly associated with changes in the redox state of NAD⁺ or NAD(P)⁺/NAD(P)H.

Representative examples of proteins and genes, the activity of which is affected or is regulated by NAD(P)⁺, are as follows. The transcriptional co-repressor CtBP (C-terminal binding protein) in cooperation with the NAD⁺-dependent anti-aging gene Sir2 serves as an energy sensor to control transcription of chromatin, depending upon an intracellular energy state, and plays a critical role in regulation of tumorigenesis (Nature Struct Mol. Biol. 2005 12 (5):423-8). NAD⁺ controls p53 anticancer gene through the action of Sir2 (Molecular & Cellular Biology 2004, 24 (22), 9958-67).

The NAD⁺-dependent gene Sirt1 induces deacetylation of PGC1-alpha to control gluconeogenic and glycolytic genes, and is associated with energy homeostasis, diabetes, and lifespan regulation by stimulation of hepatic glucose release (Nature, 434 (3), 113-118, 2005).

Sirt1 was known to play a central role in calorie restriction-mediated life span extension, aging-related diseases (such as diabetes, cancers and cardiovascular diseases), production of adipose tissue hormones (adiponectin, leptin, TNFα, & resistin), increase of insulin sensitivity, and anti-aging action by reducing cranial nerve diseases (such as Huntington's disease, Alzheimer's disease, Parkinson's and stroke) and oxidative damage due to free radicals including reactive oxygen species (Nature Reviews Molecular Cell Biology 6, 298-305, 2005). It was found that Sirt1 increases the activity of nicotinamide mononucleotide adenylyltransferase (Nmnat) to protect the cerebrum and the spinal cord, thereby being therapeutically effective for the treatment of nerve fiber diseases and degenerative cerebral diseases (multiple sclerosis, encephalomyelitis, Parkinson's disease, Alzheimer's disease, Leigh syndrome, Friedreich's ataxia, Spastic paraplegia, Deafness-dystonia syndrome, Wilson's disease, Amyotrophic lateral sclerosis, Huntington's disease) (Science, 305 (13), 1010-1013, 2005). Sirt1 promotes fat mobilization in white adipocytes by repressing PPAR-γ (peroxisome proliferator activated receptor) (Nature 429 (17), 771-776, 2004). Sirt1 regulates HIV transcription via Tat deacetylation, thus indicating that Sirt1 is a novel target for the treatment of HIV infection (PLoS Biology 3, 210-220, 2005).

Further, it is known that calorie restriction has effects on changes of physiological properties including levels of blood glucose, triglyceride and hormones as well as on behavioral characteristics including movement, activity and endurance of organisms. Therefore, through the experiment using the Sirt1 gene, it was demonstrated with a high significance that Sirt1 affecting calorie restriction is closely involved in enhancement of movement capacity, activity capacity and endurance (Science, 310 (9), 2005, 1641).

Meanwhile, it was confirmed that AMP-activated protein kinase (AMPK) is a protein that senses the energy status, redox state and phosphorylation degree in living organisms, and is activated not only by AMP but also by NAD⁺ (J. Biol. Chem. 2004, Dec. 17;279 (51):52934-9). AMPK activated by phosphorylation has been reported to exhibit various functions and actions such as in inhibition of fat synthesis, promotion of glucose uptake, promotion of fat degradation (lipolysis) and fat oxidation, promotion of glycolysis, enhancement of insulin sensitivity, suppression of glycogen synthesis, suppression of triglyceride and cholesterol synthesis, alleviation of inflammation (anti-inflammatory action), vasodilatory activity, functional improvement of cardiovascular systems, mitochondrial regeneration and muscle structural changes, anti-oxidative function, anti-aging and anti-cancer effects, In addition, due to exertion of the above-mentioned various activities and functions, AMPK is recognized as a target protein for treatments of diseases such as obesity, diabetes, metabolic syndromes, fatty liver, ischemic heart diseases, hypertension, degenerative cerebral diseases, hyperlipidemia, diabetic complications and erectile dysfunction (Nat Med. 2004 Jul;10(7):727-33; Nature reviews 3, 340-351, 2004; and Genes & Development 27, 1-6,2004).

In addition to the direct use of NAD(P)⁺ as a coenzyme or a substrate, NAD⁺ converts into cyclic adenosine diphosphate-ribose (cADPR) by the action of ADP ribosyl cyclase (CD38), whereas NADP⁺ converts into nicotinic acid adenine dinucleotide phosphate (NAADP) by the action of ADP ribosyl cyclase (CD38). cADPR and NAADP produced by CD38 functions as a second messenger implicated in the mobilization of intracellular calcium (Ca²⁺).

As methods to increase a concentration and ratio of NAD(P)⁺, a signal transducer known to have such various functions, consideration may be feasibly given to 1) regulation of salvage synthesis which is an NAD(P)⁺ biosynthetic process, 2) elevation of an NAD(P)⁺ concentration *in vivo* by activation of genes or proteins of enzymes utilizing NAD(P)H as the substrate or coenzyme, and 3) elevation of an NAD(P)⁺ concentration via supply of NAD(P)⁺ or analogues, derivatives, precursors or prodrugs thereof from external sources.

Meanwhile, NAD(P)H:quinone oxidoreductase (EC1.6.99.2) (NQO) is also called DT-diaphorase, quinone reductase, menadione reductase, vitamin K reductase, or azo-dye reductase, and such NQO is present in two isoforms, designated NQO1 and NQO2 (ROM. J. INTERN. MED. 2000-2001, Vol. 38-39, 33-50), NQO is a flavoprotein and catalyzes two electron reduction and detoxification of quinone or quinone derivatives. NQO utilizes both of NADH and NADPH as an electron donor. The activity of NQO prevents formation of very highly-reactive quinone metabolites, detoxifies benzo(d)pyrenes and quinones, and diminishes toxicity of chromium. The activity af NQO is found in all kinds of tissues, but varies from tissue to tissue. Generally, high-level expression of NQO was confirmed in cancer cell, liver, stomach and kidney tissues. NQO gene expression is triggered by xenobiotics, anti-oxidants, oxidants, heavy metals, UV light, radiation exposure, or the like. NQO is a part of numerous cellular defense mechanisms induced by oxidative stress. Associated expressions of genes implicated in such cellular defence mechanisms, including NQO gene expression, serve to protect cells against oxidative stress, free radicals and neoplasia. NQO has a very broad substrate specificity, and therefore can utilize quinone-imines, nitro and azo-based compounds as a substrate, in addition to quinones.

Among NQO enzymes, NQO1 is largely distributed in epithelial and endothelial cells. This implies that NQO1 can act as a defense mechanism against compounds absorbed via air, the esophagus or blood vessels. With recent research showing participation of NQO1 in stabilization of the p53 tumor suppressor gene through the redox mechanism, it is anticipated that NQO1 will play an important role in suppression of tumorigenesis, Since NQO I is present at a high level, particularly in many solid tumor cells, a great deal of attention has been particularly focused on NQO that is activated by quinone-based compounds.

The present invention is also directed to a method for boosting exercise capacity and/or endurance of a subject in need thereof by artificial elevation, of an NAD(P)⁺NAD(P)H ratio *in vivo.* Further, according to the present invention, artificial elevation of the *in vivo* NAD(P)⁺/AD(P)H ratio may also bring about enhancement of energy production capacity, fast relief from fatigue, vitality enhancement, and improvement of reactive oxygen species and free radical scavenging capacity.

Examples of the method for artificial elevation of the NAD(P)⁺/NAD(P)H ratio may include, but are not limited to, a method for artificial elevation of the concentration and ratio of NAD(P)⁺ by external supply of NAD(P)⁺ or analogues, derivatives, precursors or prodrugs thereof, and a method for artificial elevation of the NAD(P)⁺ concentration by external supply of compounds that activate genes or proteins of enzymes utilizing NAD(P)H as a substrate or a coenzyme.

### SUMMARY OF THE INVENTION

As a result of a variety of extensive and intensive studies and experiments based on the facts described above, the inventors of the present invention have discovered that oxidoreductase, preferably NAD(P)H:quinone oxidoreductase, and particularly NAD(P)H:quinone oxidopeductase 1 (NQO1), functions as an enzyme controlling the intracellular redox state and energy level via production of NAD(P)⁺ from NAD(P)H through the oxidation-reduction reaction. When an NAD(P)⁺/NAD(P)H ratio elevates *in vivo* or *in vitro,* cells recognize this event as an energy-deficient state and therefore the cellular metabolism is modulated to be converted into an energy production system for compensation of deficient energy using energy sources including glucose, fat and glycogen as a substrate. Therefore, when NQO1 activation conditions are established under energy excess or an imbalanced redox state, NQO1 produces NAD(P)⁺ utilizing NAD(P)H as a substrate, leading to an increase in the cytosolic NAD(P)⁺/NAD(P)H ratio.

That is, the increased state of the NAD(P)⁺/NAD(P)H ratio is recognized as deficiency of energy, which in turn allows the cells (living organisms) to activate energy production and redox-related systems to increase metabolic activity, such that NAD(P)H deficiency can be compensated. This fact means that the NQO1 protein (or NQO1 1 gene) is a novel target protein (or gene) capable of treating diseases such as obesity, diabetes, metabolic syndromes, degenerative diseases and mitochondrial dysfunction-related diseases, *i.e*. "disease syndromes",

Based on these facts, the present inventors have confirmed that compounds activating NQO1 or inducing production of NQO1 are effective for the development of therapeutic agents for degenerative diseases including obesity, diabetes and metabolic syndromes. Further, we also confirmed that therapeutic validity of such compounds and development of therapeutic agents can be secured by maintaining the NAD(P)⁺/NAD(P)H ratio at a high level under *in vivo* or *in vitro* condition. In this regard, as a method capable of preventing and treating diseases that may occur due to a low NAD(P)⁺/NAD(P)H ratio arising from energy excess or an abnormal redox state, the present inventors have confirmed that it is possible to effectively treat all kinds of diseases that may occur due to problems associated with energy excess or the imbalanced redox state by elevation of an NAD(P)⁺ concentration via the action of NQO1 using NAD(P)H as a substrate or a coenzyme and the consequential elevation in the NAD(P)⁺/NAD(P)H ratio. Therefore, the inventors of the present invention confirmed that NQO1 is a therapeutic target for all kinds of diseases that may develop due to energy excess or an abnormal redox state and simultaneously demonstrated that NQO1 as consequently effective for the prevention and treatment of diseases by elevation of the NAD(P)⁺/NAD(P)H ratio to activate proteins and genes requiring NAD(P)⁺ as a substrate or a coenzyme, resulting in regulation of the metabolism.

### DETAILED DESCRIPTION OF PREFERRED EMBODIMENTS

In accordance with an aspect of the present invention, the above and other objects can be accomplished by the provision of a method for increasing an NAD(P)⁺/NAD(P)H ratio *in vivo* or *in vitro* by oxidoreductase, preferably NAD(P)H:quinone oxidoreductase, particularly NAD(P)H:quinone oxidoreductase 1 (NQO1).

In addition to NAD(P)H:quinone oxidoreductase as mentioned above, examples of homologous enzymes capable of converting NAD(P)H into NAD(P)⁺ via use of NAD(P)H as a substrate or a coenzyme may include Nitric Oxide synthase (NOS or NOS NADPH DT-diaphorase), thioredoxin reductase (NADPH-oxidized thioredoxin oxidoreductase, E.C. 1.6.4.5), glutaflione-dependent oxidoreductase (thiol-disulfide oxidoreductase), NADPH dehydrogenase (E.C. 1.6.99.6), NADH:ubiquinone oxidoreductase, succinate:ubiquinone oxidoreductase, plasma membrane oxidoreductase, cytochrome c oxidoreductase (EC 1.10.2.2, bc(1) complex), oxoglutarate oxidoreductase, and the like.

According to the method of the present invention, conversion of NAD(P)H into NAD(P)⁺ is induced by activating NQO1 or increasing an amount of NQO1 via addition of a certain compound in *in vivo* or *in vitro* conditions, consequently increasing the NAD(P)⁺/NAD(P)H ratio. Therefore, it is possible to prevent and treat a variety of diseases, including obesity, that may occur due to a low NAD(P)⁺/NAD(P)H ratio caused by energy excess or an abnormal redox state. In addition, upon considering the facts that the NAD(P)⁺/NAD(P)H ratio is elevated primarily in association with energy expenditure *in vivo* and such energy expenditure is caused by physical exercise, an increasing activity or amount of NQO1 may lead to enhancement in activity and endurance of a subject. Such an increase in the activity or amount of NQO1 may be expressed just as quasi-exercise induced effects.

As used herein, the term *"in vivo"* refers to physiological conditions in a biological subject, and the term *"in vitro"* refers to a concept encompassing conditions of cells isolated from the biological subject and acellular conditions consisting of cell components or parts thereof. Hereinafter, the term *"in vivo* and *in vitro"* sometimes will be simply referred to as *"in vivo*/*vitro".*

Since the term *"in vivo"* according to the present invention is a concept embracing up to a cellular level, there is no particular limit to the *in vivo* subject so long as it is viable. Preferably, the subject may be a mammal. Preferably, the mammal may be a human,

With an increased NAD(P)⁺/NAD(P)H ratio via activation of NQO1, cells recognize deficiency of energy by an increase in the *in vivo*/*vitro* NAD(P)⁺ ratio and the cells activate, in turn, genes and proteins involved in energy production, or NAD(P)⁺ directly functions as a donor factor, thereby modulating a variety of metabolic processes.

Examples of a method for elevating the NAD(P)⁺/NAD(P)H ratio by NQO1 may preferably include 1) production of NAD(P)⁺from NAD(P)H at above a critical rate by increasing the activity of NQO1, 2) enhancement of consumption of an NAD(P)H substrate by increasing an amount of the NQO1 protein or an expression of the NQO1 gene, and 3) elevation of the NAD(P)⁺/NAD(P)H ratio by direct addition of NAD(P)⁺, or derivatives or prodrugs thereof.

Based on the amount of NAD(P)H in the absence of an activator for NQO1, changes in the NAD(P)⁺/NAD(P)H ratio may preferably be in the range of more than a 20% decrease of the NAD(P)H amount effected by the NQO1 activator, consequently leading to an increase in the NAD(P)⁺/NAD(P)H ratio. More preferably, the decrease of NAD(P)H may be more than 30%. However, if the decrease of NAD(P)H is excessively high, this may cause problems in *in vivo*/*vitro* metabolic processes. Therefore, a decrease of NAD(P)H may be preferably in a range of less than 80%.

In order to increase the activity of NQO1 and/or the amount of NQO1 protein or the expression of NQO1 gene, for example, use of a certain compound increasing the activity or amount of NQO1 may be contemplated.

Such a compound serves as a hydride acceptor (H⁻ acceptor). Specific examples of the compound that can be used in the present invention may include, but are not limited to, quinone-based compounds, quinone-imine-based compounds, nitre-based compounds, and axe-based compounds. In addition, these compounds may be used alone or in any combination thereof.

Representative examples of the quinone-based compound may include naphthoquinone-based compounds and derivatives thereof, such as 4-aminoalkyl-1,2-nahthoquinone, 4-thiolkyl-1,2-naphthoquinone, 4-alkoxy-1,2-naphthoquinone, furano-o-naphthoquinone, pyrano-o-naphthoquinone or a derivative thereof. Preferable 4-substituted-1,2-naphthoquinones are represented by Formula I below. Unless otherwise particularly specified in the present specification, the compound being depicted as being therapeutically effective encompasses pharmaceutically acceptable salts, prodrugs, solvates and isomers thereof. 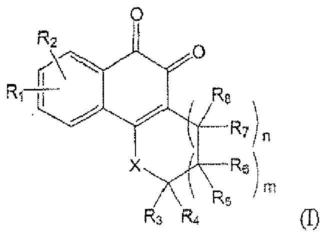 wherein
R₁ and R₂ are each independently hydrogen, halogen, alkoxy, hydroxy or lower alkyl having 1 to 6 carbon atoms;
R₃, R₄, R₅, R₆, R₇ and R₈ are each independently hydrogen, hydroxy, C₁-C₂₀ alkyl, akene or alkoxy, cycloalkyl, heterocycloakyl, aryl or heteroaryl, or two substituents of R₃ to R₈ may be taken together to form a cyclic structure;
X is oxygen, nitrogen or sulfur; and
m and n are each independently 0 or 1, with proviso that when either of m and n is 0, carbon atoms adjacent to m or n may form a cyclic structure via a direct bond.

Among those compounds, particularly preferred are compounds of Formula 1 wherein X is oxygen, m is 1, and n is 0 or 1, with the proviso that when n is 0, carbon atoms adjacent to n form a cyclic structure via a direct bond.

In examples of the preferred compounds, the compounds of Formula I wherein n is 0 may be expressed as furano-O-naphthoquinones, whereas the compounds of Formula I with n = 1 may be expressed as pyrano-O-naphthoquinones. Typical examples of furano-O-naphthoquinones or pyrano-O-naphthoquinones may include compounds of Formula II or III: 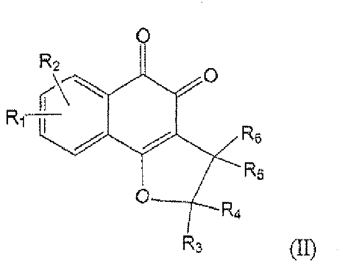 wherein R₁, R₂, R₃, R₄, R₅ and R₆ are the same as defined in Formula I. 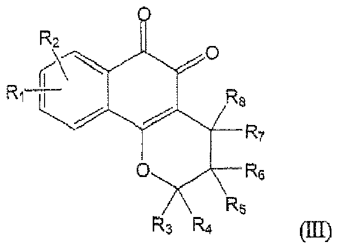 wherein R₁, R₂, R₃, R₄, R₅, R₆, R₇ and R₈ are the same as defined in Formula I.

The above-mentioned materials may be artificially synthesized or may be extracted from herbal medicinal plants, e.g; Danshen (*Salvia miltiorrhiza*), *Perovskia abrotanoides,* and the like. A person having ordinary skill in the art to which the invention pertains would readily practice synthesis of the compounds having the above chemical structure without particular difficulty, based upon the general technologies and practices in the organic chemistry synthesis field. Further, a method of extracting active ingredients of interest from herbal medicinal plants, such as Danshen, are well-known in the art and thus the detailed descriptions thereof will be omitted in the present disclosure.

In order to demonstrate a relationship between NQO1 and the NAD(P)⁺/NAD(P)H ratio in the present invention, the following experimental tests were carried out. NQO1 is rich in cytosolic NAD(P)H-depcndent oxidoreductase having a high affinity for natural and synthetic quinones and therefore NQO1 is selected as oxidoreductase for a therapeutic: target.
(1) Using a DCPIP (2, 6-dichlorophenol-indophenol) method for measuring the activity of NQO1, changes in the NAD(P)⁺/NAD(P)H ratio were measured to confirm the distribution and activity of NQO1 in tissues.
(2) In order to confirm that the NAD(P)⁺/NAD(P)H ratio can be elevated only by NQO1, an NQO1-activating drug was added to NQO1-/- cells and normal cells and changes in the NAD(P)⁺/NAD(P)H ratio were checked to confirm whether NQO1 plays a pivotal role in regulation of an intracellular NAD(P)⁺/NAD(P)H ratio,
(3) As another experiment to demonstrate that the intracellular NAD(P)⁺/NAD(P)H ratio is increased by NQO1, changes in the NAD(P)⁺/NAD(P)H ratio were measured by treatment of the NQO1 -activating drug on NQO1-/- cells with incorporation of the NQO1 gene.
(4) In confirmation of proteins that are effected by the presence/absence and activation of NQO1, effects of the NQO1 activity on expression and activity of proteins were confirmed through confirmation of phosphorylation of AMP-activated kinase (AMPK), activation of which is known to be effected by changes of the NAD(P)⁺/NAD(P)H ratio. By confirming phosphorylation of the AMPK protein via treatment of the NQO1-activating drug on NQO1-/- cells and NQO1-/- cells with insertion of the NQO1 gene, respectively., the effects of NQO1 were examined with regard to expression and activation of proteins which are susceptible to differences in energy level and redox state by activation of NQO1.
(5) In order to measure intracellular calcium influx by the presence/absence and activation of NQO1, an attempt was made to confirm the presence and degree of calcium influx by the NQO1-activating drug in NQO1+/+ cells and NQO1-/- cells. On the other hand, the NQO1 activating drug and the NQO1-inhibiting drug were treated on NQO1+/+ cells to confirm whether the calcium influx is affected via repression of NQO1 activation.
(6) In order to examine whether the NQO1-activating drug takes part in mitochondrial biogenesis and endothelial nitric oxide (NO) production responsible for erectile dysfunction, phosphorylation and the phosphorylation degree of an eNOS (endothelial nitric oxide synthase) protein were confirmed.
(7) In order to confirm changes in the *in vivo* redox state by NQO1, the NQO1-activating drug was intravenously injected into mice and liver tissues were removed to measure changes in amounts of NAD⁺ and NADH.
(8) Therapeutic effects via external addition of NAD⁺ were examined through confirmation of body weight changes, hematological changes, histological changes, gene egression and protein activity changes, fat distribution changes, hormonal changes, respiratory quotient, exercise capacity and endurance due to elevation of the *in vivo* NAD⁺ concentration following external addition of NAD⁺ or analogues, precursors or prodrugs thereof.

Therefore, since NQO1 has direct effects on elevation of the *in vivo*/*vitro* NAD(P)⁺/NAD(P)H ratio as demonstrated through various *in vivo* and *in vitro* experiments in the present invention, it was confined that NQO1 (or the NQO1 gene) is an excellent target protein (or gene) effective for prevention and treatment of obesity, diabetes, metabolic syndromes, degenerative diseases and mitochondrial dysfunction-related diseases and improvement of activity, motility and endurance.

More specifically, it was confirmed through the above experiments that an increase in the NAD(P)⁺/NAD(P)H ratio accelerates an additional increase of cytosolic and mitochondrial calcium levels, activates AMPK and Sirt proteins, and promotes mitochondrial oxidative phosphorylation and fatty acid oxidation.

The NQO1 activator and a high level of NAD⁺ activates AMPK through a reaction mechanism involving at least two steps; e.g. direct binding of AMP to AMPK and the phosphorylation of AMPK by CaMKK and LKB1. It is surmised that CaMKK takes part in the rapid early phase activation and AMPKα T172 phosphorylation, and LKB1 plays a certain role in sustained AMPK activation. On the other hand, it was confirmed that the NQO1 activator inhibits the AMPK activity in the hypothalamus of a DIO (diet induced obesity) mouse and therefore leads to decreased appetite, decreased dietary intake and body weight loss. These findings suggest that treatment with the NQO1 activator can increase energy consumption to accelerate weight loss.

In accordance with another aspect of the present invention, there is provided a method for identifying a compound to control an NAD(P)⁺/NAD(P)H ratio, comprising contacting a candidate compound group with NQO1 to identify a compound increasing the NAD(P)⁺/NAD(P)H ratio via NQO1, and monitoring the amount or activity of NQO1 by the candidate compound group.

As used herein, the term "contacting" refers to mixing of a solution containing the candidate compound group with a liquid medium in which NQO1+/+ cells were impregnated. The solution containing the candidate compound group may further contain other components including dimethyl sulfoxide (DMSO) that facilitates the uptake of compound(s) into cells. The candidate compound-containing solution may be added to a cell-impregnated medium using a delivery device such as a pipette or a syringe.

As used herein, the term "monitoring" refers to observation of the effects exerted by addition of the compound(s). For example, the effects of compound addition can be observed as changes in the NAD(P)⁺/NAD(P)H ratio, AMPK activation, inhibition of ACC activity, increased eNOS phosphorylation, expression of genes involved in energy metabolism, expression of genes involved in mitochondrial biogenesis, and the like.

As a specific example of the above-mentioned method for identifying the compound of interest, mention may be made of a method involving reacting NQO1 with a subject compound to be screened and NAD(P)H for a predetermined time, and quantifying the resulting NAD(P)⁺or the remaining NAD(P)H.

As an example of a method to quantify an amount of the produced NAD(P)⁺, there may be used a method of measuring changes in absorbance by inducement of color development due to changes in an absorption wavelength via reduction of DCPIP used as a hydride (H⁻) acceptor. As an example of a method to quantify the remaining amount of NAD(P)H, mention may be made of a method of quantifying the remaining amount of NAD(P)H via measurement of changes in absorbance due to color development of a tetrazolium salt.

Specific examples of the NQO1-activating compound may include naphthoquinone-based compounds, esters of fumaric acid, oltipraz (4-methyl-5(2-pyrazinyl)-1,2-dithiole-3-thione), curcumin, anethole dithiolethione, sulforaphane, 6-methylsulphinylhexyl isothiocyanate, caffeic acid phenethyl ester, 4'-bromoflavone, avicins, fisetin, resveratrol and derivatives thereof Representative examples of the fumaric acid esters may include dimethylfumarate, monoethylfumarate, monomethylfumarate and salts thereof. In a preferable example, the compound may be 4-alkoxy-1,2-naphlioquinone-based compound and a derivative thereof and dimethylfumarate and an analogue thereof.

In accordance with a further aspect of the present invention, there is provided use of a compound capable of increasing an amount or activity of NQO1 for the manufacture of a medicament for the treatment or prevention of a disease associated with an NAD(P)⁺/NAD(P)H ratio decrease.

In accordance with yet another aspect of the present invention, there is provided a method for treating or preventing a disease associated with an NAD(P)⁺/NAD(P)H ratio decrease, comprising administering a therapeutically effective amount of a compound capable of increasing an amount or activity of NQO1 to a subject in need thereof

As used herein, the phrase "disease associated with an NAD(P)⁺/NAD(P)H ratio decrease" refers to various types of diseases caused directly or indirectly by a decrease of the NAD(P)⁺/NAD(P)H ratio and may include, for example, obesity, obesity complications, diabetes, diabetic complications, metabolic syndromes, degenerative diseases, and mitochondrial dysfunction.

As used herein, the term "treatment" refers to stopping or delaying of the progress of the disease, when the drug of interest is used in the subject exhibiting symptoms of disease onset. The term "prevention" refers to stopping or delaying of symptoms of disease onset, when the drug of interest is used in the subject exhibiting no symptoms of disease onset but having high risk for disease onset.

For experimental demonstration of the above method, an attempt has been made to secure the basis of establishing therapeutic fields of diseases that can be treated by activation of NQO1, via confirmation of body weight changes, hematological changes, histological changes, gene expression and protein activity changes, fat distribution changes, hormonal changes, respiratory quotient (RQ), exercise capacity and endurance of the subject, resulting from activation of NQO1 after administration of the NQO1-activating drug to affected animals with obesity, diabetes, and the like.

Specifically, it is possible to achieve prophylaxis and/or treatment of clinical diseases related to metabolic syndromes by elevating the NAD(P)⁺/NAD(P)H ratio through changes in redox and energy state based on the action of NQO1. Examples of these clinical diseases may include, but are not limited to, common obesity, abdominal obesity, hypertension, arteriosclerosis, hyperinsulinemia, hyperglycemia, type 2 diabetes mellitus and dyslipidemia characteristically appearing with insulin resistance. Dyslipidemia, also known as the atherogenic lipoprotein profile of phenotype B, is characterized by significantly elevated non-esterified fatty acids, elevated very low density lipoproteins (VLDL) triglyceride rich particles, high values of ApoB, the presence of small, dense, low-density lipoprotein (LDL) particles, high values ofApoB in the presence of phenotype B, and low value of high density lipoproteins (HDL) associated with a low value of ApoAI particles.

By elevating the NAD(P)⁺/NAD(P)H ratio through changes in redox and energy state, the method of the present invention is expected to be useful for treating patients suffering from combined or mixed dyslipidemia, or hypertriglycerimia having or having not other signs of metabolic syndrome and suffering from various severities of postprandial dyslipidemia.

By elevating the NAD(P)⁺/AD(P)H ratio through changes in redox and energy state, the method of the present invention is expected to lower the cardiovascular morbidity and mortality associated with arteriosclerosis due to anti-inflammatory properties and anti-dyslipidemic properties. Examples of the cardiovascular diseases include macro-angiopathies of various internal organs causing myocardial infarction, cardiac insufficiency, cerebrovascular disease, and peripheral arterial insufficiency of the lower extremities. On the other hand, because of its insulin-sensitizing effects, the method of the present invention is also expected to prevent or retard the progress of type 2 diabetes mellitus in metabolic syndrome and development of diabetes during pregnancy. Therefore, the method of the present invention is also expected to retard the progress of chronic complications associated with clinical hyperglycemia in diabetes, for example, the micro-angiopathies causing renal disease, retinal disorders and peripheral vascular diseases of the lower extremities. Furthermore, the method of the present invention may be useful in treatment of various diseases and conditions other than disorders of the cardiovascular system, regardless of association with insulin resistance, for example polycystic ovarian syndrome, obesity, cancers, inflammatory diseases, and neurodegenerative diseases such as Mild Cognitive Impairment (MCI), Alzheimer's disease, Parkinson's disease and multiple sclerosis.

By elevating the NAD(P)⁺/NAD(P)H ratio through changes in redox and energy state, the method of the present invention exhibits inhibitory effects against the development of fatty liver (hepatic steatosis) and also activates β-oxidation of fatty acids, thereby playing a role in lowering concentration of triglycerol and thus is expected to be useful for preventing or treating fatty liver, hepatitis and hepatic cirrhosis arising from lipid dysmetabolism of alcoholic and non-alcoholic liver.

By elevating the NAD(P)⁺/AD(P)H ratio through changes in redox and energy state, the present invention varies lipid composition in various tissues. In addition, it was confirmed that the present invention can vary a fat content as well as fat distribution.

Further, the present invention also reduces plasma cholesterol and triacylglycerol levels, Therefore, it can be seen that plasma triglyceride and cholesterol levels are lowered according to the present invention.

By elevating the NAD(P)⁺/NAD(P)H ratio through changes in redox and energy state, the method of the present invention is effective for endothelial nitric oxide (NO) production via activation of eNOS (endothelial nitric oxide synthase), and thus is expected to be useful for preventing or treating ischemic cardiac diseases, mitochondrial myopathy, degenerative cerebral diseases, diabetes, cardiomyopathy, aging-related diseases, vascular diseases, hypertension and erectile dysfunction. As hypertension-causing diseases, mention may be made of cardiac insufficiency, myocardial infarction, rupture of the cerebrovascular system, thrombosis and renal damage.

By elevating the NAD(P)⁺/NAD(P)H ratio through changes in redox and energy state, the method of the present invention elicits promotion of fat oxidation and energy expenditure in peripheral tissues, thereby being expected to be effective for treatment or prevention of common obesity and also in removal of localized fat deposits such as subcutaneous and abdominal fat, *e.g.* when it is desired to remove fat from particular regions where fat is locally deposited such as removing subcutaneous fat from protuberant parts of the eye-lids, arms and hips, abdominal fat and fat of particular regions, for example, cellulite.

Further, it was conformed that the method of the present invention lowers the blood glucose level, thereby plasma insulin concentrations of animals affected with hyperinsulinism. In addition, it was confirmed that the method of the present invention enhances the effects of insulin in animals having decreased insulin sensitivity.

By elevating the NAD(P)⁺/NAD(P)H ratio through changes in redox and energy state, the method of the present invention promotes mitochondrial biogenesis, thereby increasing the active capacity of mitochondria, and at the same time induces conversion of muscle tissues into motor tissues, thereby resulting in improved exercise capacity, enhanced endurance, improved energy productivity, recovery from fatigue, increased vital power, reduction of oxidative stress through increased ability to remove reactive oxygen species (ROS) and free radicals, and therefore the method is expected to be effective for treating the diseases concerned. As diseases that may be caused by reactive oxygen species (ROS), mention may be made of the followings: arteriosclerosis, diabetes mellitus, ischemic heart diseases, cardiac hypertrophy, neurological diseases, kidney diseases, hepatocirrhosis, thrombosis, inflammatory conditions and diseases, arthritis, Retinopathy of Prematurity, ocular uveitis, senile cataract, disorders due to radiotherapy side effects, smoking-induced bronchial damage, disorders due to side effects of carcinostatic agents, cerebral edema, septicemia, lung edema, foot edema, cerebral infarction, hemolytic anemia, progeria, epilepsy, Alzheimer's disease, Down's syndrome, Crohn's disease and collagenosis.

By elevating the NAD(P)⁺/NAD(P)H ratio through changes in redox and energy state, the present invention is expected to be effective as a therapeutic target for treating diseases including obesity and obesity complications, *e.g.* hypertension, myocardial infarction, varices, pulmonary embolism, coronary artery diseases, cerebral hemorrhage, senile dementia, Parkinson's disease, type 2 diabetes, hyperlipidemia, cerebral apoplexy, various cancers (such as uterine cancer, breast cancer, prostate cancer, colon cancer and the like), heart diseases, gall bladder diseases, sleep apnea syndrome, arthritis, infertility, venous ulcer, sudden death, fatty liver, hypertrophic cardiomyopathy (HCM), thromboembolism, esophagitis, abdominal wall hernia (Ventral Hernia), urinary incontinence, cardiovascular diseases, endocrine diseases and the like.

By increasing the NAD(P)⁺/NAD(P)H ratio through changes in redox and energy state, the present invention is expected to be effective for prevention or treatment of diabetes as well as diabetic complications including for example hypoglycenia, ketoacidosis, hyperosmolar coma, macrovascular complications, diabetic retinopathy, diabetic neuropathy, diabetic nephropathy and the like.

By elevating the NAD(P)⁺/NAD(P)H ratio through changes in redox and energy state, the present invention is expected to be effective as a therapeutic target for treating dysfunction of mitochondria, a small energy-production organelle of a cell, due to decreased oxygen consumption, and aging-related degenerative diseases, for example neurodegenerative diseases such as Alzheimer's disease, Parkinson's disease and Huntington's disease.

Specifically, by elevating the NAD(P)⁺/NAD(P)H ratio through changes in redox and energy state, the present invention is expected to be effective as a therapeutic target for treating diseases arising from mitochondrial dysfunction, e.g. swelling due to mitochondrial membrane dysfunction, functional disorders due to oxidative stress such as by the action of reactive oxygen species or free radicals, dysfunction due to genetic factors, and diseases due to defects in mitochondrial oxidative phosphorylation for energy production, including multiple sclerosis, encephalomyelitis, cerebral radiculitis, peripheral neuropathy, Reye's syndrome, Friedrich's ataxia, Alpers syndrome, MELAS, migraine, psychosis, depression, seizure and dementia, paralytic episode, optic atrophy, optic neuropathy, retinitis pigmentosa, cataract, hyperaldosteronemia, hypoparathyroidism, myopathy, amyotrophy, myoglobinuria, muscular hypotonia, myalgia, the decrease of exercise tolerance, renal tubulopathy, renal failure, hepatic failure, liver function failure, hepatomegaly, red blood cell anemia (iron-deficiency anemia), neutropenia, thrombocytopenia, diarrhea, villous atrophy, multiple vomiting, dysphagia, constipation, sensorineural hearing loss (SNHL), epilepsy, mental retardation, Alzheimer's disease, Parkinson's disease and Huntington's disease.

By elevating the NAD(P)⁺/NAD(P)H ratio through changes in redox and energy state, the present invention is expected to be effective as a therapeutic target for therapy and/or prophylaxis of multiple metabolic syndrome (metabolic syndrome) characteristically appearing with hyperinsulinism, insulin resistance, obesity, glucose intolerance, type 2 diabetes mellitus, dyslipidemia, cardiovascular diseases or hypertension in particular.

Where appropriate, desired treatment or effects can be achieved by direct addition of NAD⁺ to a subject from an external source. In this regard, the present invention has confirmed though elevation of the NAD⁺ concentration therapeutic effects of external NAD⁺ addition by confirmation of body weight changes, hematological changes, histological changes, gene expression and protein activity changes, fat distribution changes, hormonal changes, respiratory quotient (RQ), exercise capacity and endurance of the subject.

Further, the present invention provides a pharmaceutical composition comprising (a) a therapeutically effective amount of a compound capable of increasing an amount or activity of NQO1 and (b) a pharmaceutically acceptable carrier, diluent or vehicle, or any combination thereof, *i.e.* an NQO1-enhancing composition.

The term "pharmaceutical composition" as used herein means a mixture of the above compound with other chemical components, such as carriers or diluents. The pharmaceutical composition facilitates administration of the compound to an organism. Various techniques of administering a compound are known in the art and include, but are not limited to oral, injection, aerosol, parenteral and topical administrations. Pharmaceutical compositions can also be obtained by reacting compounds of interest with acids such as hydrochloric acid, hydrobromic acid, sulfuric acid, nitric acid, phosphoric acid, methanesulfonic acid, p-toluenesulfonic acid, salicylic acid and the like.

The term "therapeutically effective amount" means an amount of an active ingredient that is effective to relieve or reduce to some extent one or more of the symptoms of the disease in need of treatment, or to retard initiation of clinical markers or symptoms of a disease in need of prevention, when the compound is administered. Thus, a therapeutically effective amount refers to an amount of the active ingredient which exhibit effects of (i) reversing the rate of progress of a disease; (ii) inhibiting to some extent further progress of the disease; and/or, (iii) relieving to some extent (or preferably eliminating) one or more symptoms associated with the disease. The therapeutically effective amount may be empirically determined by experimenting with the compounds concerned in known *in vivo* and *in vitro* model systems for a disease in need of treatment.

The term "carrier" means a chemical compound that facilitates the incorporation of a compound into cells or tissues. For example, dimethyl sulfoxide (DMSO) is a commonly utilized carrier as it facilitates the uptake of many organic compounds into the cells or tissues of an organism.

The term "diluent" defines chemical compounds diluted in water that will dissolve the compound of interest as well as stabilize the biologically active form of the compound. Salts dissolved in buffered solutions are utilized as diluents in the art. One commonly used buffer solution is phosphate buffered saline (PBS) because it mimics the ionic strength conditions of human body fluid. Since buffer salts can control the pH of a solution at low concentrations, a buffer diluent rarely modifies the biological activity of a compound.

The compounds described herein may be administered to a human patient per *se,* or in the form of pharmaceutical compositions in which they are mixed with other active ingredients, as in combination therapy, or suitable carriers or excipient(s). Techniques for formulation and administration of the compounds may be found in "Remington's Pharmaceutical Sciences," Mack Publishing Co., Easton, PA, 18th edition, 1990.

The pharmaceutical composition of the present invention may be manufactured in a manner that is itself known, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in a conventional manner using one or more pharmaceutically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as is suitable and understood in the art; *e.g.*, in Remington's Pharmaceutical Sciences above. In the present invention, the compounds of Formula I may be formulated into injectable and parenteral preparation depending upon intended purpose.

For infection, the agents of the present invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compound of the present invention to be formulated as tablet, pill, powder, granule, dragee, capsule, liquid, gel, syrup, slurry, suspension and the like, for oral ingestion by a patient. Preferred are capsule, tablet, pill, powder and granule, and capsule and tablet are particularly useful. Tablet and pill are preferably prepared in an enteric coating. Pharmaceutical preparation for oral use can be obtained by mixing one or more excipients with one or more compounds of the present invention, optionally grinding the resulting mixture, and processing the mixture of granules, after adding suitable auxiliaries, if desired, to obtain tablets or dragee cores. Suitable excipients may be fillers such as sugars, including lactose, sucrose, mannitol and sorbitol; and cellulose substances such as, for example, corn starch, wheat starch, rice starch, potato starch, gelatin, gum tragacanth, methyl cellulose, hydroxypropylmethyl-cellulose, sodium carboxymethyl cellulose, and/or polyvinylpyrrolidone (PVP). If desired, there may be added disintegrating agents such as cross-linked polyvinyl pyrrolidone, agar, or alginic acid or a salt thereof such as sodium alginate, lubricants such as magnesium stearate and carries such as binders.

Pharmaceutical preparations which can be used orally may include push-fit capsules made of gelatin, as well as soft, sealed capsules made of gelatin and a plasticizer, such as glycerol or sorbitol. The push-fit capsules can contain the active ingredients in admixture with fillers such as lactose, binders such as starches, and/or lubricants such as talc or magnesium stearate. In soft capsules, the active compounds may be dissolved or dispersed in suitable solvents, such as fatty acid, liquid paraffin, or liquid polyethylene glycols. In addition, stabilizers may also be added. All formulations for oral administration should be in dosage forms suitable for such administration.

The compounds may be formulated for parenteral administration by injection, e.g., by bolus injection or continuous infusion. Formulations for injection may be presented in unit dosage forms, *e.g.*, in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Alternatively, the active ingredient may be in powder form for combination with a suitable vehicle, *e.g*., sterile pyrogen-free water, before use.

The compounds may also be formulated in rectal compositions such as suppositories or retention enemas, e.g., containing conventional suppository bases such as cocoa butter or other glycerides.

Pharmaceutical compositions suitable for use in the present invention include compositions in which the active ingredients are contained in an amount effective to achieve its intended purpose. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

When the pharmaceutical composition of the present invention is formulated into a unit dosage form, the compound as the active ingredient is preferably contained in a unit dose of about 0.1 to 5,000 mg. The amount of the compound administered will be determined by the attending physician, depending upon body weight and age of patients being treated, characteristic nature and the severity of diseases. However, for adult patients, a dose of the active ingredient administered to the patient is typically within a range of about 1 to 1000 mg/kg BW/day, depending upon frequency and intensity of administration. For intramuscular or intravenous administration into adult patients, the total of about 1 to 500 mg per day as a single dose will be sufficient, but the use of a higher daily dose may be preferred for some patients.

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a graph showing changes in an intracellular NADH concentration by NQO1;
FIG. 2 is a bar graph showing an activity of NQO1 in the presence/absence of an NQO1-activating enzyme;
FIG. 3 is a view showing changes in an intracellular NAD(P)H fluorescence concentration in the presence/absence of an NQO1 activity;
FIG. 4 is a view showing changes in NAD(P)H fluorescence concentration of HEK cells in the presence/absence of an NQO1 gene;
FIG. 5 is a view showing changes in an intracellular NAD(P)H fluorescence concentration with treatment of dicoumarol;
FIG. 6 is a bar graph showing amount and activity of NQO1 measured in Lean and DIO mouse tissues,;
FIG. 7 is a bar graph showing changes in an intracellular ATP concentration by an NQO1 activator;
FIG. 8 is a graph showing changes in an intracellular energy concentration with time by NQO1;
FIG. 9 is a bar graph showing changes in AMP/ATP, NAD⁺/NADH and NADP⁺/NADPH ratios by an NQO1 activator *in vivo*;
FIGS. 10 and 11 are bar graphs showing changes in AMP/ATP and NAD⁺/NADH ratios with time after administration of an NQO1 activator to Lean and DIO mice, respectively;
FIG. 12 is a bar graph showing effects of NQO1 on β-oxidation of fatty acids in C57BL/6 mice;
FIG. 13 is a bar graph showing effects of an NQO1 activator on activities of ACC, CPT and HAD, and oxidation of Malanyl-CoA and ¹⁴C palmitoyl-CoA in DIO mice;
FIG. 14 is a photograph showing effects of NQO1 an regulation of AMPK and ACC phosphorylation in cells;
FIG. 15 is a photograph showing phosphorylation of AMPKα and ACC measured in liver, WAT, EDL and soleus of DIO mice;
FIG. 16 is a photograph showing effects of NQO1 on phosphorylation of AMPK;
FIG. 17 is a photograph showing effects of an NQO1 activator on phosphorylation of AMPKα and ACC;

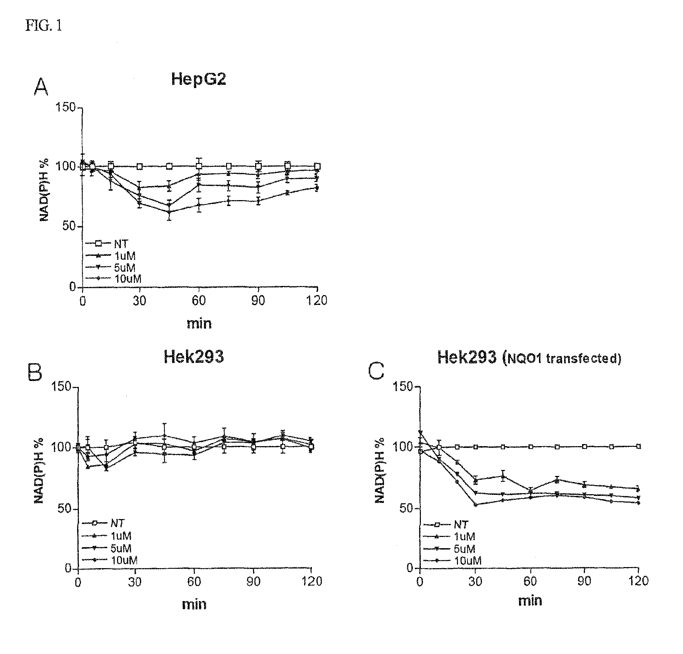

FIG. 1 is a bar graph showing effects of an NQO1 activator on activities of AMPK and ACC;

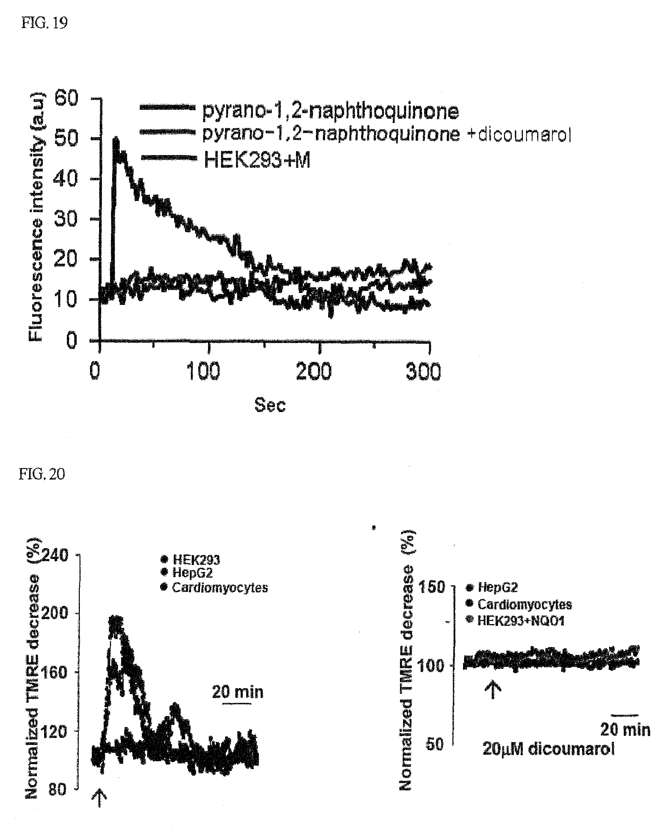

FIG. 19 is a graph showing effects of NQO1 on changes in an intracellular calcium concentration;
FIG. 20 is a graph showing real-time changes in mitochondrial membrane potential measured using TMRE fluorescence and conical microscopy;

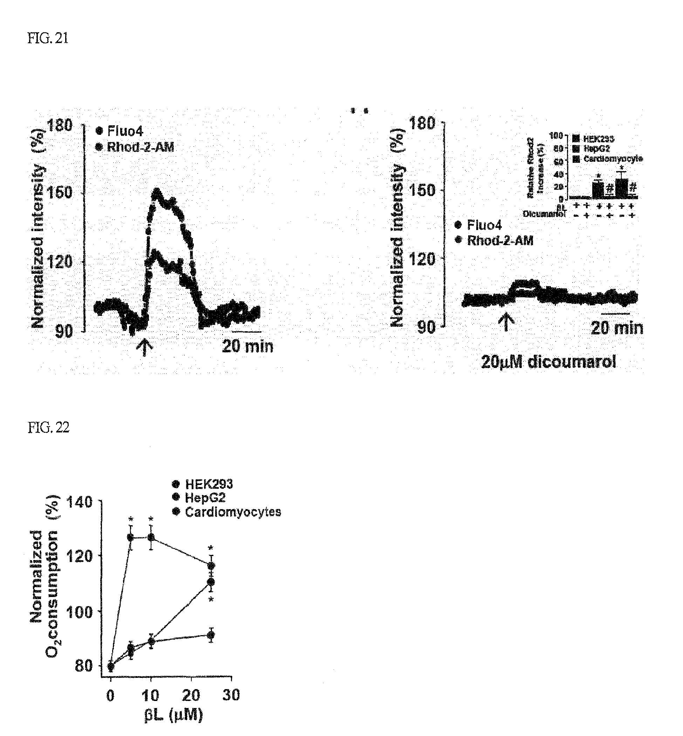

FIG. 21 is a graph showing measurement results of Fluo4 and Rhod-2-AM fluorescence, each representing a concentration of Ca²⁺ ions in cytoplasm ([Ca²⁺]c) and mitochondria ([Ca²⁺]m), respectively;
FIG. 22 is a graph showing monitoring results of mitochondrial oxygen consumption using a Clark-type oxygen electrode;

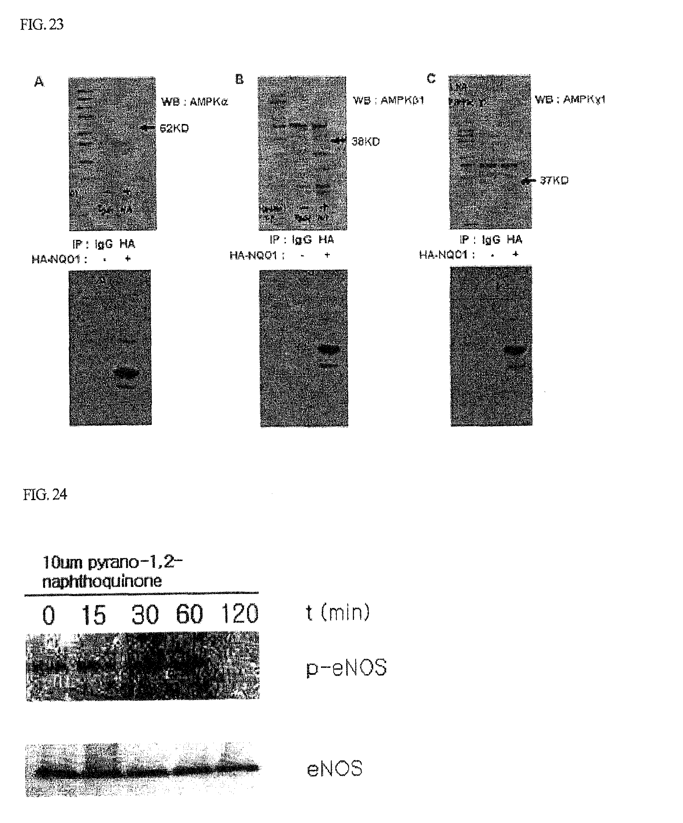

FIG. 23 is a photograph confirming binding between NQO1 and AMPKα, β or γ;
FIG. 24 is a photograph showing effects of an NQO1 activator on phosphorylation of endothelial nitric oxide synthase (eNOS);

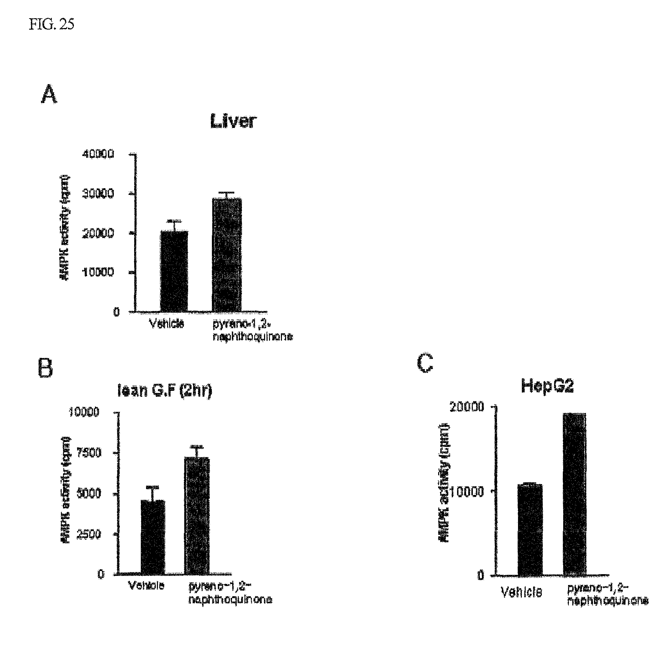

FIG. 25 is a bar graph showing effects of an NQO1 activator on activation of AMPK in C57BL/6 mice;

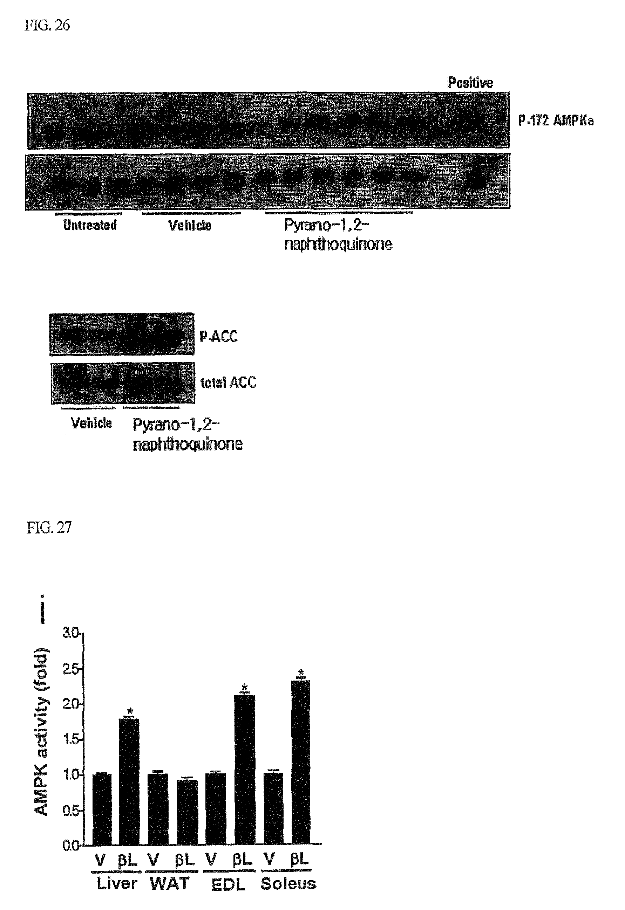

FIG. 26 is a photograph showing affects of an NQO1 activator on AMPK & ACC phosphorylation in C57BL/6 mice;
FIG. 27 is a bar graph showing effects of an NQO1 activator on AMPK activity in various organs of DIO mice;

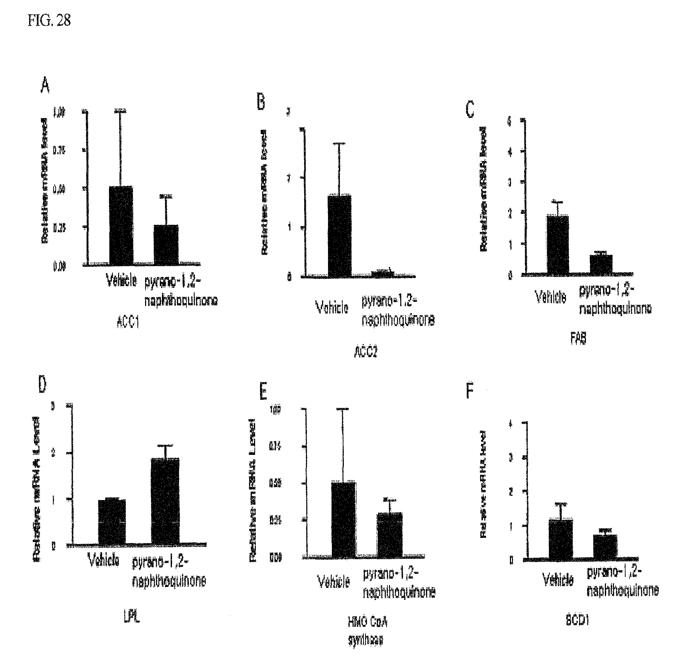

FIG. 28 is a bar graph showing effects of an NQO1 activator on transcript expression of proteins implicated in fat metabolism of C57BL/6 mice;

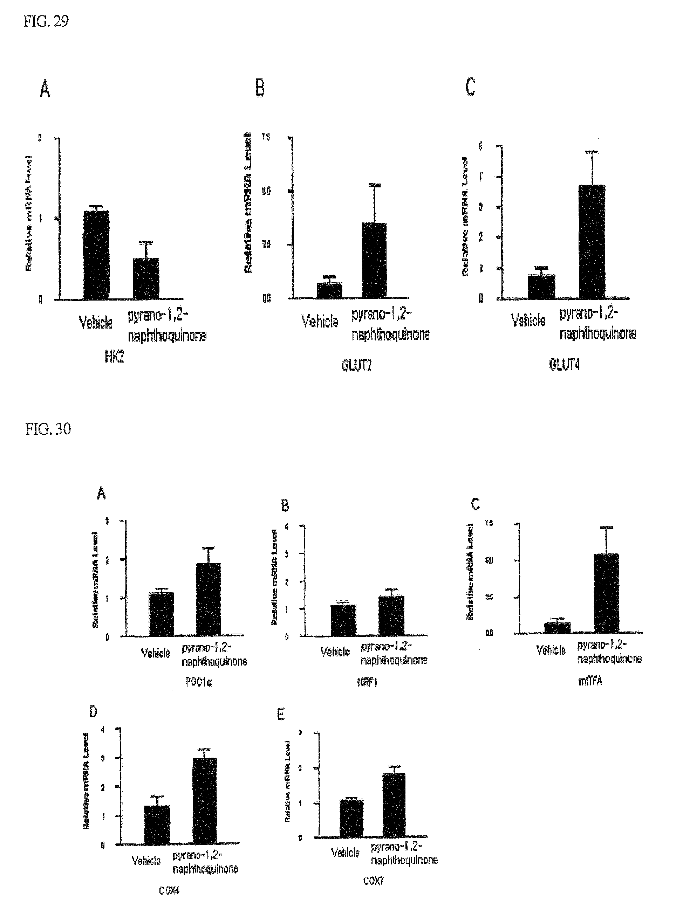

FIG. 29 is a bar graph showing effects of an NQO1 activator on transcript expression of proteins implicated in glucose metabolism of C57BL/6 mice;
FIG. 30 is a bar graph showing effects of an NQO1 activator on transcript expression of proteins implicated in mitochondrial biogenesis of C57BL/6 mice;

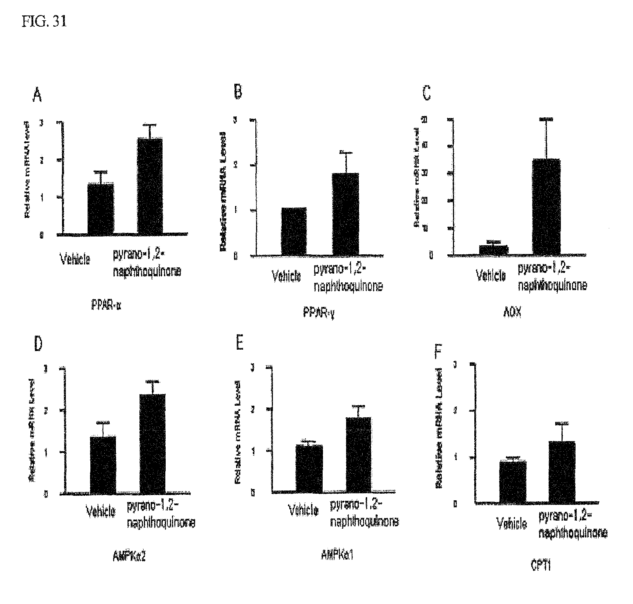

FIG. 31 is a bar graph showing effects of an NQO1 activator on transcript expression of proteins implicated in energy metabolism in C57BL/6 mice;

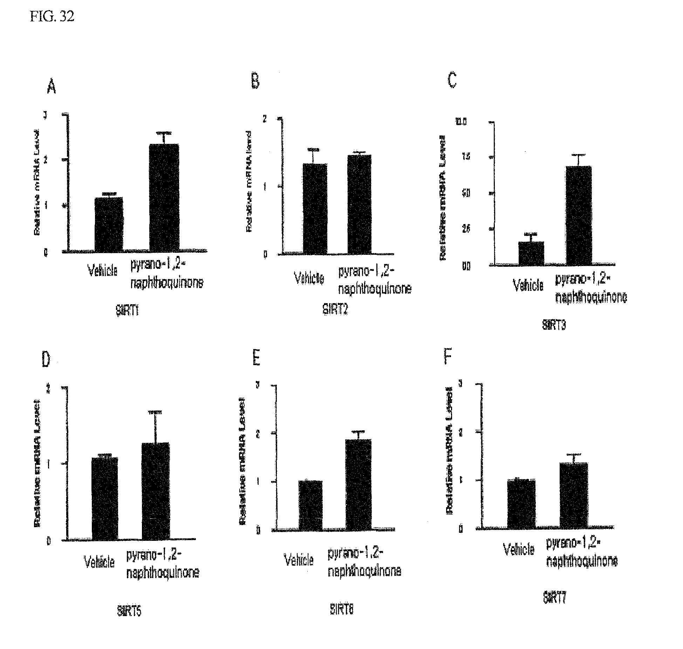

FIG. 32 is a bar graph showing effects of an NQO1 activator on transcript expression of SIRT-related proteins in C57BL/6 mice;

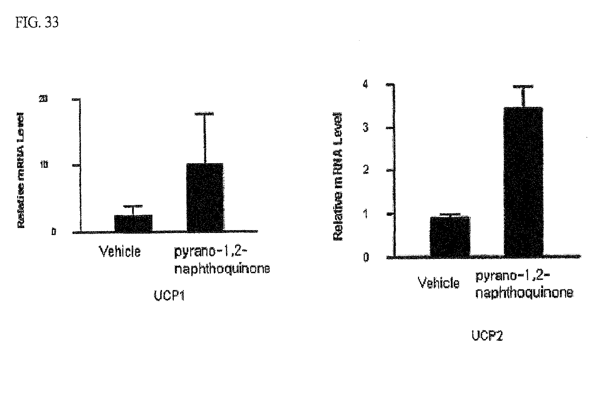

FIG. 33 is a bar graph showing effects of an NQO1 activator on transcript expression of UCP1 and UCP2 genes in C57BL/6 mice;

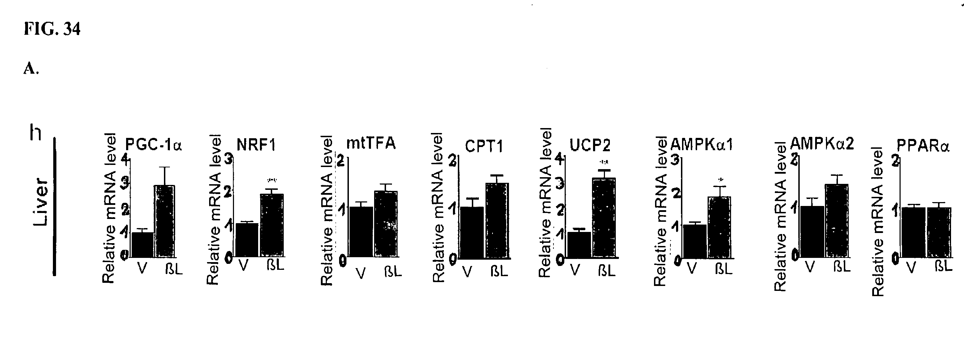

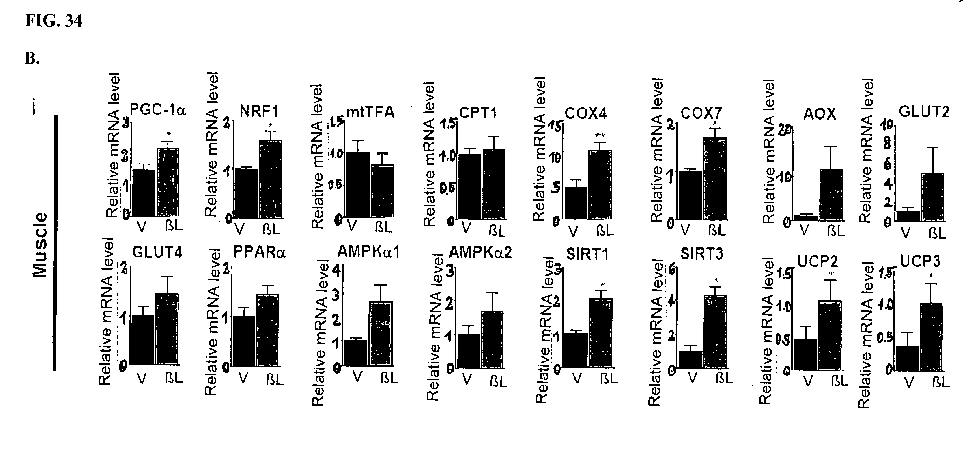

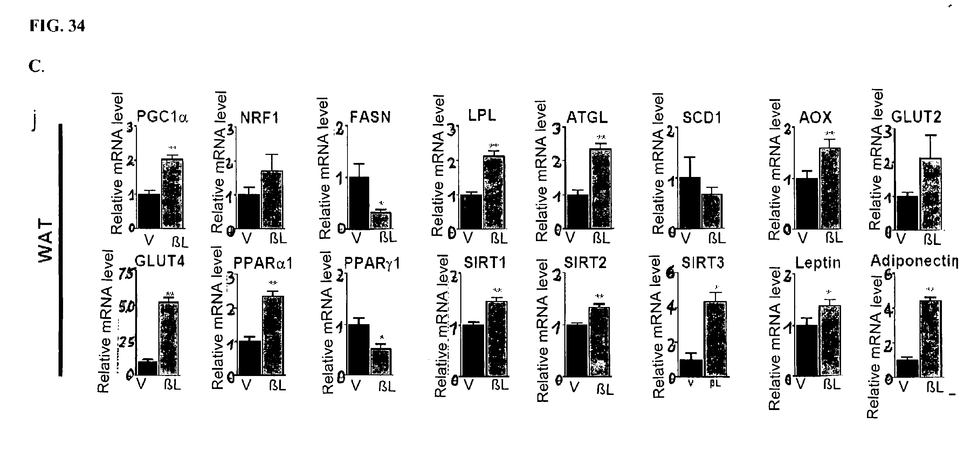

FIG. 34 is a bar graph showing effects of an NQO1 activator on gene expression rate of indicated genes in various organs;

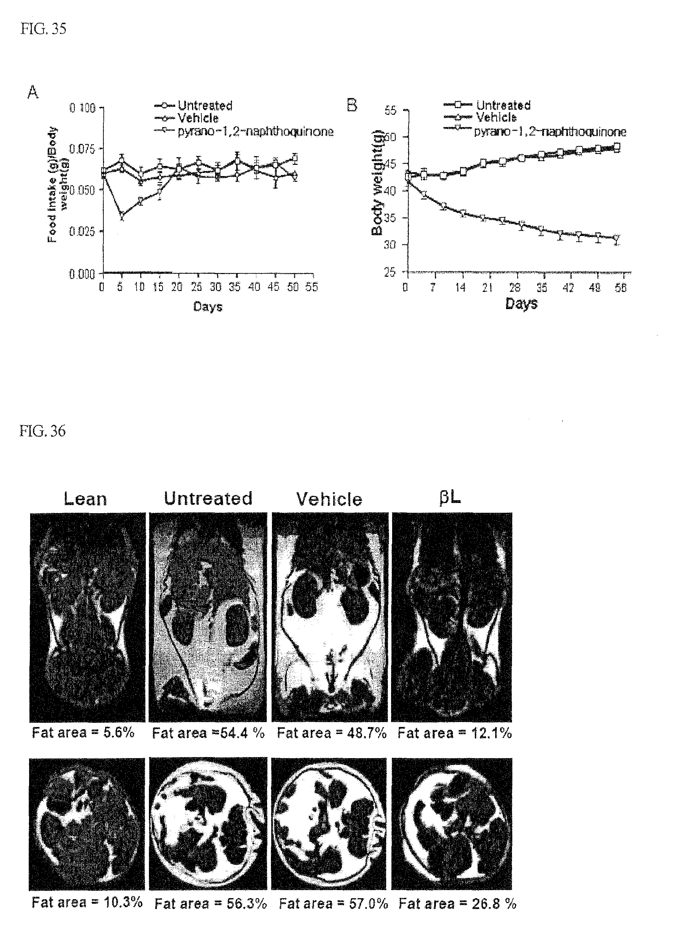

FIG. 35 is a graph showing changes in body weight and dietary intake over time, after administration of an NQO1 activator to C57BL/6 DIO mice;
FIG. 36 is MRI showing visceral fats of untreated Lean mouse group (n=5), untreated DIO mouse group (n=10), vehicle-treated DIO mouse group (n=10) and βL-treated DIO mice group (n=10) (laparotomized after 8 week-treatment);

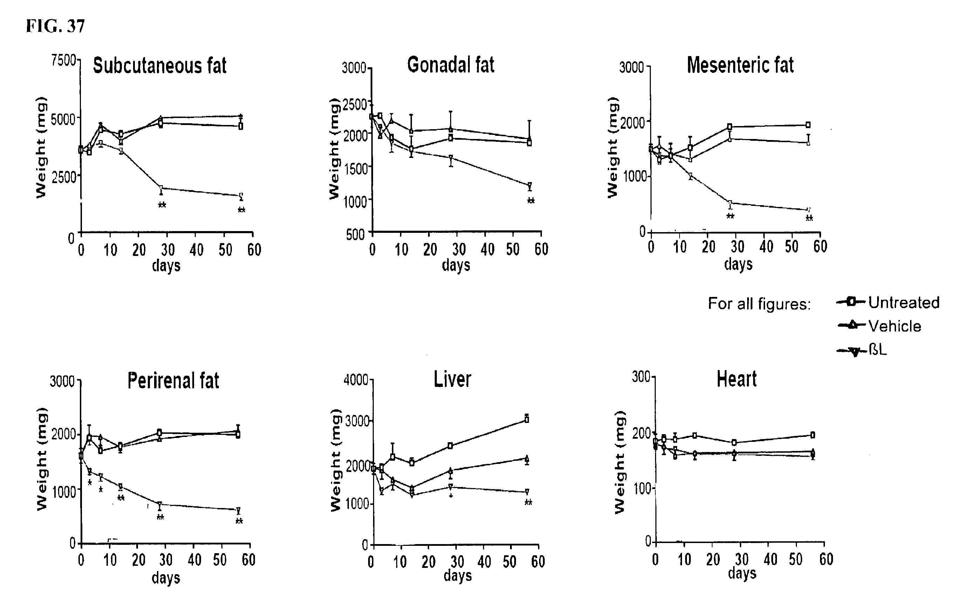

FIG. 37 is a graph comparing weight changes in various organs between the non-treatment group, the treatment group and control group after administration of an NQO1 activator to DIO mices

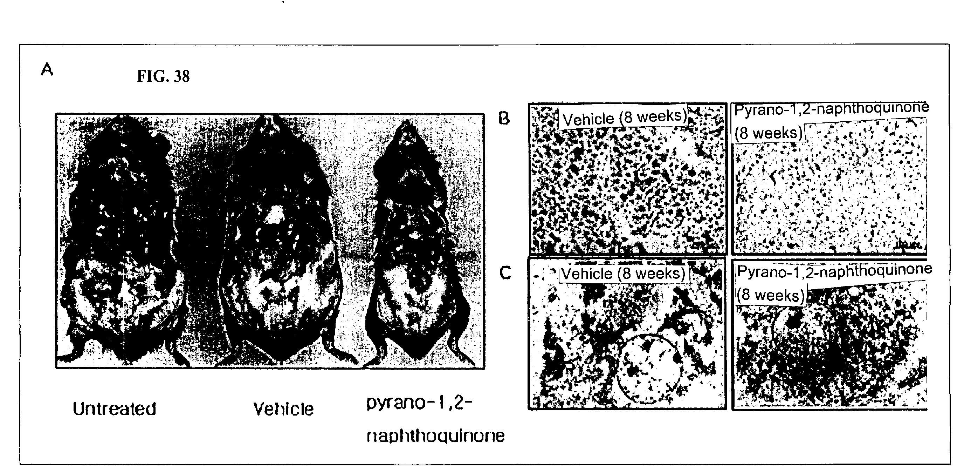

FIG. 38 is a photograph showing whole laparotomized states of animals after administration of an NQO1 activator to C57BL/6 DIO mice and results of oil red O staining and EM examination on fat accumulation in liver tissues;

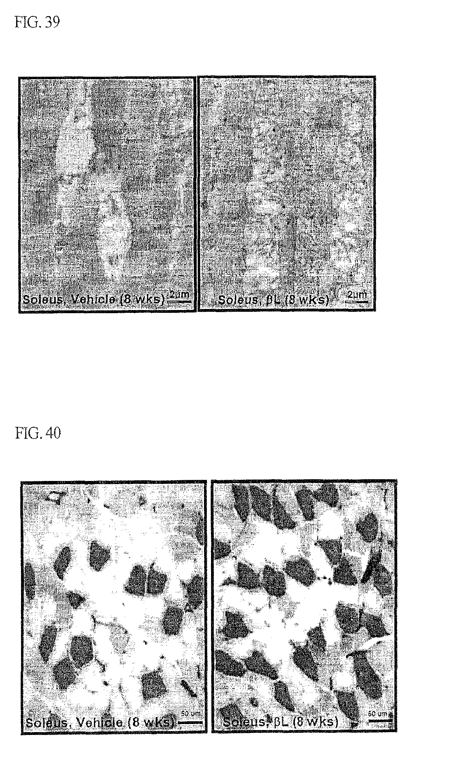

FIG. 39 is a micrograph showing results of EM examination and analysis on soleus muscle in vehicle-treated DIO mice and βL-treated DIO mice;
FIG. 40 is an EM of type 1 soleus muscle fibers in vehicle-treated DIO mice and βL-treated DIO mice;

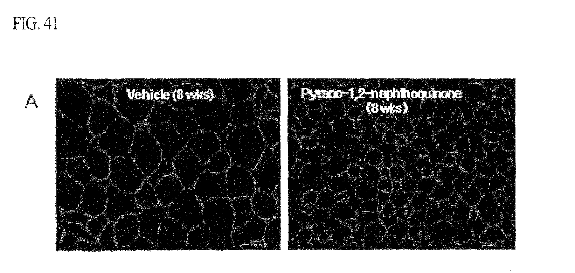

FIG. 41 is a photograph showing comparison results of the size of adipocytes in gonadal adipose tissues after administration of an NQO1 activator to C57BL/6 DIO mice;

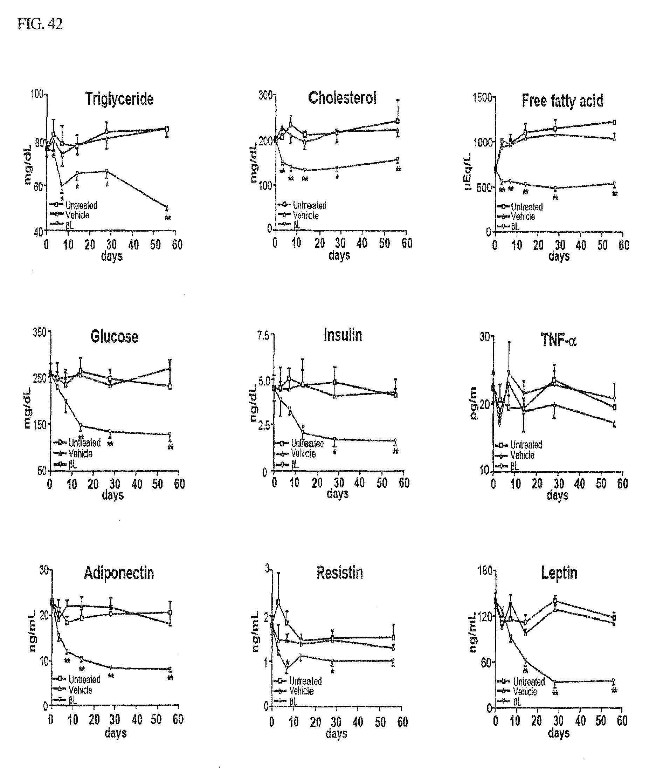

FIG. 42 is a graph showing effects of an NQO1 activator on metabolic parameters (TG, total cholesterol, free fatty acids, glucose, insulin, TNFα, adiponectin, resistin, leptin, and the like);

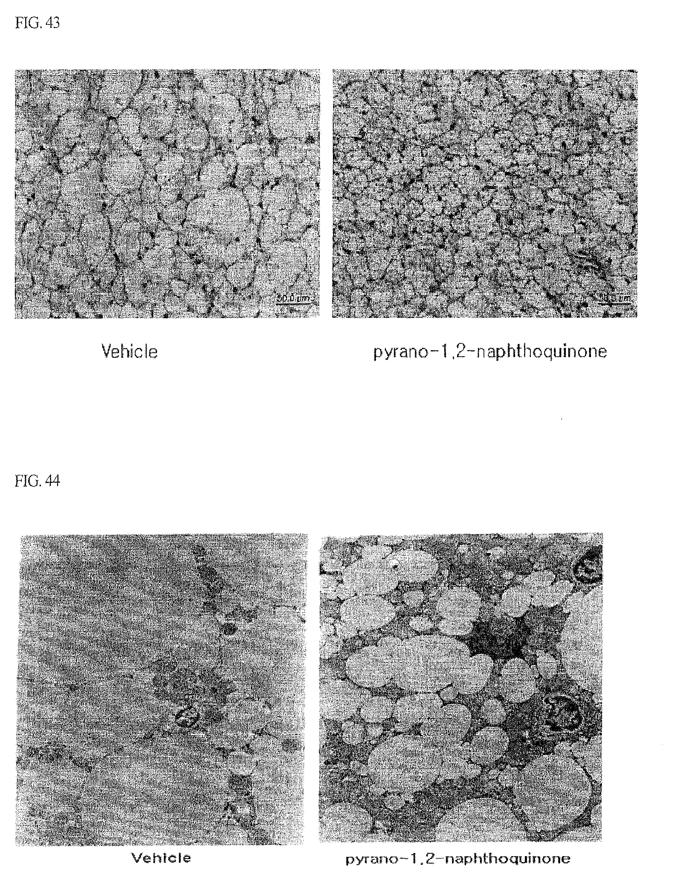

FIG. 43 is a photograph showing comparison results of H&E staining of brown adipose tissues aller administration of an NQO1 activator to C57BL/6 DIO mice;
FIG. 44 is an EM of brown adipose tissues after administration of an NQO1 activator to C57BL/6 DIO mice;

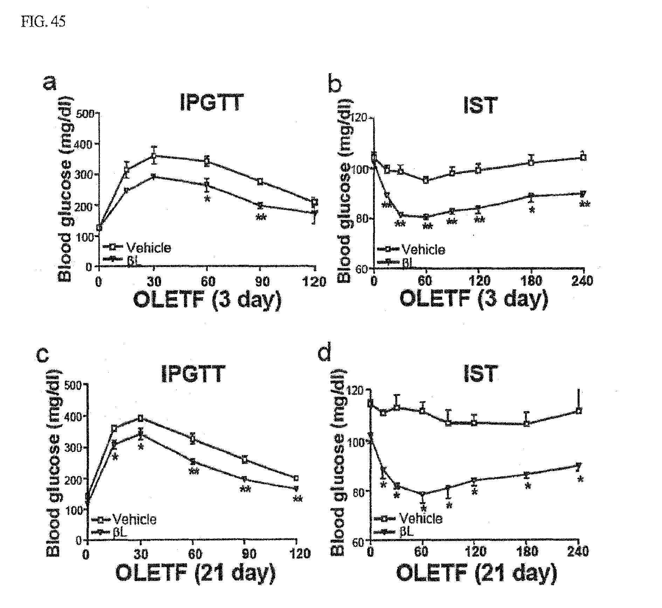

FIG. 45 is a graph showing glucose tolerance and insulin sensitivity in vehicle- or βL-treated OLETF rats;

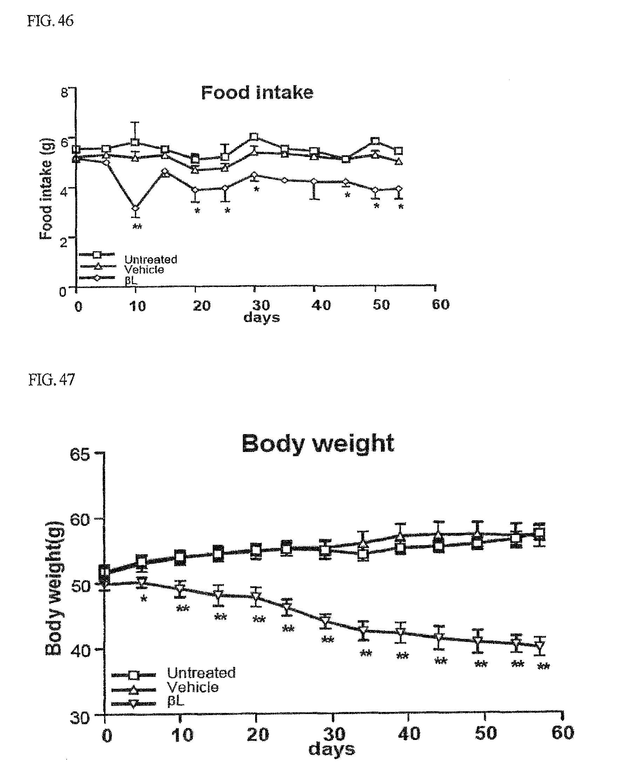

FIGS. 46 and 47 are graphs showing changes in dietary intake/body weight and body weight after treatment of a vehicle or βL on ob/ob mice;

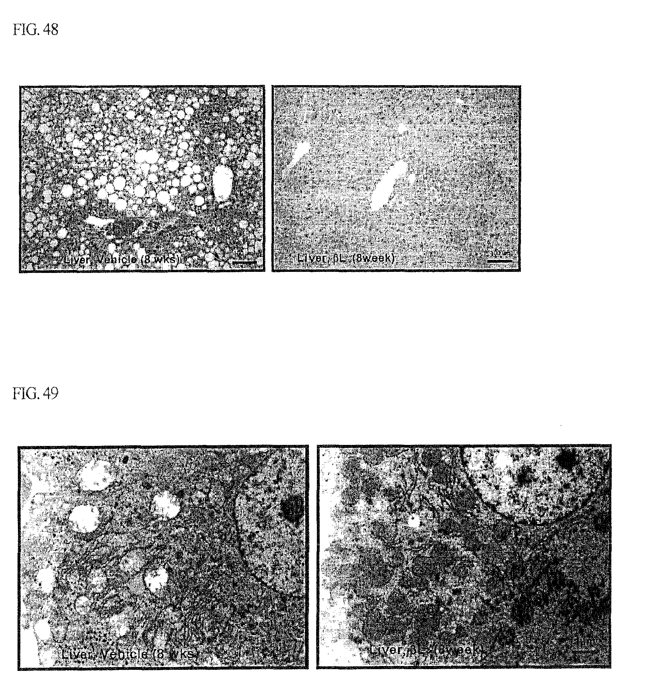

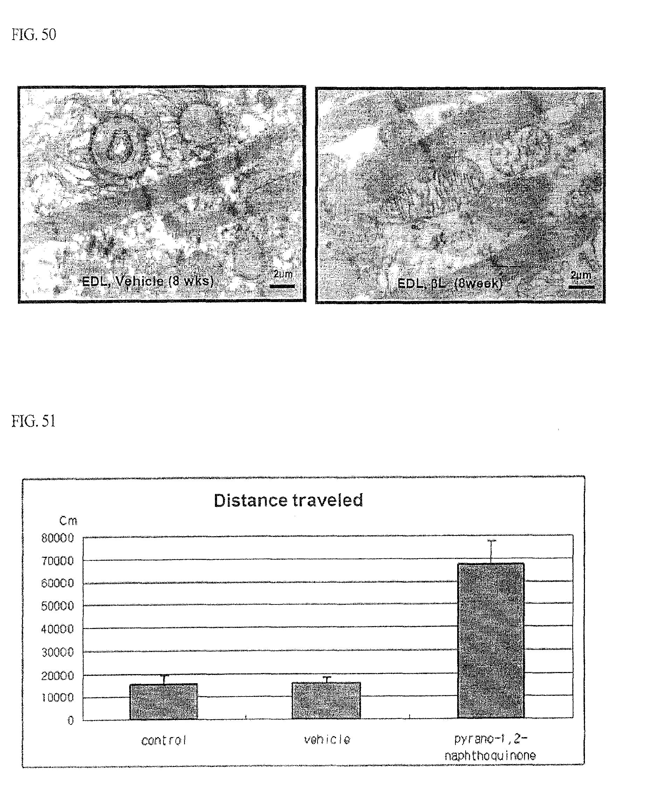

FIGS. 48 to 50 are EMs showing fat accumulation amounts and tissues after treatment of a vehicle or βL on ob/ob mice. FIG. 48: micrograph of hematoxylin and eosin-stained liver tissues (Scale bar, 100 µm), FIG. 49: micrograph showing TEM analysis of the liver tissues (Scale bar, 5 µm), and FIG. 50: micrograph showing TEM analysis of EDL muscle tissues (Scale bar, 20 µm);
FIG. 51 is a graph showing effects of an NQO1 activator on spontaneous locomotor activity after administration of the NQO1 activator to C57BL/6 DIO mice;

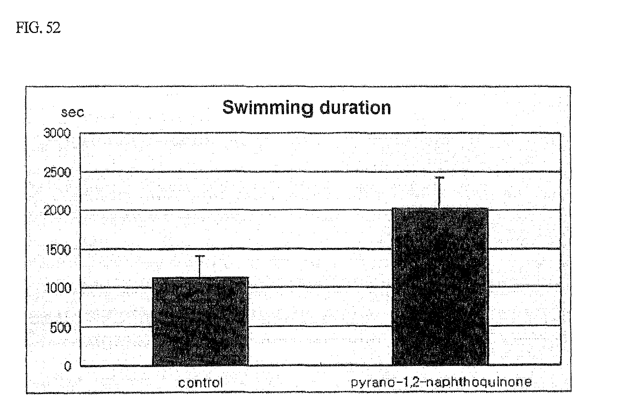

FIG. 52 is a graph showing effects of an NQO1 activator on enhancement of physical endurance after administration of the NQO1 activator to C57BL/6 DIO mice;

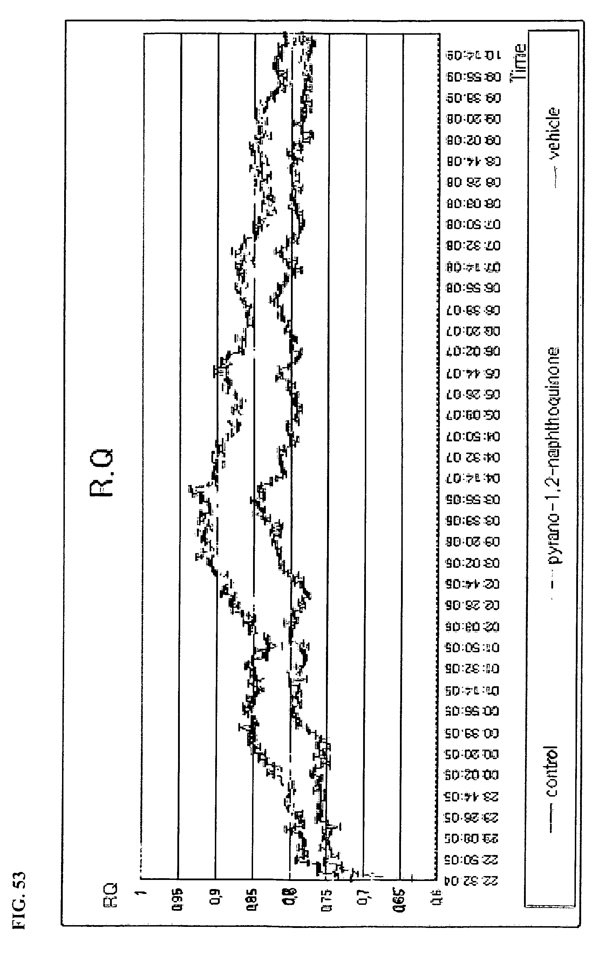

FIG. 53 is a graph showing effects of an NQO1 activator on Respiratory Quotient (RQ) after administration of the NQO1 activator to C57BL/6 DIO mice;

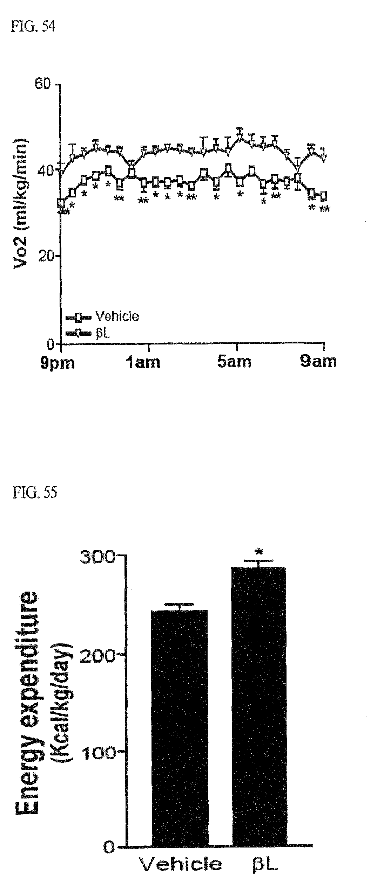

FIGS. 54 and 55 are graphs showing measurement results of VO₂ and energy consumption in vehicle- or βL-treated animals by indirect calorimetry;

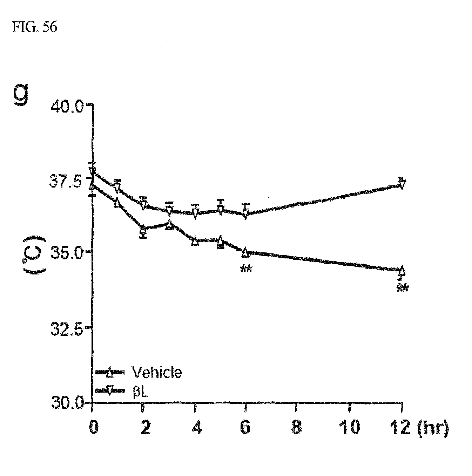

FIG. 56 is a graph showing a mean body temperature measured in vehicle- or βL-treated animals;

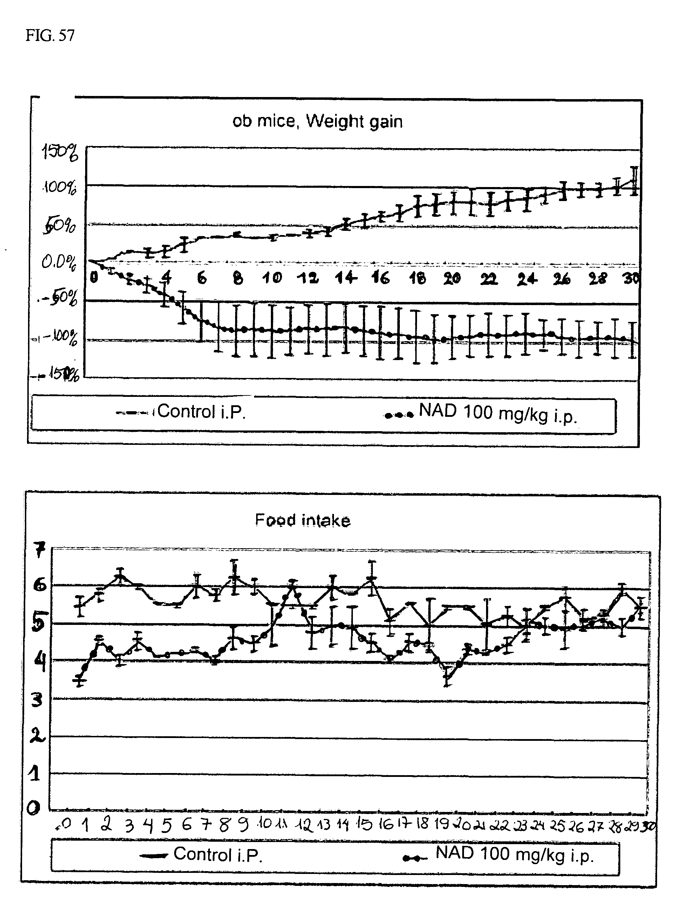

FIG. 57 is graph showing changes in body weight and dietary intake, after administration of NAD⁺ to leptin receptor-deficient ob/ob mice; and

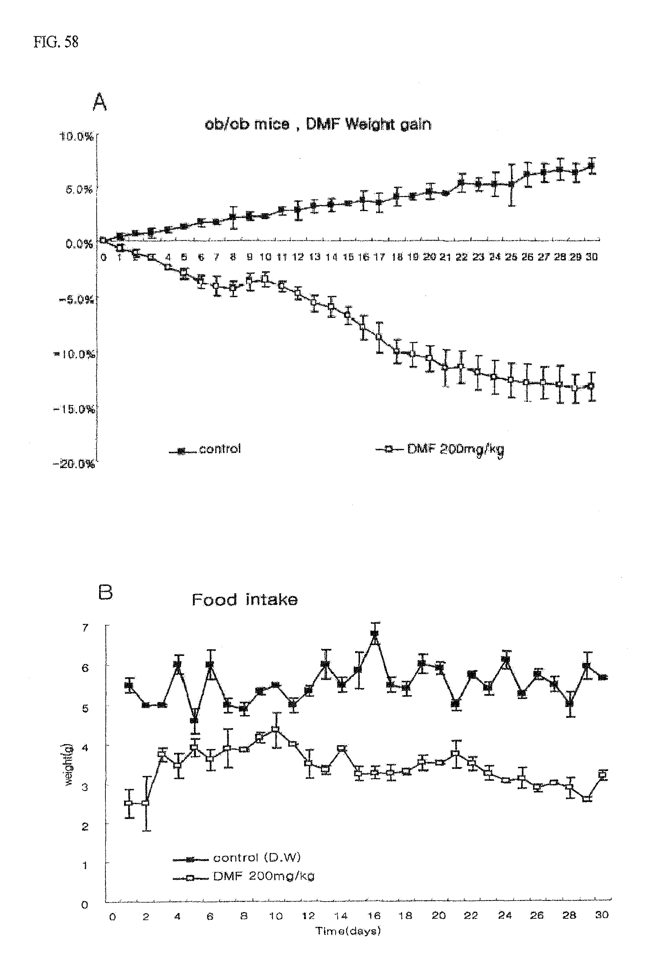

FIG. 58 is graph showing changes in body weight and dietary intake, after administration of dimethylfumarate to leptin receptor-deficient ob/ob mice.

### EXAMPLES

Now, the present invention will be described in more detail with reference to the following Examples. These examples are provided only for illustrating the present invention and should not be construed as limiting the scope and spirit of the present invention.

### Materials and Methods

Hereinafter, materials and methods used in the present invention will be given as follows.

### (1) Material

Culture media, cell culture reagents and materials used in experiments were purchased from Life Technologies, Inc., Sigma, and Fisher Scientific, respectively. NQO1 antibodies were purchased from Santa Cruz, ACC, pS79ACC, AMPK, pT172AMPK and HA antibodies were purchased from New England Biolabs, Fluo-4 was purchased from Molecular Probe, and Cell Counting Kit-8 (CCK-8 kit) was purchased from Dojindo Laboratories. 14C-palmitic acid was purchased from Perkin Elmer. Actin antibodies and other reagents including NAD⁺, NADH and hrNQO1 were purchased from Sigma.

### (2) Plasmids

Plasmids used in this experiment were pSG5alpha-HA(Hemaglutinin)-NQO1(NAD(P)H dehydrogenase) and pSG5alpha-HA-NQO1-C609T(1).

### (3) Cell culture

HEK293, MCP-7, MN9D and MN9X cells were cultured and grown in DMEM supplemented with 10% fetal bovine serum (FBS) at 37°C under 5% CO₂. Human hepatoma cell line, HepG2 was grown in an RPMI 1640 medium supplemented with 10% FBS. These culture media contained 100 U/mL of penicillin and 100 µg/mL of streptomycin.

### (4) Transfection

Transfection of plasmids into cells was carried out using a LipofectAMINE Plus reagent (Invitrogen, San Diego, CA). First, the plasmids were mixed and reacted with the Plus reagent for 15 min, and then the reaction mixture was reacted for another 15 min with addition of LipofectAMINE. The resulting reaction products were treated on the cells which were then maintained for 3 hours. Thereafter, the culture medium was replaced with a serum-containing medium and the cells were further grown for 24 hours.

### (5) Immunoblotting

Cells were lysed in SDS sample buffer (62.5 mM Tris-HCl (pH 6.8), 6% (w/v) SDS, 30% glycerol, 125 mM DTT, and 0.03% (w/v) bromophenol blue). The total cell lysates were boiled at a temperature of 100°C for 5 min and electrophoresed on a sodium dodecyl sulfate-polyacrylamide gel, and proteins were transferred on a nitrocellulose membrane. The membrane was reacted in TBS (5% (w/v) milk and 0.1% Tween 20) for 1 hour, and then reacted with primary antibodies for 2 hours. Next, the membrane was developed using ERP-conjugated secondary antibodies (Phototope-HRP Western Blot Detection Kit, New England Biolabs, Beverly, MA).

### (6) Calcium influx

Cells were grown on a coverslip in a culture medium for 24 hours, maintained in an RPMI 1640 serum-free medium for 1 hour, and pretreated with a calcium indicator Fluo-4 (5 µM) for 30 min. After pretreatment, the coverslip was mounted on a slide glass. Changes in an intracellular calcium concentration were observed using a fluorescence microscope (Carl Zeiss) for 30 sec, and cells were treated with a candidate compound (*e.g*. pyrano-1,2-naphthoquinone) simultaneously with consecutive imaging of calcium changes using a fluorescence microscope (Carl Zeiss) for 2 sec. Dicoumarol was pretreated in conjunction with Fluo-4 (5 µM) for 30 min, and the coverslip was mounted on a slide glass. Changes in the intracellular calcium concentration were observed using a fluorescence microscope (Carl Zeiss) for 30 sec, and cells were treated with pyrano-1,2-naphthoquinone (10 µM), followed by continuous shooting of calcium changes using a fluorescence microscope for 2 sec. Using an LSM Image Browser program (Carl Zeiss), 300 images taken for 10 min every 2 seconds were sorted and ordered in a time sequence and plotted on a graph.

### (7) Detection and confirmation of increases in NAD⁺ production via NQO1 (Confirmation of NADH decrease)

It may be possible to quantify NAD⁺ and NADPH in mouse tissues (liver and muscle) using HPLC-MS (High Performance Liquid Chromatography-Mass Spectroscopy), whereas quantification of NADH via cell culture is not easy due to a very low level of NADH (does not fall within detection range). Therefor, relative comparison was carried out using a Cell Counting Kit-8 (CCK-8).
**1) LC-MS**: Samples were analyzed using an Agilent 1100 series LC/MSD system equipped with G1322A degasser, G1312A binary pump, G1315A photo-diode-array detector, 59987A electrospray interface and 5989B mass spectrometer. ES-MS analysis was performed using an Agilent 5989 electrospray (ES) mass spectrometer (MS) with an Agilent Atmospheric Pressure ionization (API) internee fitted with a hexapole ion guide. Chromatogram was automatically integrated with the aid of Chemstation software, and concentrations were determined using a standard calibration curve.
   **1-1) Extraction of adenine and oxidized pyridine nucleotide (AOPN) from cells and liver tissues**: Acid extraction was used for analysis of an AOPN concentration in the cells and liver tissues. 400 µℓ of 6% HClO₄ was added to the cells cultured in a 100 mm dish, whereas a two-fold volume of 6% HClO₄ was added to the liver tissues which were then immediately homogenized. Each mixture was centrifuged at 14,000 g and 4°C for 10 min, and the resulting supernatants were neutralized by addition of 1 M borate buffer (pH 11) containing 4 mM EDTA in an amount of 75%. Samples were aliquoted and stored at a temperature of -80°C.
   **1-2) Extraction of reduced pyridine nucleotide (RPN) from liver tissues**: Analysis of an RPN concentration in liver tissues was earned out using alkali extraction. An equal volume of ice-cold 0.5 M KOH was added to the liver tissues which were then immediately homogenized. Thereafter, a two-fold volume of ice-cold distilled water was added and mixed with the liver tissues for 2 min, and the mixture was centrifuged using an Amicon ultrafiltration membrane (MlLLIPORE) at 14,000 g and 4°C for 40 min. The thus-obtained filtrate was neutralized by addition of 1 M KH₂PO₄(pH 6.5) in an amount of 10%. Samples were aliquoted and stored at a temperature of -70°C. Concentration determination was made within 72 hours.
   **1-3) Chromatography analysis conditions:** A mobile phase was 100 mM ammonium acetate:MeOH (98:2, v/v), and preparations were filtered through, a 0.22-µm Millipore filter. A flow rate was set to 0.8 mL/min and detection was made at 254 nm. Separation was carried out using a ZORBAX Bonus-RP C18 column (3.5 µm, 4.6 x 150 mm I.D., Waters, USA). The samples were maintained at 4°C in a thermostatted autosampler until they were loaded onto the column.
**2) Determination of intracellular NAD(P)H using a water-soluble tetrazolium salt-8 (WST-8) in living cells:** A water-soluble tetrazolium salt was used to monitor the amount of NAD(P)H through its reduction to a yellow colored formazan dye. The total amount of NAD(P)H within viable cells in the medium was determined periodically by a spectrophotometer. Cells were seeded in 96-well plates (2X10⁴ cells/well) and were cultured in 100 µℓ of a medium containing fetal bovine serum (FBS) and antibiotics for 24 hours. Immediately after treatment of the cells with a candidate compound (e.g., pyrano-1,2-naphthoquinone) at indicated concentrations and a 1/10 volume of a CCK-8 solution (Dojindo Molecular Technology), absorbance measurements were carried out using a spectrophotometer. The CCK-8 solution consisted of a water-soluble 2-(2-methoxy-4-nitrophenyl)-3-(4-nitrophenyl)-5-(2,4-disulfophenyl)-2H-tetrazolium monosodium salt (WST-8, 5 mM) and 1-methoxy-5-methylphenazinium methylsulfate (1-methoxy PMS, 0.2 mM) as an electron mediator. WST-8 produced a water-soluble yellow colored formazan dye through its bioreduction either in the presence of an electron carrier, 1-methoxy PMS and NAD(P)H or directly by NAD(P)H. Therefore, the reduction in tetrazolium salts mostly depends on the amount of intracellular NAD(P)H. The amount of formazan dye produced by the living cells in the medium was determined periodically at a wavelength of 450 nm (reference filter: 650 nm) by a spectrophotometer and compared to the values of control. In addition, a medium blank was prepared with only medium and physiological saline.

### (8) Separation of S9 supernatant

Various tissues (liver, brain, muscle, lung, kidney, and the like) were removed from DIO (diet induced obesity) mice and 4-fold volume of 50 mM Tris (pH 7.4) buffer containing 0.25 M sucrose and a protease inhibitor cocktail (Roche) was added to homogenize the tissues. The hamogenizates were subjected to ultracentrifugation at 105,000 g and 4°C for 1 hour to obtain cytosolic fractions. As storage of the supernatant leads to a decrease in NQO1 activity, it is preferred to use immediately the supernatant after preparation thereof. S9 supernatants for NADH recycling assay were aliquoted and stored at -80°C until use thereof and determination was made within 72 hours.

### (9) Determination of NQO1 activity

An enzyme reaction solution contained 25 mM Tris/HCl (pH 7.4), 0.2 mg/mL bovine serum albumin (BSA), 200 µM NADH (electron donor), 50 µM 2,6-dichlorophenolindophenol (DCPIP, electron acceptor) and 10 µg of S9 protein. Dicoumarol-sensitive NQO1 activity was measured in the presence/absence of 10 µM dicoumarol. The enzymatic reaction was initiated with addition of the S9 supernatant and was carried out at 37°C. The reaction rate was determined by observing a decrease in absorbance due to reduction of DCPIP at 600 nm for 10 min. For determination of the NQO1 activity, dicoumarol-sensitive NQO1 activity was measured based on a difference of the reaction rate in the presence/absence of 10 µM dicoumarol.

### (10) NAD(P)H recycling assay

An NADH recycling assay was carried out using the above-extracted S9 supernatant or human recombinant NQO1 (hrNQO1). 20 µg of the S9 supernatant or 0.5 unit of hrNQO1 in function with 200 µM NAD(P)H were added to a 25 mM Tris/HCl (pH 7.4) solution containing 0.2 mg/mL bovine serum albumin. The enzymatic reaction was initiated with addition of a 200 µM candidate compound and absorbance changes were monitored and recorded at 340 nm for 10 min. Like the NQO1 activity assay, NAD(P)H recycling degree can be calculated based on a difference of the reaction rate in the presence/absence of 10 µM dicoumarol (NQO1 inhibitor).

### (11) Beta-oxidation assay

**1) Preparation of 14C-palmitic acid injection:** 0.02 N NaOH was added to 1-14C palmitic acid dissolved in 100% EtOH, which was then dried. The thus-powdered sodium palmitate was dissolved and homogeneously mixed in 0.1% BSA/PBS and warmed to 40°C.
**2) Injection of albumin-bound 14C-palmitic acid:** The injection solution prepared in Section 1 was injected into tail veins of DIO mice at a dose of 10 microCi/150 µℓ/head, and the liver was removed 10 min later.
**3) Fat extraction from liver tissues:** The removed liver was immediately and rapidly frozen in liquid nitrogen and weighed. A 5-fold weight of a 5 mM Tris-HCl buffer solution containing 0.25 M sucrose, 5 mM MgCl₂, 1 mM mercaptoethanol and 0.5 mM EDTA was added to the liver tissues which were then homogenized. Equal amounts of each sample was taken and transferred to fresh 15 mL containers to which a 2.5-fold amount of C/M solution (chloroform:MetOH = 2:1) was added, respectively. The mixtures were thoroughly mixed using a homogenizer for 2 min, centrifuged at 2500 rpm for 20 min to separate chloroform layers that were then transferred to fresh containers. The remaining materials were thoroughly mixed with 2-fold amounts of the C/M solution for 2 min, and centrifuge to separate chloroform layers. This cycle was repeated 2-3 times. The thus-pooled chloroform layers were concentrated, followed by addition of 5 mM deoxycholate and transfer to scintillation vials. Then, more than 10-fold volumes of a toluene cocktail solution were added to chloroform layers of the vials and the mixtures were thoroughly mixed using a stirrer, and radioactivity was measured using a scintillation counter.

### (12) Light microscopic observation

The removed tissues were cut into a proper size, fixed in 10% formalin, washed with water, and embedded in paraffin to prepare blocks, according to a conventional method. The thus-prepared blocks were sectioned into a 4 µm thickness and subjected to hematoxylin and eosin (H&E) staining for observation of general changes.

### (13) Transmission electron microscopic observation

For observation of cellular microstructures, the removed tissues were sliced into a size of 1 mm³, pre-fixed in 2.5% glutaraldehyde (0.1M PBS, pH 7.4, 4°C) for 2 to 4 hours, washed, and post-fixed in a 1% osmium tetroxide solution for 2 hours. Then, according to a conventional method, the tissues were dehydrated with serial concentrations of ethanol and the solvent was replaced with propylene oxide. The tissues were embedded in an epoxy resin mixture, and the embedded tissues were subjected to thermal polymerization to prepare blocks. The thus-prepared blocks were sectioned into a thickness of 0.5 to 1 µm using a ultramicrotome and stained with toludine blue. Observation sites were selected under a light microscope and the tissues were ultrasectioned to a thickness of 60 to 70 nm. The ultrasectioned specimens were subjected to dual staining of uranyl acetate and lead citrate and were observed under a transmission electron microscope (H-600, Hitachi, Japan) at an accelerating voltage of 75 kV.

### (14) Oil Red O staining

Cryosectioned tissues were sectioned into a thickness of 5 µm, dried in air for 1 hour and reacted in a filtered 0.5% oil red O solution for 20 min. The tissue sections were washed with tap wafer, subjected to double staining with Gill hematoxylin for 20 to 30 sec, water-washed, and observed.

### (15) Perilipin staining

Adipose tissues were sectioned into a thickness of 3 µm, deparaffinized, exposed to antigens in a citrate buffer under high pressure for 4 min and washed. The mixture was reacted in a 3% hydrogen peroxide solution for 10 min, and the reaction product was washed three times with a TBST solution for 3 min. The product was reacted with primary antibodies (guinea pig, anti-perilipin) against 200-fold diluted perilipin for 1 hour and washed with a TBST solution. Secondary antibodies (donkey anti-guinea pig, cy3-conjugated) were added and reacted with the product for 30 min. The reaction product was washed three times with a TBST solution for 3 min, mounted with Fluormount G and then observed under a fluorescence microscope.

### (16) Oligonucleotide sequence

| Gene symbol | Direction | oligo sequence | Tm(°C) |
|---|---|---|---|
| mPGC1-alpha | Forward | CGATGTGTCGCCTTCTTGCT | 57 |
| | Reverse | CGAGAGCGCATCCTTTGG | |
| mAMPK alpha1 | Forward | TTCCGAAGTATCTCTTTCCTGAG | 55 |
| | Reverse | ACGCAAATAATAGGGGTTTACAA | |
| mAMPk alpha2 | Forward | AGGAAGTGTGTGAGAAATTCGAG | 57 |
| | Reverse | CCAGGTAAAGCTGTAAGCTCATT | |
| mGLUT2 | Forward | GCTGTACTGAGTTCCTTCCAGTT | 57 |
| | Reverse | GTCCTGAAATTAGCCCACAATAC | |
| mHK2 | Forward | CGGATATTGAAGACGATAAGGAC | 57 |
| | Reverse | TTCATCCTTCTCTTAACCTCCAG | |
| mACC1 | Forward | TGCTGAGATTGAGGTAATGAAGA | 57 |
| | Reverse | ATACAGGACTGATGTGATGTTGC | |
| mACC2 | Forward | CAGCTGTCTGTCCTACAGAGGT | 59 |
| | Reverse | CAGGTCCAGTTTCTTGTGTTCTC | |
| mFAS | Forward | CCTGCTATCATCTGACTTCCTCT | 57 |
| | Reverse | AGGGTGGTTGTTAGAAAGATCAA | |
| mSCD1 | Forward | AGTACGTCTGGAGGAACATCATT | 57 |
| | Reverse | GCTTGTAGTACCTCCTCTGGAAC | |
| mHMG-CoA synthase | Forward | GTCTAATTTGATGCAGCTGTTTG | 53 |
| | Reverse | AAAGATCATGAAGCCAAAATCAT | |
| mLPL | Forward | AGGACACTTGTCATCTCATTCCT | 57 |
| | Reverse | AGACATCTACAAAATCAGCGTCA | |
| mCPT1 | Forward | TTACTTCAAGGTCTGGCTCTACC | 59 |
| | Reverse | CTCCTTTACAGTGTCCATCCTCT | |
| mAOX | Forward | TATGGTGTCGTACTTGAATGACC | 57 |
| | Reverse | GGGTCACATCCTTAAAGTCAAAG | |
| mUCP1 | Forward | GGGACCTACAATGCTTACAGAGT | 59 |
| | Reverse | GTACAATCCACTGTCTGTCTGGA | |
| mUCP2 | Forward | AGCCTACAGATGTGGTAAAGGTC | 57 |
| | Reverse | GCTCATAGGTGACAAACATCACT | |
| mtTFA | Forward | ATACCTTCGATTTTCCACAGAAC | 55 |
| | Reverse | TCATTTCATTGTCGTAACGAATC | |
| mPPAR gamma | Forward | ATCTTAACTGCCGGATCCAC | 55 |
| | Reverse | TGGTGATTTGTCCGTTGTCT | |
| mPPAR alpha | Forward | TGCAGACCTCAAATCTCTGG | 55 |
| | Reverse | TAGCCTTGGCAAATTCTGTG | |
| mGLUT4 | Forward | CAGCGAGTGACTGGAACACT | 57 |
| | Reverse | CAGCATAGCCCTTTTCCTTC | |
| mNRF1 | Forward | TGATGGAGAGGTGGAACAAA | 55 |
| | Reverse | GGTTTCCCCAGACAGGACTA | |
| mCOX4i1 | Forward | GACTACCCCTTGCCTGATGT | 55 |
| | Reverse | GCAGTGAAGCCAATGAAGAA | |
| mCOX7ah | Forward | GCTCTGGTCCGGTCTTTTAG | 56 |
| | Reverse | TCACTTCTTGTGGGGGAAG | |
| mSIRT1 | Forward | AGTAAGCGGCTTGAGGGTAA | 55 |
| | Reverse | AAATCCAGATCCTCCAGCAC | |
| mSIRT2 | Forward | CTTGCCAAGGAGCTCTATCC | 55 |
| | Reverse | ACGATATCGGGCTTTACCAC | |
| mSIRT3 | Forward | CACTACAGGCCCAATGTCAC | 55 |
| | Reverse | TCACAACGCCAGTACAGACA | |
| mSIRT4 | Forward | CGGTTCCATCTCGGTTATCT | 55 |
| | Reverse | CCACAATTCTCACCCAACAG | |
| mSIRT5 | Forward | CGATTCATTTCCCAGTTGTG | 55 |
| | Reverse | CTCCCTCCGGTAGTGGTAAA | |
| mSIRT6 | Forward | GGGAGCTGAGAGACACCATT | 55 |
| | Reverse | GTCTGCACATCACCTCATCC | |
| mSIRT7 | Forward | ACCTCCTGCATCCCTAACAG | 55 |
| | Reverse | AGAGGCGGGGATATTTCTTT | |
| ATGL | Forward | CGCCTTGCTGAGAATCACCAT | - |
| | Reverse | AGTGAGTGGCTGGTGAAAGGT | |
| Leptin | Forward | GGTGTGAAAGAACCTGAGCTGAGG | - |
| | Reverse | CAGTGGATGCTAATGTGCCCTG | |
| Adiponectin | Forward | TCTCCTGTTCCTCTTAATCCTGCC | - |
| | Reverse | CATCTCCTTTCTCTCCCTTCTCTCC | |

### (17) Construction of DIO mice via dietary intake and treatment of candidate compounds on mice

Animals were continuously fed high-fat diet pellets for 3 to 4 weeks, thereby establishing DIO mice. Mice were raised in a breeding room with a 12 h/12 h light/dark (L/ D) cycle. Animals were divided into three groups, *i.e.*, a group with administration of physiological saline (n = 3), a group with administration of a vehicle (n = 5) and a group with administration of 50 mg/kg of a candidate compound (n = 7). Samples were orally administered to animals every day. On Days 7, 28 and 56, each tissue was removed, frozen in liquid nitrogen, and stored at -80 °C for short and long periods of time.

### (18) Real-time quantitative PCR

On Days 7, 28 and 56, liver, adipose and muscle tissues were isolated from two groups of diet-induced DIO mice and total RNAs were extracted using Trizol. cDNAs were synthesized using 1 µg of total RNAs, and polymerase used for PCR was prepared using M-MLV polymerase according to a method instructed by the manufacture. Real-time quantitative PCR was carried out using the prepared cDNAs. PCR for each oligo was performed using Tm values given in Tables above.

### (19) cDNA microarray analysis

A cDNA microarray analysis was carried out using an affymetrix mouse genome 430A 2.0 array chip. Using trizol, about 13 µg of total RNA was extracted in a concentration of more than 1 µg/l µℓ from liver, adipose and muscle tissues of DIO mice on Days 7, 28 and 56, followed by a microarray analysis. Functional classification was made using NetAffx which is available from the Affymetrix website.

### (20) AMPK kinase assay

In order to examine an activity of AMPK kinase, tissues used in experiments were lysed in an AMPK reaction solution (20 mM HEPES-NaOH, pH 7.0, 0.4 mM dithiothreitol, and 0.01% Brij-35) to separate proteins. The proteins were bound with AMPK-recognizing antibodies for 2 hours. Thereafter, using protein A/G agarose, an AMPK protein was separated from the total proteins after reaction for 1 hour, and 7 µℓ of a SAMS substrate peptide and 10 µℓ of γ[32P]ATP (1 mCi/100 µℓ) were added thereto and reacted at 30°C for 15 min. The reaction product was spotted on a 2 cm X 2 cm P81 paper square, washed three times with 0.75% phosphoric acid for 5 min, 5 mL of scintillation cocktail in conjunction with the paper was placed in a vial, and counts per minute (CPM) were measured using a scintillation counter.

### (21) Animal model

All experimental animal procedures were performed according to a guideline of Institutional Animal Care and Use Committee, Chungnam National University School of Medicine. OLETF rats were purchased from Otsuka Research Institute. Male ob/ob and C57BL/6 mice were purchased from Jackson Laboratory. Animals were housed in a breeding room maintained at a constant temperature of 2°C and a 12 h/12 h light/dark (L/ D) cycle. 4-week-old C57BL/6 male mice were fed ad libitum high fat diet (HFD, 24% (w/w) and 45% calories as fat, Research Diets Inc., New Brunswick, NJ 08901, US) for 7 weeks. Mouse groups were divided into an untreated group, a vehicle-treated group (calcium silicate), a pair-fed group and a candidate compound-treated group (calcium silicate-coated βL nanoparticles). Body weight and dietary intake of animals were daily measured. Upon completion of experiments, some mice of each group were anesthetized and received MRI examinations. The remaining mice of the groups were anatomized and tissue weight thereof was measured. Epidydimal fat, liver, soleus muscle and extensor digitorum longus (EDL) were prepared, and immunohistochemical, oil-red O and pH-sensitive ATPase staining were performed for analysis.

### (22) Cells and Western blot analysis

HepG2 (human hepatoma cell line), HEK293 (Human embryonic kidney cell line), and C2C12/L6 myoblasts were purchased from American Type Culture Collection (ATCC, Manassas, Virginia, USA). LKB-/- MEFs were purchased from RA. DePinho. Rat ventricular myocytes were enzymatically isolated from the heart of adult mice by a known method. Phosphorylation of AMPK (Thr172) and ACC (Ser79) was measured by Western blot analysis using Cell Signaling Technologies and pan- and phospho-specific antibodies (Upstate Biotechnology, Inc., Lake Placid, N.Y.).

### (23) Fluorescence imaging

Experiments were carried out using a microfluorometric system including an inverted fluorescence microscope (Eclipse TE300, Nikon, Japan) equipped with dry-type fluorescence objective lens (x40, numerical aperture of 0.85), a photomultiplier tube (type R1527, Hamamatsu Photonics, Hamamatsu, Japan), and a Deltascan illuminator (Photon Technology International Inc., Lawrenceville, NJ).

In order to monitor monochromatic fluorescence (350 nm), light was illuminated using a 75 W xenon arc lamp directed via a 10 Hz chopper wheel; specific fluorescence intensity was measured at a wavelength of 405 ± 15 nm and 460 ± 10 nm. Even though autofluorescence at 460 nm may occur from unknown intracellular components or cytosolic NAD(P)H, most of the light detected by the method of the present invention was generated from mitochondrial NAD(P)H.

### (24) Determination of mitochondrial membrane potential

For determination of the inner mitochondrial membrane potential (ΔΨₘ), 20 nM of TMRE (tetramethylrhodamineethyl ester) as a fluorescent indicator was continuously applied to be loaded on cells. In order to monitor mitochondrial Ca²⁺, cells on glass coverslips were loaded with 10 µM Rhod-2 AM via cold loading/warm incubation. In order to monitor cytosolic Ca²⁺, cells were co-loaded with 10 µM. Fluo-4 AM at 37°C for 30 min. The cells were washed twice with a KB solution, and mounted in a perfusion chamber. Fluorescent imaging was taken using a confocal laser scanning microscope (LSM-510 META, Carl Zeiss) at a magnification of x200 and x400, in conjunction with appropriate laser lines and a filter set. Image analysis was carried out using LSM-510 META software (Zeiss). Images for TMRE (red fluorescence), Rhod-2 (red fluorescence) and Fluo-4 (green fluorescence) were obtained using an inverted Zeiss 510META laser-scanning confocal microscope equipped with x63 numerical aperture, 1.4 oil-immersion planapochromat lens. Green fluorescence and red fluorescence were illuminated with light of 488 nm and 543 nm. Incident light was passed through a 545 nm dichroic mirror and observed via a 500 to 530 nm bandpass (green) filter and a 560 nm long-pass (red) barrier filter.

### (25)_Enzyme assays

For Enzyme assays, cytoplasm was extracted from mouse tissues. Dicoumarol-sensitive NQO1 activity was determined by a conventional method known in the art: This method is based on an absorbance decrease at 600 nm due to bioreduction of DCPIP. Total AMPK activity was determined with a known method using synthetic "SAMS" peptides and [γ-³²P] ATP. ACC activity was determined by quantifying fixation of ¹⁴CO₂ to the acid-stable product in the presence/absence of citrate (2 mM) as an allosteric activator of ACC. CPT (carnitine palmitoyl transferase) activity was measured in L6 myoblasts and soleus muscle by a known method. HAD (3-hydroxyacyl-CoA dehydrogenase) activity was determined in L6 myoblasts and soleus muscle by monitoring conversion of acetoacetyl-CoA into L-3-hydroxybutyryl CoA and the concomitant oxidation of NADH into NAD⁺. The reaction was monitored at 340 nm by a known method.

### (26) Fatty acid oxidation

[¹⁴C]palmitoyl-CoA (Perkin Elmer) oxidation was determined in L6 myoblasts and soleus muscle by a known method using labeled CO₂ and 0.2 mL of benzethonium chloride solution.

### (27) Determination of Malonyl-CoA contents

Contents of Malonyl-CoA were determined by a known method, *e.g.* HPLC. Acyl-CoA esters were separated by HPLC using 0.2 M ammonium acetate-containing acetonitrile/water (1.75:98.25). Samples were washed with 20 min, and subjected to 100 min linear gradient elution from acetonitrile/water (10:90) containing 0.2 M ammonium acetate.

### (28) Physiological measurement

O₂ consumption of animals was recorded by a known method. For indirect calorimetry, mice were individually housed in calorimetry chambers (Oxymax OPTO-M3 system; Columbus instruments, Columbus, OH). Animals were allowed to acclimate to a new environment for 48 hours prior to the experiment, and amounts of O₂ consumption and CO₂ production were measured for 24 hours every 30 minutes. Mice were given free access to food and drinking water during the first 24h period, and animals were given access to drinking water only during the second 24h period. Blood samples were taken from a heparinized tube, immediately placed on ice, centrifuged and stored at -20°C for future use. Enzymatic colorimetry was employed for quantification of triglyceride (TG), total cholesterol, free fatty acids, and glucose (Beckman Instruments, CA). Plasma insulin (Linco Research, MO), TNFα (R&D System), adiponectin (Linco Research, MO), resistin (KOMED) and leptin (Linco Research, MO) were quantified by ELISA,

### (29) HPLC-MS/MS analysis

In order to assay ATP, ADP, AMP, NAD and NADP levels, 450 µℓ of 5 mM ammonium acetate was added to 50 µℓ of a liver sample extracted with perchloric acid (HClO₄) which was then diluted ten times. Diluted samples were vortexed and mixed for 1 min, and the mixture was filtered through a 0.2 µm nylon syringe filter (Whatman, Brentford, UK). Then, 5 µℓ of each aliquot was injected into a HPLC system. Each standard solution (50 µℓ) for these analytes was added to 50 µℓ of an extraction solution containing 2-chloroadenosine (internal standard) and 400 µℓ of 5 mM ammonium acetate, and mixed and filtered in the same manner as in the above samples. For analysis of NADH and NADPH, each 100 µℓ of liver samples extracted with a potassium hydroxide (KOH) solution was diluted twice with an equal amount of a 5 mM ammonium acetate (KOH solution for standards). HPLC system consists of an Agilent 1100 series vacuum degasser, a binary pump, an autosampler, a thermostatted column compartment, and a DAD detector (Agilent Technologies, Palo Alto, CA). Adenine and nucleotide analogues were separated by Waters XTerra MS C18 2.1 X 150 mm, 3.5 µm column (Waters, Milford, Massachusetts, USA) chromatography using a gradient method. A mobile phase for adenine and nucleotide analogues was 5 mM ammonium acetate (A) and methanol (B). NADH⁺-related compounds were separated by linear gradient elution of initial 98% (A) to final 70% (B) at a time point of 8 min. The mobile phase was developed at 70% (B) for 7 min. Finally, the mobile phase was returned to 98% (A) at a point of 16 min, and reequilibrated to 98% (A) for 12 min. A flow rate was set to 0.2 mL/min throughout the entire period of time. An injection volume was 5 µℓ. Electrospray-ionization mass spectrometry (ESI-MS) was carried out in a positive ion mode using MDS Sciex API 4000 Triple Quadrupole Mass Spectrometer (Applied Biosystems, Ontario, CA). Analytes were observed at unit resolution in the multiple reaction monitoring (MRM) mode monitoring the transition m/z: ATP, m/z 508 → 136; ADP, m/z 428 → 136; AMP, m/z 348 → 136; NAD, m/z 664 → 136; NADP, m/z 744 → 136; NADH, m/z 665 → 136; NADPH, m/z 745 → 136; and 2-chloroadenosine, m/z 302 → 170.

### (30) Glucose tolerance test and insulin stimulation test

Glucose tolerance test (GTT) was carried out for 12h-fasted mice by a known method. As described above, 12h-fasted mice received an insulin stimulation test.

### (31) DNA microarray and quantitative PCR

Adipose, liver or muscle tissues were pooled from male mice treated with a vehicle or a candidate compound for 4 weeks and the pooled tissues were subjected to microarray analysis. Total RNA was prepared from the tissues homogenized with a Trizol reagent (Invitrogen, Carlsbad, CA). Probes for the microarray analysis were prepared from 10 µg of the total RNA, and hybridized to Mouse 430A GeneChips (Affymetrix, Santa Clara, CA). The hybridized array was scanned, and raw data was extracted using Microarray Analysis Suite 5.0 (Affymetrix). For PCR quantitation, 1 µg of total RNA was reversely transcribed into cDNA using Superscript II and oligo(dT) primers. The constructed cDNA was amplified with the LightCycler FastStart DNA Master SYBR Green I kit and LightCycler, according to the manufacturer's instruction (Roche Diagnostics, Indianapolis, IN). Expression data were normalized to β-actin expression.

### (32) ICV cannulation and injection

Using a known method, 23-gauge stainless steel cannulae (Plastics One, Roanoke, VA) were implanted into the 3rd ventricle of mice (1.8 mm caudal to the bregma and 5.0 mm ventral to the sagittal sinus). After a 7-day recovery period and an overnight fast, mice were given intracerebroventricular (ICV) injection of a vehicle (0.2% DMSO) or a candidate compound at a dose of 2µℓ over 1 min. Dietary intake of animals was monitored for 7 hours after injection of the compounds, and the body weight of mice was measured 24 hours after injection of the compounds. Following independent evaluation, only results obtained from animals with correctly positioned cannula were included in the analysis.

### (33) Statistical analyses

The experimental results were expressed as mean±SD or mean±SEM (standard error of mean). Differences in statistical significance between individual groups were tested using a Student's t-test and analysis of variance (ANOVA). Dfferences in statistical significance were taken into consideration for P<0.05.

### (34) Other method

Concentrations of proteins were measured using a Bradford method (Bio-Rad). As a reference protein, bovine serum albumin (BSA) was used. All experiments were performed in triplicate.

### Example 1: Changes in intracellular NADH concentration by NQO1 activator

This experiment was intended to demonstrate that the reduction of an NQO1 activator, pyrano-1,2-naphthoquinone (hereinafter, often referred to as "βL"), occurs in an NQO1-dependent manner.

FIG. 1A shows an NQO1 activator concentration-dependent decrease of NADH upon real-time measurement of an intracellular NADH concentration after treatment of the human hepatoma cell line HepG2 having an NQO1 activity with the NQO1 activator pyrano-1,2-naphthoquinone for 2 hours.

FIG. 1B shows no change in an NADH concentration irrespective of an NQO1 activator concentration and a time period upon real-time measurement of an intracellular NADH concentration while treating HEK293 cells having no NQO1 activity with pyrano-1,2-naphthoquinone for 2 hours.

FIG. 1C shows an NQO1 activator concentration-dependent decrease of NADH upon real-time measurement of an intracellular NADH concentration while treating the cells with pyrano-1,2-naphthoquinone for 2 hours after insertion of an NQO1 gene into HEK293 cells having no NQO1 activity. This result demonstrates that the NADH decrease by pyrano-1,2-naphthoquinone is NQO1-dependent.

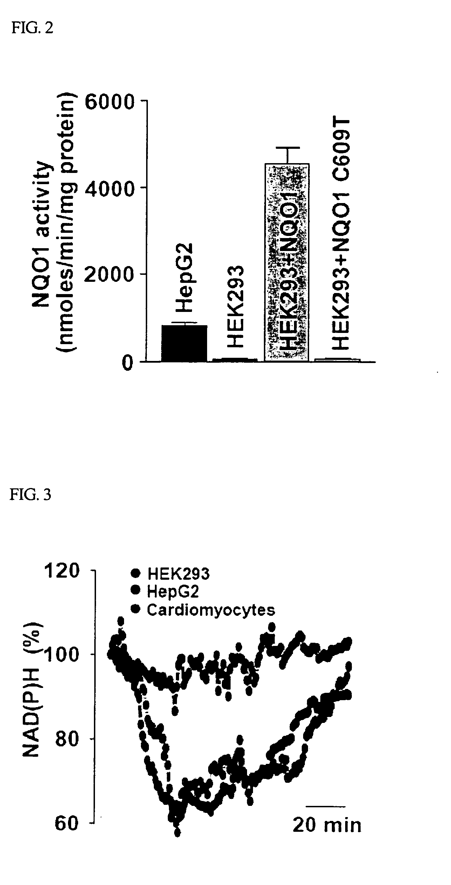

Specifically, the NQO1 activity and changes in the intracellular NAD(P)H fluorescence concentration in the presence/absence of NQO1 activity were measured in HepG2, HEK293 and pSG5 HA-NQO1 or pSG5 HA-NQO1 C609T-inserted HEK293 cells. The results showed that βL promoted NQO1-dependent NAD(P)H oxidation, thereby leading to an about 40% decrease of NAD(P)H in HepG2 cells having NQO1 activity and cardiomyocytes (see FIGS. 2 and 3).

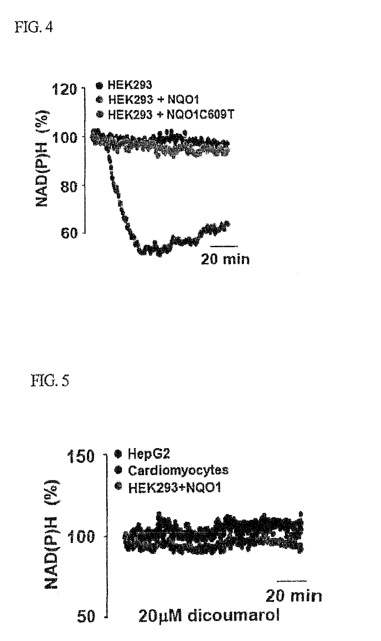

However, a control group of HEK293 cells without the NQO1 activity exhibited no such changes, HEK293 cells with insertion of a wild-type NQO1 gene exhibited βL-facilitated NAD(P)H oxidation, and HEK293 cells with insertion of a catalytically inactive NQO1C609T gene exhibited no oxidation of NAD(P)H (see FIG. 4). Further, βL-accelerated NAD(P)H oxidation was limited by the specific NQO1 inhibitor, dicoumarol (see FIG. 5).

### Example 2: Changes in amount and activity of NQO1 in tissues of Lean mice and DIO mice

In order to confirm distribution of NQO1 in tissues and cells, various tissues of mice were removed and ultracentrifuged, Using the purified cytosolic fractions, dicoumarol-sensitive NQO1 activity was measured based on differences in reaction rate with/without of 10 µM dicoumarol. An activity value of NQO1 was expressed as the reduced 2,6-dichlorophenolindophenol/min/mg proteins.

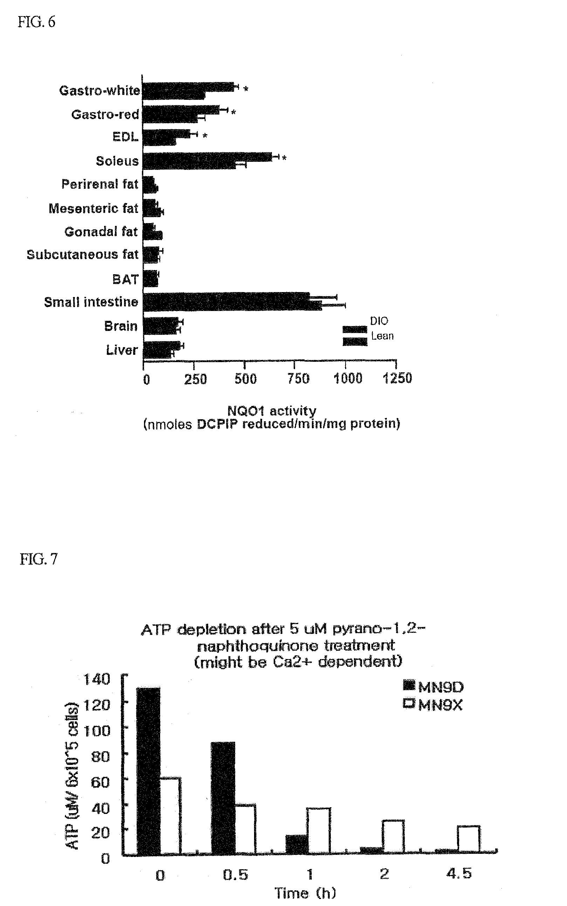

FIG. 6 shows the presence of NQO1 activity in various tissues of Lean and DIO mice. The NQO1 activity was compared between Lean and DIO mouse tissues. As shown in FIG. 6, the NQO1 activity was particularly higher in muscle and liver of DIO mice than Lean mice.

### Example 3: Changes in intracellular ATP concentration by NQO1 activator

FIG. 7 shows a sharp decrease in an intracellular ATP concentration of neuronal cells by pyrano-1,2-naphthoquinone. Dopaminergic (MN9D) and non-dopaminergic (MN9X) neuronal cells were treated with 5 µM pyrano-1,2-naphthoquinone and the ATP concentration was periodically measured. As a result, the ATP concentration was decreased in MN9D and MN9X cells, and MN9D cells exhibited more sensitive responsiveness to pyrano-1,2-naphthoquinone, as compared to MN9X cells.

### Example 4: Changes in intracellular energy level by NQO1

This Example was intended to investigate whether NQO1 plays an important role in regulation of energy concentration, via confirmation of changes in the energy concentration by treatment of cells with a drug that activates NQO1 in the cells.

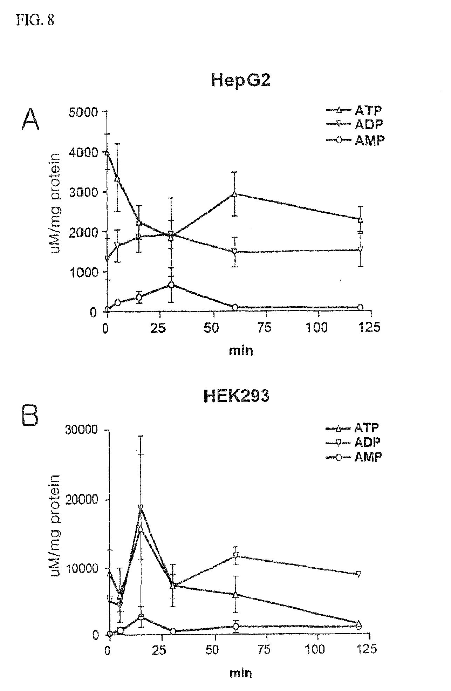

FIG. 8A shows that an ATP amount was decreased to a minimum value and an AMP amount was increased to a maximum value 30 min after treatment of cells with pyrano-1,2-naphthoquinone, upon measurement of intracellular ATP, ADP and AMP levels over time by LC-MS while treating the hepatoma cell line HepG2 with 10 µM pyrano-1,2-naphthoquinone for 2 hours. FIG. 8B shows that HEK293 cells exhibit no significant changes in the intracellular ATP level as compared to HepG2 cells, upon measurement of intracellular ATP, ADP and AMP levels over time by LC-MS while treating HEK293 cells having no NQO1 activity with pyrano-1,2-naphthoquinone for 2 hours. Therefore, it can be seen that a decrease of ATP concentration by the NQO1 activator is NQO1-dependent.

### Example 5: Changes in NAD⁺/NADH, AMP/ATP and NADP⁺/NADPH ratios in living organisms by NQO1 activator

This Example was intended to investigate whether an NQO1 activator has effects through NQO1 on changes of an NAD⁺/NADH ratio *in vivo.* 5 mg/kg of pyrano-1,2-naphthoquinone was intravenously administered to a tail vein of DIO mice and changes in an NADH amount and an NAD⁺/NADH ratio of liver tissues were examined for 6 hours.

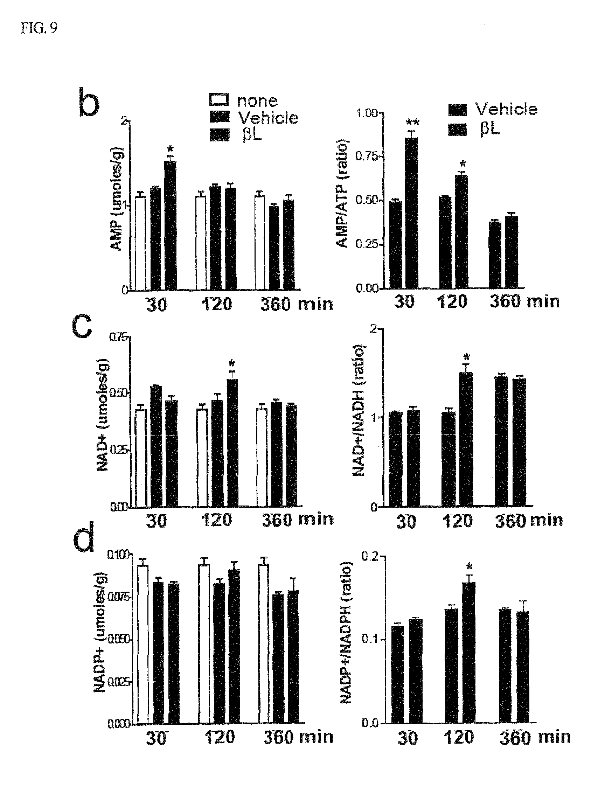

FIG. 9B shows that treatment of βL on cells resulted in a maximum increase of AMP concentration at a point of 30 min and AMP concentration was recovered to a normal level at a point of 120 min, whereas the AMP/ATP ratio was increased to the highest level at a point of 30 min and then gradually decreased and recovered the initial state at a point of 360 min.

FIG. 9C shows a pattern of changes in NAD⁺ and NADH amounts in terms of NAD⁺/NADH ratio. As can be seen, the NAD⁺ amount and NAD⁺/NADH ratio was increased to a maximum value at a point of 120 min, and then gradually decreased. That is, intravenous (IV) injection of βL resulted in transient increases of NAD⁺ and AMP levels and therefore an experimental group exhibited higher AMP/ATP and NAD⁺/NADH ratios in the liver as compared to a control group.

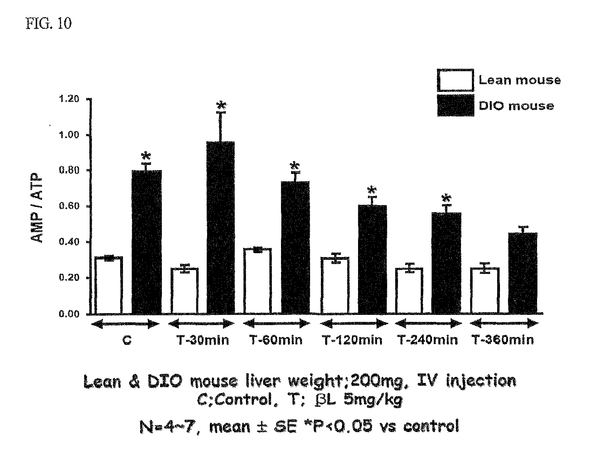

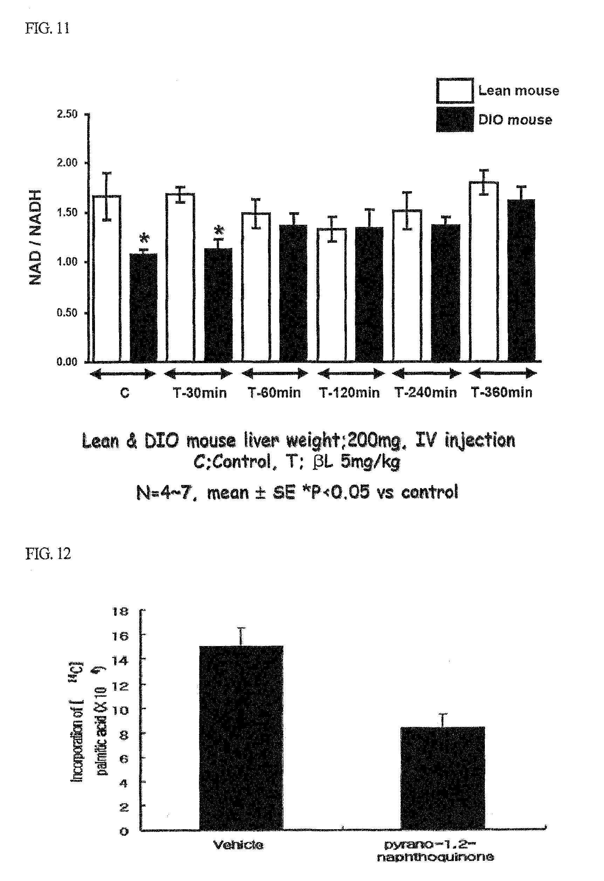

Further, FIG. 9D shows that βL treatment resulted in no significant change in a numerical value of NADP⁺, but the NADP⁺NADPH ratio was increased to a maximum value at a point of 120 min, due to a decreased NADPH concentration. According to the same experimental method as described above, βL was administered to Lean mice and DIO mice, respectively. Results of changes in the AMP/ATP ratio and the NAD⁺/NADP ratio are given in FIGS. 10 and 11, respectively. As can be seen from FIGS. 10 and 11, Lean mice exhibited less changes in the AMP/ATP ratio and NAD⁺/NADP ratio compared with a control group, whereas DIO mice exhibited significant changes in the AMP/ATP and NAD⁺/NADP ratios, showing convergence of those ratios to values of Lean mice over time. Therefore, it can be seen that the NQO1-activating compound makes a great contribution to changes in the AMP/ATP ratio and NAD⁺/NADP ratio in obese subjects.

### Example 6: Effects of NQO1 on β-oxidation of C57BL/6 mice

A study was conducted to investigate effects of an NQO1 activator pyrano-1,2-naphthoquinone on β-oxidation of C57BL/6 mice.

FIG. 12 shows measurement results of amounts of 14C-palmitic acid incorporated into the liver (triglyceride, TG) after IV injection of 14C-palmitic acid to a tail vein of DIO mice fed with a vehicle or 50 mg/kg of pyrano-1,2-naphthoquinone for 7 days. 10 µCi of 14C-pahnitic acid was IV-injected to a tail vein of mice and 10 min later the liver was removed and homogenize Fat was extracted from the homogenizate, and radioactivity was measured by a liquid scintillation counter. The amount of 14C-pa1mi1ic acid incorporated into hepatic triglyceride (TG) was about 45% lower in mice treated with pyrano-1,2-naphthoqumone, as compared to that of vehicle-treated mice (control group), This result indicates that mice tested with pyrano-1,2-naphthoquinone utilize larger amounts of palmitic acid in extrahepatic tissues, as compared to the control group.

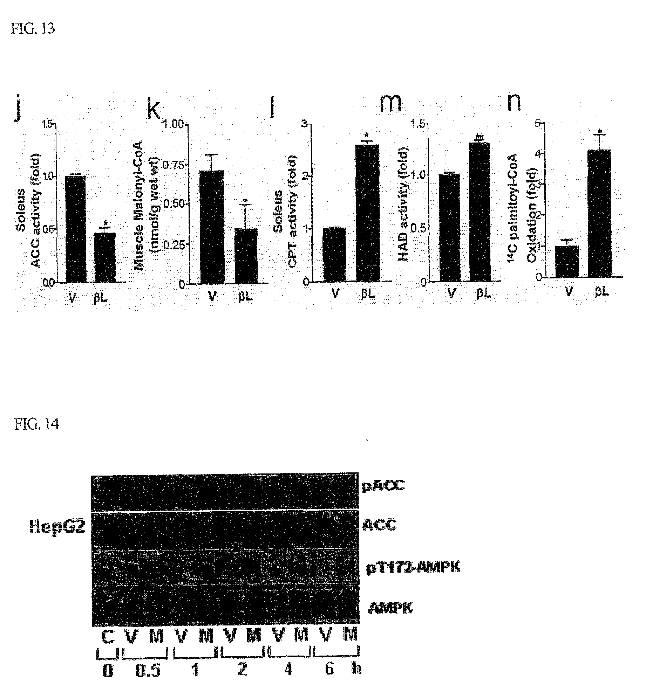

FIGS. 13 j and 13-k show that ACC activity and malonyl CoA level, which are critical inhibitors of CPT and mitochondrial fatty acid oxidation, were decreased in response to βL treatment. On the other hand, FIGS. 13-l,13-m and 13-n show that CPT and HAD activity and ³⁴C-palmitoyl CoA β-oxidation were significantly increased in the muscle of the βL-treated DIO mice, as compared to the control group. These results suggest that βL treatment brings about transient NQO1-mediated elevation in the NAD(P)/NAD(P)H ratio, which in turn leads to activation of AMPK *in vivo* and acceleration of mitochondrial oxidative phosphorylation and fatty acid oxidation. That is, such facts mean that chronic treatment of βL can accelerate mitochondrial oxidative phosphorylation and enhance adaptive mitochondrial biogenesis. Therefore, βL can be used as a therapeutic agent for metabolic diseases.

### Example 7: Regulation of AMPK and ACC phosphorylation by NQO1 activator

This Example was carried out to confirm whether an NQO1 activator has effects through NQO1 on phosphorylation of AMPK and ACC that are intracellular energy-regulating proteins. In order to examine phosphorylation of AMP kinase and ACC (acetyl-CoA carboxylase) by the NQO1 activator pyrano-1,2-naphthoquinone, HepG2 cells (Human hepatoma cell line) were seeded onto a 6-well plate at a density of 1 x 10⁵ cells per well, and cultured in a RPMI+10% FBS medium. After growing the cells for 24 hours, the culture medium was replaced with a serum-free RPMI medium and cells were treated with pyrano-1,2-naphthoquinone (10 µM) for 30 min, 1 hr, 2 hrs, 4 hrs and 6 hrs, in combination with a control (DMSO). Anti-ACC and Anti-pS79-ACC were used to observe phosphorylated ACC, whereas Anti-AMPK and Anti-pT172-AMPK were used to observe phosphorylated AMP kinase. As shown in FIG. 14, phosphorylation of AMP kinase by pyrano-1,2-naphthoquinone could be observed from the initial time point (30 min), and it can be confirmed that such phosphorylation effects lasted up to 6 hours. In addition, it can be confirmed that ACC, which is known as a target protein of AMP kinase, was also phosphorylated. These results show that activation of AMPK by the action of NQO1 activator can suppress the activity of acetyl-CoA carboxylase, which is a crucial regulatory enzyme of lipogenesis and as a result, NQO1 can play a certain role in fatty acid oxidation.

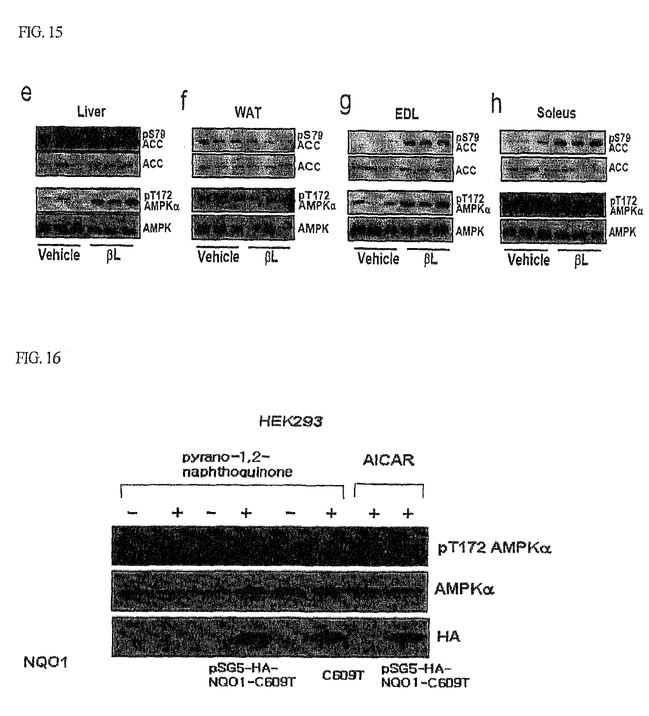

Using various proteins extracted from liver, gonadal fat (WAT), EDL and soleus muscle of DIO mice treated with a vehicle or βL for 2 days, T172 phosphorylation of AMPKα and S79 phosphorylation of ACC in each tissue were measured by Western blot analysis. As a result, S79 phosphorylation of ACC and T172 phosphorylation of AMPKα were increased in the liver and muscle (EDL, Soleus), while gonadal fat of DIO mice corresponding to βL treatment exhibited no such effects (see FIG. 15).

### Example 8: Effects of NQO1 on phosphorylation of AMPK

In order to investigate the importance of an NQO1 protein in phosphorylation of AMP kinase, HepG2, MCF-7 and HEK293 cells were seeded onto 60 mm plates at a density of 1 x 10⁶ cells/plate. After growing the cells for 24 hours, the culture medium was replaced with a serum-free medium, and cells were treated with pyrano-1,2-naphthaquinone (10 µM) for 30 min, respectively, in combination with a control group (DMSO). Cells were lysed in RIPA buffer and amounts of all proteins were quantified using a spectrophotometer, and 50 µg of the protein was subjected to electrophoresis. AMP kinase phosphorylation was examined using Anti-AMPK and Anti-pT172-AMPK, whereas the NQO1 protein was assayed using Anti-NQO1 From the fact that AMP kinase phosphorylation by pyrano-1,2-naphthoquinone (10 µM) was not observed in HEK293 cells, it can be seen that the NQO 1 protein is crucial for phosphorylation of AMP kinase.

FIG. 16 shows effects of NQO1 on phosphorylation of AMP kinase. In order to examine that an activity of NQO1 is important for the phosphorylation of AMP kinase, HEK293 cells were seeded onto a 6-well plate at a density of 1 x 10⁵ cells/well, cultured for 24 hours, and transfected with pSG5-HA-NQO1 (500 ng) and pSG5-HA-NOQ1-C609T (500 ng) plasmids. After 24 hours, the culture medium was replaced with a serum-free medium, and the cells were treated with pyrano-1,2-naphthoquinone (10 µM) and AICAR (2 mM) for 30 min, in combination with a control (DMSO). AMP kinase phosphorylation were assayed using Anti-AMPK and Anti-pTl72-AMPK, whereas transfected NQO1 was assayed using Anti-HA. From no phosphorylation of AMP kinase in NQO1-C609T having no NQO1 activity, the enzymatic activity of NQO1 protein is critical for phosphorylation of AMP kinase by pymno-1,2-naphthoquinone,

Further, relationship between NAD(P)H oxidation and AMPK phosphorylation/activation by the action of NQO1 was observed in βL-treated HepG2 cells with high expression of NQO1, MCF-7 cells with a low expression of NQO1, and HEK293 cells with no expression of NQO1.

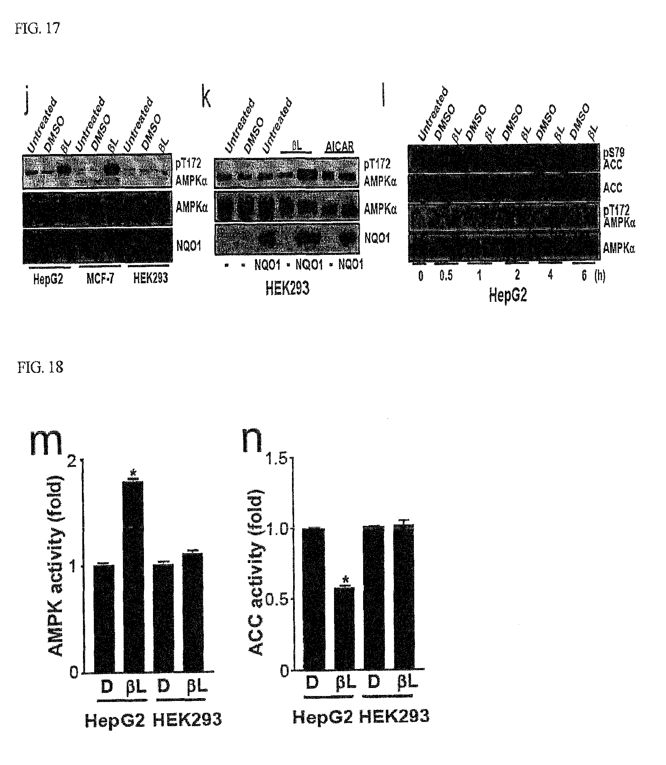

PIG. 17-j shows that NQO1 activation in βL-treated cells is correlated with T172 phosphorylation of AMPK. FIG. 17-k shows that treatment of HEK293 cells with AICAR (5-aminomidazole-4-carboxamide-1-beta-D-ribofuranoside) also leads to an increase in phosphorylation of AMPK. In addition, AMPK phosphorylation/activation was not promoted in HEK293 cells, except for HEK293 cells transfected with wild type NQO1. FIG. 17-1 and FIG. 18 show that βL accelerates ACC (acetyl-CoA carboxylase) phosphorylation as well as AMPK phosphorylation/activation and decreases ACC activation in HepG2 cells.

### Example 9: Effects of NQO1 on charges in intracellular calcium concentration and mitochondrial membrane potential

This Example was intended to examine calcium influx in HepG2 cells by an NQ01 activator pyrano-4,2-naphthoquinone and to examine changes in calcium influx by an NQO1 inhibitor dicoumarol. Cells were cultured for 24 hours and then pre-treated with Fluo-4 for 30 min. Cells immobilized on a coverslip were transferred to a slide glass and treated with pyrana-1,2-naphthoquinone (10 µM), and at the same time images were taken every 2 seconds for 10 min using a fluorescence microscope. In order to monitor changes in intracellular calcium influx by dicoumarol (5 µM), cells were pretreated with Fluo-4 (5 µM) and dicoumarol for 30 min, hollowed by treatment of pyrano-1,2-naphthoquinone (10 µM) and fluorescence microscopic observation.

As shown in FIG. 19, it was observed that the calcium influx by pyrano-1,2-naphthoquinone exhibited a calcium peak at a point of about 10 sec after treatment thereof and the calcium influx was inhibited by dicoumarol. Further, the calcium peak was not appeared in NQO1-deficient HEK293 cells. This fact indicates that NQ41 activity is closely related with calcium influx.

As shown in FIG. 20, TMRE (tetramethylrhodamine ethyl ester) fluorescence was decreased by about 20% in βL-treated NQO1-proficient HepG2 cells and cardiomyocytes, thereby leading to changes in mitochondrial membrane potential (ΔΨm). However, such effects were not observed in HEK293 cells. Effects of βL on ΔΨm were transient and dicoumarol-treated cells did not exhibit such effects. Therefore, it can be seen that changes in the mitochondrial membrane potential (ΔΨm) are NQO1-dependent.

On the other hand, activities of calcium-dependent kinases (CDKs), mitochondrial enzymes or any other calcium-dependent process are modulated with changes in calcium homeostasis. FIG. 21 shows measurement results of Fluo4 and Rhod-2-AM fluorescence, each representing a cytosolic Ca²⁺ concentration ([Ca²⁺]c) and a mitochondrial Ca²⁺ concentration ([Ca²⁺]m). That is, it was continuously observed that increases of the cytosolic Ca²⁺ concentration ([Ca²⁺]c) and the mitochondrial Ca²⁺ concentration ([Ca²⁺]m) in βL-treated cells exhibited similar kinetic behavior. In addition, it was revealed that effects of βL on the intracellular calcium concentration were susceptible to inhibition of NQO1 activity by dicoumarol. These results suggest that the NQO1 activity is closely correlated with a calcium concentration

Further, FIG. 22 shows that mitochondrial oxygen consumption in HepG2 cells and cardiomyocytes is increased by βL, but such increased oxygen consumption is not observed in HEK293 cells. These results suggest that NQO1-mediated βL reduction with the accompanying fast oxidation of NAD(P)H shifts the cellular redox state, and accelerates the mitochondrial oxidative phosphorylation.

### Example 10: Confirmation of binding between NQO1 and AMPKα, β or γ

In order to confirm binding between NQO1 and AMPKα, β or γ, HEK293 cells were seeded onto a DMEM+10% FBS medium in a 100 mm plate, cultured to reach cell mass of 80%, and transfected with HA-NQO1. 24 hours after transfection, cells were lysed and immunoprecipitation was carried out using 500 µg of a protein.

As shown in FIG. 23, it can be seen that NQO1 bound to none of AMPKα, β and γ.

### Example 11: Effects of NQO1 activator on phosphorylation of endothelial nitric oxide synthase (eNOS)

It is well-known that activation of AMPK activates NRF-1 and facilitates mitochondrial biogenesis. In addition, NO/cGMP activates PGC-1α and NRF-1 to facilitate mitochondrial biogenesis. In order to ascertain whether an NQO1 activator, which activates A11PK, is involved in production of nitric oxide (NO), a degree of phosphorylation, leading to an increase in activity of endothelial nitric oxide synthase (eNOS), was determined. In order to examine phosphorylation of eNOS by the action of NQOl activator, Human Umbilical Vein Endothelial Cells (HUVEC) were seeded onto a 60-mm plate at a density of 1 x 10⁵ cells, and cultured in EBM2+5% FBS medium for 24 hours. The culture medium was replaced with a serum-free EBM2 medium, and cells were treated with pyrano-l,2-naphthoquinone (10 µM) for a predetermined time. Phosphorylated eNOS was observed using Anti-pS1177 eNOS.

As shown in FIG. 24, phosphorylation of eNOS reached a maximum increase 30 min after treatment of pyrano-1,2-naphthoquinone and then gradually diminished, thereby being not observed 2 hours later. An increase in phosphorylation of eNOS by pyrmlo-12-naphthoquinone presents the possibility that pyrano-1,2-naphthoquinone may be therapeutically used for ischemic heart diseases and mitochondrial myopathy, as well as mitochondrial dysfunction-related diseases (for example, degenerative cerebral diseases, diabetes, cardiomyopathies, and diseases associated with senescence).

### Example 12: Effects of NQO1 activator on activation of AMPK in C57BL/6 mice

FIG. 25 shows that an NQO1 activator activates AMPK in C57BL/6 mice. A vehicle and 5 mg/kg of pyrano-1,2-naphthoquinone were administered to tail veins of C57BL/6 mice for 2 hours and 4 hours, respectively. Liver and gonadal adipose tissues were removed and activity of AMPK kinase was assayed. A degree of activation was expressed as a CPM value of radioisotopes. Using the same manner, HepG2 cells, a cell line derived from human liver, were treated with 10 µM pyrano-1,2-naphthoquinone for 30 min, and then an assay for AMPK kinase activity was carried out. It was confirmed that the activity of AMPK increased in HepG2 cells. As can be seen from the results in FIG. 25, administration of pyrano-1,2-naphthoquinone leads to an increased AMPK activity in the liver and gonadal adipose tissues and hepatocytes.

### Example 13: Effects of NQO1 activator on phosphorylation of AMPK & ACC in DIO mice

FIG. 26 is a photograph showing effects of an NQO1 activator on AMPK & ACC phosphorylation in CS7BL16 mice. In order to investigate anti-obesity effects of an NQO1 activator, the NQO1 activator was administered daily to DIO mice at a dose of 50 mg/kg via an oral route, and effects of the NQO1 activator on phosphorylation of AMPK and ACC, which play an important role in energy metabolism and lipogenesis in the liver and gonadal adipose tissues, were examined As shown in FIG. 26, it was confirmed through Western blot analysis that the NQO1 activator pyrano-1,2-naphthoquinone has an effect on phosphorylation of AMPK and ACC in the gonadal and liver tissues of C57BL/6 DIO mice. Phosphorylated AMPK is believed to activate metabolism associated with energy. Whereas, it is believed that ACC, which is affected by activation of AMPK, is phosphorylated and lipogenic activity thereof is then inhibited, which will then exert some effects on lipid metabolism including inhibition of obesity.

FIG. 27 shows that the AMPK activity was higher in Liver, EDL (extensor digitorum longus) and soleus muscle of DIO mice with oral administration of βL at a dose of 50 mg/kg, as compared to a vehicle-treated control group. Oral administration effects of βL reached a maximum 2 hours after administration and lasted up to about 6 hours after administration thereof (data not shown).

### Example 14: Effects of NQO1 activator on expression of genes involved in lipid metabolism of DIO mice

In order to investigate anti-obesity effects of an NQO1 activator, the NQO1 activator was administered daily to DIO mice at a dose of 54 mg/kg via an oral route, and an attempt was made to confirm levels of mRNAs of acetyl CoA carboxylase (ACC) 1, ACC2, fatty acid synthase (FAS), lipoprotein lipase (LPL), and stearoyl-CoA desaturase 1 (SCD1), which participate in lipid metabolism in the hepatic and gonadal adipose tissues, by real-time quantitative PCR These enzymes are very important for lipid metabolism; it is known that ACC catalyzes formation of malonyl CoA from acetyl CaA, FAS catalyzes formation of palmitate from malonyl CoA, and SCD1 catalyzes formation of monounsaturated fat, thus playing a critical role in formation of triacylglycerol, a major energy store. Therefor, these enzymes are closely correlated with obesity, diabetes, and lipid metabolism-relad diseases. As shown in FIG. 28, expression levels of mRNAs of ACC1 and ACC2, FAS, LPL, and SCD1 remarkably decreased in the experimental groups to which pyrano-1,2-naphthoquinone was administered, as compared to a control group, and the LPL mRNA level in experimental groups increased approximately 2-fold as compared to the control group. Therefore, from the results of such increased or decreased expression of genes for the above-mentioned enzymes, it can be inferred that NQO1 will be feasible as a therapeutic target for the treatment of metabolic diseases.

### Example 15: Effects of NQO1 activator on gene expression of proteins involved in glucose metabolism of DIO mice

Pyrano-1,2-naphthoquinone was administered daily to DIO mice at a dose of 50 mg/kg via an oral route, and levels of mRNAs for hexokinase 2 (HK2), glucose transporter (GLUT) 2 and GLUT4 in the liver and gonadal adipose tissues were confirmed by real-time quantitative PCR. GLUT is well-known as a protein that mediates intracellular uptake and expenditure of blood glucose in organs such as liver, adipocytes and myocytes, whereas HK2, an enzyme belonging to a glucokinase class, phosphorylates proteins that are thus allowed to enter glycolytic pathways. As can be seen from the results of FIG. 29, an HK2 mRNA level is decreased as compared to a control group, whereas mRNAs of GLUT2 and GLUT4, two enzymes involved in glucose transportation, exhibited significant increases in their expression. Increased levels of GLUT2 and GLUT4 facilitate intracellular uptake of blood glucose, thus presenting the possibility of NQO1 as a target for treatment of diabetes.

### Example 16: Effects of NQO1 activator on gene expression of proteins involved in mitochondrial biogenesis of DIO mice

Pyrano-1,2-naphthoquinone was administered daily to DIO mice at a dose of 50 mg/kg via an oral route, and levels of mRNAs of peroxisome proliferator-activated receptor coactivator alpha 1 (PGC1α), nuclear respiratory factor 1 (NRF1), mitochondrial transcription factor (mtTFA), and cytochrome c oxidase (COX) 4 and COX7 in the liver and gonadal adipose tissues were confirmed by real-time quantitative PCR Proteins shown in FIG. 30 are representative enzymes responsible for regulation of biogenesis of mitochondria which plays a critical role in the biosynthesis of energy in cells, and are also known to be involved in regulation of various cellular physiological processes. Although there are slight differences in amount of mRNAs between these enzymes, pyrano-1,2-naphthoquinone-administered groups exhibited increased levels of mRNAs for all enzymes, as compared to the control group. Since abnormal activity of mitochondria is reported in a variety of metabolic syndromes, these results show the possibility that NQO1 can be a therapeutic target for the treatment of metabolic diseases, mitochondrial dysfunction-related diseases and energy metabolism-related diseases, via amelioration of such phenomena.

### Example 17: Effects of NQO1 activator on expression of genes involved in energy metabolism of DIO mice

Pyrano-1,2-naphthoquinone was administered daily to DIO mice at a dose of 50 mg/kg via an oral rout, and levels of transcripts of genes involved in energy metabolism in the liver and gonadal adipose tissues were measured using real-lime quantitative PCR Referring to enzymes shown in FIG. 31, PPAR alpha and gamma are enzymes responsible for transcriptional regulation of enzyme involved in energy expenditure, AMPK plays a central role in the maintenance of cell energy homeostasis by sensing the intracellular AMP/ATP ratio, and AOX catalyzes to activate oxidative phosphorylation via oxidation of acyl CoA which resides in a certain step of a lipid metabolism process. In addition, CPT1 is also an enzyme involved in energy metabolism, and is well-known as an enzyme that enables the passage of long chain acyl CoA into mitochondria, not taking a route coward synthesis of triacylglycerol. In the group to which pyrano-1,2-naphthoquinone was administered, levels of mRNA of peroxisome proliferator activated receptor (PPAR) alpha were not changed, whereas PPAR gamma exhibited about a two-fold increases in mRNA levels thereof In addition, even though there are differences to some extent in mRNA levels between acyl CoA oxidase (AOX), AMP-actLvated protein kinase (AMPK.) alpha 1 and AMPK alpha 2, and carnitine palmitoyltransferase 1, the pyrano-1,2-naphthoquinone-administered groups exhibited increased levels in mRNAs of such enzymes, as compared to the control group. Increased expression levels of such genes show the possibility that NQO1 can be as a protein target for the treatment of energy metabolism-elated diseases.

### Example 18: Effects of NQO1 activator on expression of SIRT-related transcripts in DIO mice

hyrano-1,2-naphthoquinone was administered daily to DIO mice at a dose of 50 mg/kg via an oral route, and levels of transcript of Sirtuin (SIRT) genes in gonadal adipose tissues were measured on Days 7, 28 and 56 of administration, using real time quantitative PCR Referring to SIRT-related transcripts, there are known 7 species of transcripts in humans. In particular, SIRT1 is well-known as an enzyme involved in longevity and it is also reported that SIRT1 greatly increases when calories are ingested with limitation. As can be seen from FIG. 32, SITR1, SIRT3 and SIRT6 significantly increased, while SIRT2, SIRT5 and SIRT7 did not exhibit any noticeable difference between the experimental groups and control group.

### Examples 19: Effects of NQO1 activator on expression of transcripts of UCP1 and UCP2 genes in DIO mice

Pyrano-1,2-naphthoquinone was administered daily to DIO mice at a dose of 50 mg/kg via an oral route, and levels of transcripts of uncoupling protein 1 & 2 (UCP 1 & 2) genes in the liver and gonadal adipose tissues were measured using real-time quantitative PCR. UCP 1 & 2 are enzymes that perform energy expenditure via heat generation, and it is reported that these enzymes function to consume energy without involving production of reactive oxygen species (ROS) and are also closely correlated with the incidence of obesity. As shown in FIG. 33, administration of pyrano-1,2-naphthoquinone has led to significant increases in mRNA levels of UCP1 & 2. These results show the possibility of NQO1 as a safe therapeutic for the treatment of metabolic syndromes, via reduction of stress due to ROS that is additionally produced in an energy production process.

### Example 20: Effects of NQO1 activator on expression of genes involved in glucose and fat metabolism in various organs

In order to investigate effects of βL on the expression of genes involved in glucose and fat metabolism in liver, skeletal muscle and white adipose tissue (WAT), experiments were carried out using gene expression microarray analysis and mouse A42 Affymetrix GeneChip.

FIG. 34 shows results of RT-PCR quantification on gene expression rates of indicated genes of vehicle-treated mice and βL-treated mice. FIGS. 34-h and 34-i show the increased expression of several genes linked to mitochondrial biogenesis and energy metabolism in βL-treated mice. For example, βL treatment significantly induced the expression of PGC-1α and NRF-1 implicated in the mitochondrial biogenesis in the liver and muscle (FIGS. 34-h and 34-i), and mitochondrial genes COX4, COX7, AOX, UCP2 and UCP3 were strongly upregulated in muscle of the βL-treatment group (FIG. 34-i).

Expression of glucose transporter (GLUT) 2 and GLUT 4 was induced in muscle of the βL-treated mice (FIG. 34-i). Interestingly, Sirt1 and Sirt3, which are importantly responsible for caloric restriction, were induced in muscle and WAT of the βL-treated mice (FIGS. 34-I and j).

In addition, in adipose tissues, expression of lipolytic genes LPL and ATGL, fatty acid oxidation genes PPARα and SIRT1, and glucose uptake genes Glut2 and Glut4 were higher in βL-treated mice than vehicle-treated mice (FIG. 34-j).

### Example 21: Effects of NQO1 activator administration on changes over time in body weight and dietary intake in DIO mice

FIG. 35 shows changes in dietary intake/body weight and weight changes for 56 days, after daily administration of pyrano-1,2-naphthoquinone into obese mice at a dose of 50 mg/kg via an oral route. Pyrano-1,2-naphthoquinone-administered group exhibited decreases in dietary intake for the first two weeks, and thereafter the dietary intake level recovered similar to that of a control group. These results are believed to be due to decomposition of fat being facilitated and therefore sufficient amounts of energy are generated. In addition, even though mice were fed high-fat diet, animals exhibited a continuous weight loss for 56 days, as compared to a control group. Such body weight loss was due to decreases in subcutaneous and visceral adipose tissues, as shown by MRI images of coronal and transverse sections in FIG 36.

FIG. 37 is a graph comparing weight changes in various organs between the treatment group and control group after administration of pyrano-1,2-naphthoquinone to C57BL/6 DIO for 56 days. As shown in FIG. 37, amounts of subcutaneous, mesenteric, perirenal and gonadal fats were gradually decreased over the first four weeks after administration of pyrano-1,2-naphthoquinone, followed by the steady state for the remaining four weeks.

FIG. 38 shows whole laparotomized states of animals after administration of pyrano-1,2-naphthoquinone to C57BL/6 DIO mice for 56 days and results of oil red O staining and EM examination on fat accumulation in liver tissues.

As can be seen from FIG. 38A, C57BL/6 DIO mice with administration of pyrano-1,2-naphthoquinone for 56 days exhibited conspicuous decreases in visceral fat and body weight, and a reduced size of liver tissues that were turned into red color. In order to confirm improvement in condition of fatty liver, accumulated fat in the liver was stained using oil red O staining and as a result, it was confirmed that fat has diminished by 90% or more, as compared to a control group (FIG 38B). FIG 38C is an EM of liver tissues, showing remarkably decreased fat vacuoles and glycogen stores as compared to a control groups, recovery of normal mitochondrial morphology, significant increases in mitochondrial numbers, and improved morphology of endoplasmic reticulum.

As shown in FIG. 39, muscle of the untreated animals exhibited swelling, entanglement and insufficient numbers of mitochondria, whereas βL-treated DIO mice and ob/ob mice exhibited normal morphology and increased numbers of mitochondria. Further, as shown in FIG. 40, the number of type I myofibers increased in the soleus muscle of βL-treated DIO mice.

FIG. 41 shows perilipin staining results of gonadal adipose tissues following laparotomy of animals on Day 56 after daily administration of pyrano-1,2-naphthoquinone to C57BL/6 DIO mice at a dose of 50 mg/kg via an oral route. As can be seen from FIG. 41, the size of adipocytes was remarkably decreased.

FIG. 42 shows changes in triglyceride (TG), cholesterol, free fatty acid, glucose, insulin, TNFα, resistin and leptin levels in the blood collected on Days 3, 7, 14, 28 and 56, after daily administration of pyrano-1,2-naphthoquinone to C57BL/6 DIO mice at a dose of 50 mg/kg via an oral route. As can be seen therefrom, blood fat and glucose levels were significantly improved and further, insulin resistance and leptin resistance were also improved. Further, the blood level of resistin, which causes insulin resistance, was also significantly improved. From these results, it is expected that pyrano-1,2-naphthoquinone will be highly effective for the treatment of fatty liver, hyperlipidemia, type 2 diabetes and insulin resistance.

FIG. 43 shows H&E staining results of brown adipose tissues of mice on Day 56 after daily administration of pyrano-1,2-naphthoquinone to C57BL/6 DIO mice at a dose of 50 mg/kg via an oral route. As can be seen from FIG. 43, the size of adipocytes remarkably decreased.

FIG. 44 shows results of EM examination of brown adipose tissues taken on Day 56 after daily administration of pyrano-1,2-naphthoquinone to C57BL/6 DIO mice at a dose of 50 mg/kg via an oral route. As can be seen therefrom, the size of adipocytes remarkably decreased.

### Example 22: Effects of NQO1 activator administration on changes over time in insulin sensitivity and glucose tolerance of OLETF rats

FIG. 45 is a graph showing glucose tolerance and insulin sensitivity measured in vehicle-treated OLETF rats and βL-treated OLETF rats. Glucose and insulin were i.p. and i.v. injected, respectively, and the blood glucose level was measured. As a result, as shown in FIGS. 45a and 45c and FIGS. 45b and 45d, OLETF rats exhibited improvement in glucose tolerance and insulin sensitivity on Days 3 and 21 after βL-treatment.

### Example 23: Changes in leptin receptor-deficient (ob/ob) mice by administration of NQO1 activator

FIGS. 46 and 47 show changes in dietary intake/body weight and changes in body weight for 56 days, according to daily administration of pyrano-1,2-naphthoquinline into leptin receptor-deficient (ob/ob) mice at a dose of 200 mg/kg via an oral route. Dietary intake/body weight was notably decreased at around 10 days of administration, and thereafter the dietary intake level recovered 70% of that of a control group. These results show that administration of pyrano-1,2-naphthoquinone also effectively decreases body weight in leptin receptor-deficient (ob/ob) mice.

In order to examine fat accumulation in the liver tissue, animals were laparotomized 56 days after administration of pyrano-1,2-naphthoquinone, and H&E staining and EM examination were performed on the liver tissue. FIG. 48 shows through the results of H&E staining on liver tissue that almost all fat vacuoles have disappeared as compared to the control group. Such results present expectation that administration of pyrano-1,2-naphthoquinone will be highly effective to treat fatty liver in leptin receptor-deficient (ob/ob) mice as well. From FIG. 49, the results of EM examination on liver tissues showed remarkably decreased fat vacuoles and glycogen stores as compared to the control group, recovery of normal mitochondrial morphology, significant increases in mitochondrial numbers, and improved morphology of endoplasmic reticulum. From FIG. 50, the results of EM examination on a muscle tissue of animal limbs showed the recovery of normal mitochondrial morphology in the treatment group as compared to strange morphology of mitochondria shown in the control group, and significant increases in mitochondrial numbers.

### Example 24: Effects of NQO1 activator on spontaneous locomotor activity

Pyrano-1,2-naphthoquinone was administered to C57BL/6 DIO mice, and 3 hours later spontaneous locomotor activity was measured using Versa MAX Activity Monitors & Analyzer (AccuSan Instruments, Columbus, OH, USA). The monitor used to measure motion of animals was a 41 cm x 41 cm Plexiglas chamber (height: 30 cm) equipped with infrared rays at intervals of 2.5 cm along the x- and y-axes, respectively, whereby 16 scanning lines are respectively arranged on front/rear and right/left sides of the chamber. In order to distinguish between spontaneous locomotor and stereotypic/grooming behavior, animal activity was measured by taking continuous interference of two different scanning lines caused by mice as an effective determination standard. The pyrano-1,2-naphthoquinone-administered group, the vehicle-administered group and the control group were respectively placed in each measuring apparatus, and activity and motion of animals were measured for 7 hours. For acclimation of the animals to the new environment, mice were placed in the apparatus 2 hours prior to measurement. The measurement results thus obtained are shown in FIG. 51. As shown in FIG. 51, the vehicle-administered group and control group exhibited substantially no difference therebetween, but the pyrano-1,2-naphthoquinone-administered group exhibited a significant difference in the motion and activity of animals.

### Example 25: Effects of NQO1 activator on enhancement of physical endurance

This Example was intended to measure a difference in physical endurance of mice through a swimming test For this purpose, water was placed in a cylindrical trough having a diameter of 9.5 cm and a height of 25 cm, and pyrano-1,2-naphthoquinone was administered to C57BL/6 DIO mice. 3 hours later, a sample-administered group and a control group were placed simultaneously into each cylindrical trough for measurement, and physical endurance of each group was measured and compared. The results thus obtained are shown in FIG. 52. As shown in FIG. 52, it was confirmed that the pyrano-1,2-naphthoquinone-administered group exhibited about a two-fold increased swimming duration even with a single administration of pyrano-1,2-naphthoquinone, as compared to the control group.

### Example 17: Effects of NQO1 activator on Respiratory Quotient (RQ)

This Example was intended to examine effects of pyrano-1,2-naphthoquinone on fat metabolism via measurement of Respiratory Quotient (RQ). Oxygen consumption and carbon dioxide production were measured using an Oxyscan open-circuit indirect calorimeter (AccuScan Instruments, Columbus, OH). This apparatus consisted of enclosed acrylic chambers (21 × 21 × 21 cm). Fresh air was drawn into each chamber at a rate of 1500 mL/min and then O₂ and CO₂ were allowed to pass through detectors, The concentrations of the gases were recorded in mL/kg body weigh/min RQ was calculated as the volume of CO₂ produced (VCO₂) divided by the volume of O₂ consumed (VO₂). A pyrano-1,2-naphthoquinone-administered group, a vehicle-administered group and a control group were placed in each apparatus, and RQ was measured for 7 hours. For acclimation of the animals to the new environment, mice were placed in the apparatus 2 hours prior to measurement As shown in FIG. 53, the thus-measured results have confirmed that the pyrano-1,2-naphthoquinone-admmistered group exhibited a significant difference in a RQ value, as compared to the vehicle-administered group and control group.

Indirect calorimetry showed increases in night-time and day-time oxygen consumption (FIG. 54) and higher energy consumption in βL-treated mice than the control group (FIG. 55). Upon measuring a mean body temperature of animals after exposure of mice to an ambient temperature of 4°C for 12 hours, βL-treated mice were more resistant to the cold (FIG. 56).

### Example 27: Effects of NAD⁺ administration on loss of body weight

This Example was intended to examine effects of varying *in vivo* NAD(P)⁺/NAD(P)H ratio by arbitrary external supply of NAD(P)⁺, a method capable of elevating the NAD(P)⁺/NAD(P)H ratio *in vivo.* 100 mg/kg of NAD⁺ was administered, daily to leptin receptor-deficient (ob/ob) mice via an intraperitoneal (i.p.) route. A control mice group was given i.p. administration of physiological saline at the same dose. Changes in dietary intake/body weight (A) and changes in body weight (B) were measured for 30 days.

As shown in FIG. 57, dietary intake/body weight of the treatment group was notably decreased at around 10 days of NAD⁺ administration, and thereafter animals exhibited dietary intake similar to that of a control group. This is because fat degradation was facilitated and therefore sufficient amounts of energy were generated in the NAD⁺ administration group, despite similar dietary intake between the treatment group and the control group. In addition, even though they were genetically leptin-deficient ob/ob mice, the NAD⁺-administered mice exhibited a continuos weight loss for 30 days, as compared to the control group. These results show that treatment of NAD(P)⁺ can effectively decrease body weight of leptin receptor-deficient (ob/ob) mice by elevating the NAD(P)⁺NAD(P)H ratio via external administration of NAD(P)⁺.

### Example 28: Effects of NQO1 activator dimethylfumarate (DMF) administration on body weight loss

FIG. 58 shows changes in body weight and dietary intake, after administration of an NQO1 activator dimethylfumarate (DMF) to leptin receptor-deficient ob/ob mice. 200 mg/kg of DMF was daily administered to leptin receptor-deficient (ob/ob) mice via an oral route. A control group received daily i.p. administration of physiological saline at the same dose. Changes in body weight (A) and changes in dietary intake (B) were measured for 30 days. It was confirmed that dietary intake of the treatment group was regulated to a 60 to 70% level of the control group. These results show that administration of the NQO1 activator dimethylfumarate (DMF) leads to an effective decrease in body weight of leptin receptor-deficient (ob/ob) mice.

### Example 29: Experiments on whether there are effects of various NQO1-activating candidate compounds on NAD(P)H decrease and body weight loss of ob/ob mice

For a variety of NQO1-activating candidate compounds as listed in Table I below, a decrease of NAD(P)H was measured based on the aforesaid experimental method (disclosed in Section 10 hereinbefore: NADH recycling assay). In addition, according to the experimental method given in Example 23, the body weight of ob/ob mice after four weeks was measured.

The results thus obtained are given in Table I below. In this connection, the decreasing degree of NAD(P)H was expressed as a relative amount of NAD(P)H measured upon administration of the NQO1-activating candidate compound, by taking the amount of NAD(P)H in the absence of the NQO1 activator NAD(P)H to be 100. In addition, the degree of the body weight loss was expressed as a percentage of the body weight of ob/ob mice with administration of the NQO1-activating candidate compound, relative to the body weight of ob/ob mice without administration of the NQO1-activating candidate compound (control) after four weeks.

**[Table 1]**

| name | structure | Residual amount of NAD(P)H(%) | Weight reduction In ob/ob mice (4 wk) |
|---|---|---|---|
| 2-Methyl-2,3-dihydro-naphtho[1,2-*b*]furan-4,5-dione | 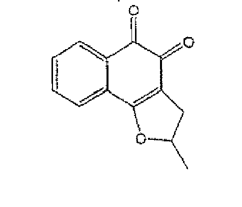 | 49.73±1.98 | -39.5±3.1 |
| 2,3,3-Trimethyl-2,3-dihydro-naphtho[1,2-*b*]furan-4,5-dione | 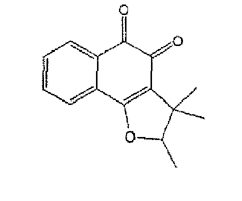 | 64.39±1.69 | -46.2±11.1 |
| 2,2-Dimethyl-2,3-dihydro-naphtho[1,2-*b*]furan-4,5-dione | 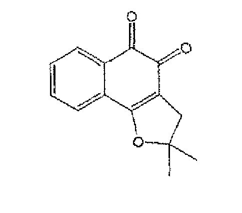 | 45.9±3.90 | -27.8±8.1 |
| 2,2,3-Trimethyl-2,3-dihydro-naphtho[1,2-*b*]furan-4,5-dione | 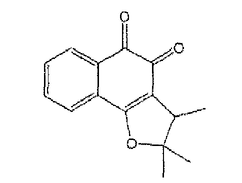 | 52.75±1.88 | -27.9±0.5 |
| 2,2-Dimethyl-3,4-dihydro-2*H*-benzo[*h*]chromene-5,6-dione (βL) | 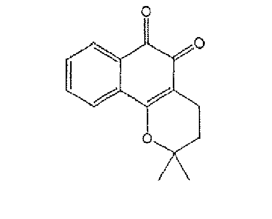 | 61.27±1.09 | -16.8±2.9 |
| 10-Isopropyl-7a-methyl-7a,8,9,11a-tetrahydro-7*H*-benzo[*c*]xanthene-5,6-dione | 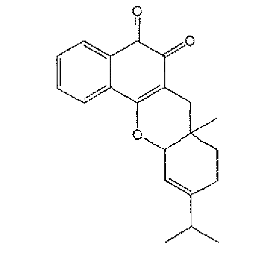 | 77.55±4.19 | -6.7±2.1 |
| 2-(2,4-Dimethyl-penta-1,3-dienyl)-2,3-dimethyl-3,4-dihydro-2*H*-benzo[*h*]chromene-5,6-dione | 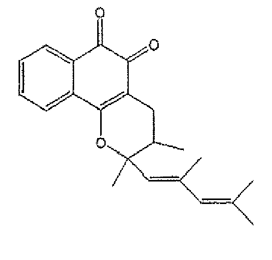 | 98.06±5.14 | -2.2±0.07 |
| 2-Methyl-2-(2-methyl-propenyl)-3,4-dihydro-2*H*-benzo[*g*]Chromene-5,10-dione | 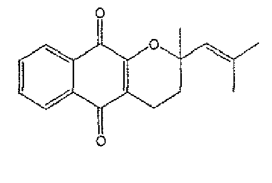 | 99.74±2.69 | -0.7±2.0 |
| 3-Isoprophy-12a-methyl-2,4a,12,12a-tetradro-7*H*-benzo[*b*]xanthene-6,11-dione | 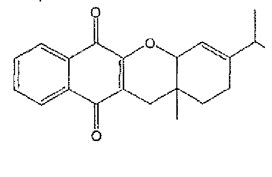 | 99.45±3.56 | 0.3±2.8 |
| 2,2-Dimethyl-3,4-dihydro-2*H*-benzo[*g*]chromene-5,10-dione | 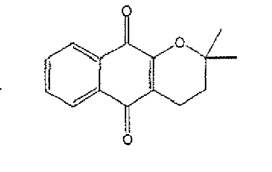 | 95±4.5 | 1.3±2.8 |
| 2-Methyl-1,4-naphthoquinone (Menadione) | 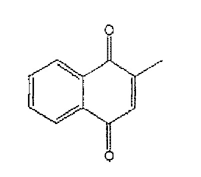 | 85.65±2.58 | -2.7±2.0 |
| CoQ10 | 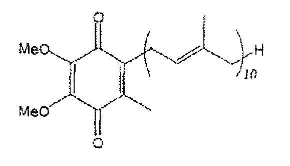 | 101.69±1.32 | 2.3±3.5- |

As can be seen from Table 1, a decrease of NAD(P)H was accompanied by a significant decrease of the body weight In particular, it can be confirmed that administration of 4-substituted-1,2-naphthoquinone, *e.g*. 2-Methyl-2,3-dihydro-naphtho[1,2-*b*]furan-4,5-dione, 2,3,3-Trimethyl-2,3-dihydro-naphtho[1,2-*b*]furan-4,5-dione, 2,2-Dimethyl-2,3-dihydro-naphtho[1,2-*b*]furan-4,5-dione, 2,2,3-Trimethyl-2,3-dihydro-naphtho[1*,*2*-b*]furan-4,5-dione, 2,2-Dimethyl-3,4-dihydro-2*H-*benzo[*h*]chromene-5,6-dione, or 10-Isopropyl-7a-methyl-7a,8,9,11a-tetrahydro-7*H-*benzo[*c*]xanthene-5,6-dione, resulted in a significant decrease of NAD(P)H and the consequent body weight reduction.

### INDUSTRIAL APPLICABILITY

As apparent from the foregoing, according to the present invention, by elevating an NAD(P)⁺/NAD(P)H ratio *in vivo* or *in vitro* via activation of oxidoreductase such as NQO1, it is possible to treat various diseases including obesity, diabetes, metabolic syndromes, degenerative diseases and mitochondrial dysfunction-related diseases and increase locomotor activity and endurance of organisms. Further, it is possible to screen and develop drug compounds capable of enhancing the NAD(P)⁺/NAD(P)H ratio, using NQO1 protein and gene as a target.

Although the preferred embodiments of the present invention have been disclosed for illustrative purposes, those skilled in the art will appreciate that various modifications, additions and substitutions are possible, without departing from the scope of the invention as disclosed in the accompanying claims.

### Preferred embodiments:

1. A method for elevating an NAD(P)⁺/NAD(P)H ratio *in vivo* or *in vitro* by regulation or addition of oxidoreductase.
2. The method according to item 1, wherein the elevation of the NAD(P)⁺/NAD(P)H ratio is carried out in a mammal.
3. The method according to item 2, wherein the mammal is a human.
4. The method according to item 1, wherein the oxidoreductase is NAD(P)H:quinone oxidoreductase 1 (NQO1).
5. The method according to item 4, wherein the NAD(P)⁺/NAD(P)H ratio is increased by enhancing the activity of NQO1.
6. The method according to item 5, wherein the change in the NAD(P)⁺/NAD(P)K ratio by NQO1 is more than a 20% decrease of NAD(P)H, based on the amount of NAD(P)H in the absence of an activator for NQO 1, thereby increasing the NAD(P)⁺/NAD(P)H ratio.
7. The method according to item 6, wherein a decrease of NAD(P)H is more than 30%.
8. The method according to item 5, wherein an AMP/ATP ratio is increased by elevation of the NAD(P)⁺/NAD(P)H ratio.
9. The method according to item 4, wherein consumption of NAD(P)H as a coenzyme or substrate is increased by increasing the amount of an NQO1 protein or the expression of an NQO1 gene.
10. The method according to item 5, wherein the activity of NQO1 is increased using a compound capable of increasing the activity or amount of NQO1.
11. The method according to item 10, wherein the compound is an H-acceptor.
12. The method according to item 11, wherein the compound at least one selected from the group consisting of quinone-based compounds, quinone-imine-based compounds, nitro-based compounds, azo-based compounds, and any combination thereof.
13. The method according to item 12, wherein the compound is at least one selected from the group consisting of naphthoquinone-based compounds and derivatives thereof.
14. The method according to item 12, wherein the compound is at least one selected from the group consisting of fumaric acid esters and derivatives thereof, oltipraz (4-methyl-5(2-pyrazinyl)-1,2-dithiole-3-thione), curcumin, anethole dithiolethione, sulforaphane, 6-methylsulphinylhexyl isothiocyanate, caffeic acid phenethyl ester, 4'-bromoflavone, avicins, fisetin, resveratrol and any combination thereof.
15. The method according to item 13, wherein the naphthoquinone-based compound is 4-aminoalkyl-1,2-naphthoquinone, 4-thioalkyl-1,2-naphthoquinone, 4-alkoxy-1,2-naphthoquinone, furano-o-naphthoquinone, pyrano-o-naphthoquinone or a derivative thereof.
16. The method according to item 14, wherein the fumaric acid ester is dimethylfumarate, monoethylfumarate, monomethylfumarate or a salt thereof.
17. The method according to item 13, wherein the naphthoquinone-based compound or derivative thereof is a compound represented by Formula I: 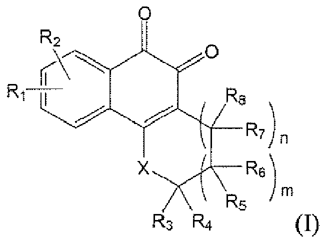 wherein
   R₁ and R₂ are each independently hydrogen, halogen, alkoxy, hydroxy or lower alkyl having 1 to 6 carbon atoms;
   R₃, R₄, R₅, R₆, R₇ and R₈ are each independently hydrogen, hydroxy, C₁₋C₂₀ alkyl, alkene or alkoxy, cycloalkyl, heterocycloalkyl, aryl or heteroaryl, or two substituents of R₃ to R₈ may be taken together to form a cyclic structure;
   X is oxygen, nitrogen or sulfur; and
   m and n are each independently 0 or 1, with proviso that when either of m and n is 0, carbon atoms adjacent to m or n may form a cyclic structure via a direct bond.
18. The method according to item 17, wherein X is oxygen, m is 1, and n is 0 or 1 with the proviso that when n is 0, carbon atoms adjacent to n form a cyclic structure via a direct bond.
19. The method according to item 17, wherein the compound is represented by Formula II: 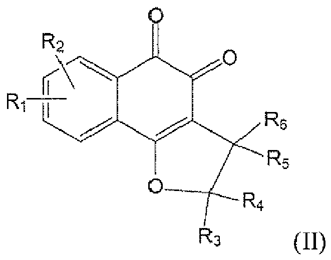 wherein
   R₁, R₂, R₃, R₄, R₅, R₆, and are the same as defined in Formula 1.
20. The method according to item 17, wherein the compound is represented by Formula III: 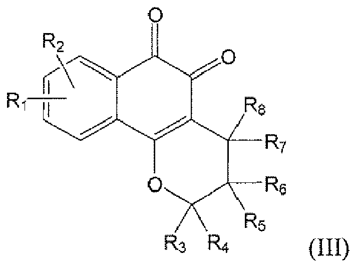 wherein R₁, R₂, R₃, R₄, R₅, R₆, R₇ and R₈ are the same as defined in Formula I.
21. The method according to item 20, wherein the compound is 2,2-Dimethyl-3,4-dihydro-2*H*-benzo[h]chromene-5,6-dione.
22. A method for identifying a compound capable of elevating an NAD(P)⁺/NAD(P)H ratio *in vivo* or *in vitro* via NQO1, comprising:
   contacting a candidate compound group with NQO1; and
   monitoring an amount or activity of NQO1.
23. The method according to item 22, wherein the method includes reacting NQO1 with a compound to be screened and NAD(P)H for a predetermined time and quantifying the resulting NAD(P)⁺ or the remaining NAD(P)H.
24. The method according to item 23, wherein the quantification of the produced NAD(P)⁺ includes measuring absorbance changes by inducement of color development due to changes in an absorption wavelength via reduction of DCPIP used as a hydride (H') acceptor.
25. The method according to item 23, wherein quantification of the remaining NAD(P)H includes measurement of absorbance changes due to color development of a tetrazolium salt.
26. The method according to item 22, wherein this method includes reacting NQO1 with a compound to be screened for a predetermined time and quantifying a decrease of NAD(P)H.
27. The method according to item 26, wherein the method includes reacting NQO1 with a compound to be screened for a predetermined time and quantifying a decrease of an intracellular ATP concentration or an increase of an intracellular AMP concentration.
28. The method according to item 22, wherein the monitoring step includes observing an increase of an intracellular calcium concentration.
29. The method according to item 22, wherein the monitoring step includes observing the degree of AMPK phosphorylation and activation.
30. The method according to item 22, wherein the monitoring step includes observing an increase of ACC phosphorylation and/or a decrease of ACC activity.
31. Use of a compound capable of increasing an amount or activity of NQO1 for the manufacture of a medicament for the treatment or prevention of a disease associated with an NAD(P)⁺/NAD(P)H ratio decrease.
32. The use according to item 31, wherein the compound is at least one selected from the group consisting of quinone-based compounds, quinone-inine-based compounds, nitro-based compounds, azo-based compounds, and any combination thereof.
33. The use according to item 32, wherein the compound is at least one selected from the group consisting of naphthoquinone-based compound and a derivative thereof, a fumaric acid ester and a derivative thereof, oltipraz (4-methyl-5(2-pyrazinyl)-1,2-dithiole-3-thione), curcumin, anethol di-thiolethione, sulforaphane, 6-methylsulphinylhexyl isothiocyanate, caffeic acid phenethyl ester, 4'-bromoflavone, avicins, fisetin, resveratrol and any combination thereof.
34. The use according to item 33, wherein the compound is selected from the group consisting of 4-alkoxy-1,2-naphthoquinone-based compound and a derivative thereof, and dimethylfumarate and an analogue thereof.
35. A method for treating or preventing a disease associated with an NAD(P)⁺/NAD(P)H ratio decrease, comprising administering a therapeutically effective amount of a compound capable of increasing an amount or activity of NQO1 to a subject in need thereof.
36. The method according to item 35, wherein the disease is obesity, obesity complications, diabetes, diabetic complications, metabolic syndromes, degenerative diseases, or mitochondrial dysfunction.
37. The method according to item 35, wherein the compound is at least one selected from the group consisting of quinone-based compounds. quinone-imine-based compounds, nitro-based compounds, azo-based compounds, and any combination thereof.
38. An NQO1 -enhancing composition comprising (a) a therapeutically effective amount of a compound capable of increasing an amount or activity of NQO1 and (b) a pharmaceutically acceptable carrier, diluent or vehicle, or any combination thereof.
39. The composition according to item 38, wherein the compound is at least one selected from the group consisting of quinone-based compounds, quinone-imine-based compounds nitro-based compounds, azo-based compounds, and any combination thereof.
40. The composition according to item 39, wherein the compound is at least one selected from the group consisting of naphthoquinone-based compound and a derivative thereof, a fumaric acid ester and a derivative thereof, oltipraz (4-methyl-5(2-pyrazinyl)- 1,2-dithiole-3-thione), curcumin, anethole dithioethione, sulforaphane, 6-methylsulphinylhexyl isothiocyanate, caffeic acid phenethyl ester, 4'-bromoflavone, avicins, fisetin, resveratrol, and any combination thereof.
41. The composition according to item 40, wherein the compound is selected from the group consisting of 4-alkoxy-1,2-naphthoquinone-based compound and a derivative thereof and dimethylfumarate and an analogue thereof.
42. A method for elevating an NAD(P)⁺/NAD(P)H ratio *in vivo,* comprising administering NAD(P)⁺ or a derivative, precursor or prodrug thereof to a subject in need thereof.
43. A method for inducing improvement of exercise capacity and/or endurance of a subject by artificial elevation of an NAD(P)⁺/NAD(P)H ratio *in vivo.*

## Claims

1. A method for elevating an NAD(P)⁺/NAD(P)H ratio *in vivo* except a human or *in vitro* by regulation or addition of oxidoreductase,
wherein the oxidoreductase is NAD(P)H:quinone oxidoreductase 1 (NQO1), wherein the NAD(P)+/NAD(P)H ratio is increased by enhancing the activity of NQO1, and
wherein the change in the NAD(P)+/NAD(P)H ratio by NQO1 is more than a 20% decrease of NAD(P)H, based on the amount of NAD(P)H in the absence of an activator for NQO1, thereby increasing the NAD(P)+/NAD(P)H ratio.

2. The method according to claim 1, wherein a decrease of NAD(P)H is more than 30%.

3. The method according to claim 1, wherein an AMP/ATP ratio is increased by elevation of the NAD(P)⁺/NAD(P)H ratio.

4. The method according to claim 1, wherein consumption of NAD(P)H as a coenzyme or substrate is increased by increasing the amount of an NQO1 protein or the expression of an NQO1 gene.

5. The method according to claim 1, wherein the activity of NQO1 is increased using a compound capable of increasing the activity or amount of NQO1.

6. The method according to claim 5, wherein the compound is an H-acceptor.

7. The method according to claim 6, wherein the compound is at least one selected from the group consisting of quinone-based compounds, quinone-imine-based compounds, nitro-based compounds, azo-based compounds, and any combination thereof.

8. The method according to claim 7, wherein the compound is at least one selected from the group consisting of naphthoquinone-based compounds and derivatives thereof.

9. The method according to claim 7, wherein the compound is at least one selected from the group consisting of fumaric acid esters and derivatives thereof, oltipraz (4-methyl-5(2-pyrazinyl)-1,2-dithiole-3-thione), curcumin, anethole dithiolethione, sulforaphane, 6-methylsulphinylhexyl isothiocyanate, caffeic acid phenethyl ester, 4'-bromoflavone, avicins, fisetin, resveratrol and any combination thereof.

10. The method according to claim 8, wherein the naphthoquinone-based compound is 4-aminoalkyl-1,2-naphthoquinone, 4-thioalkyl-1,2-naphthoquinone, 4-alkoxy-1,2-naphthoquinone, furano-o-naphthoquinone, pyrano-o-naphthoquinone or a derivative thereof.

11. The method according to claim 9, wherein the fumaric acid ester is dimethylfumarate, monoethylfumarate, monomethylfumarate or a salt thereof.

12. The method according to claim 8, wherein the naphthoquinone-based compound or derivative thereof is a compound represented by Formula I: 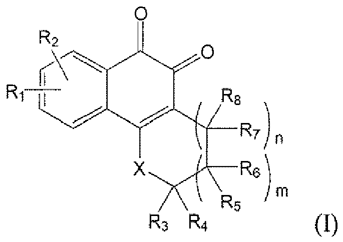 wherein
R₁ and R₂ are each independently hydrogen, halogen, alkoxy, hydroxy or lower alkyl having 1 to 6 carbon atoms;
R₃, R₄, R₅, R₆, R₇ and R₈ are each independently hydrogen, hydroxy, C₁-C₂₀ alkyl, alkene or alkoxy, cycloalkyl, heterocycloalkyl, aryl or heteroaryl, or two substituents of R₃ to R₈ may be taken together to form a cyclic structure;
X is oxygen, nitrogen or sulfur; and
m and n are each independently 0 or 1, with proviso that when either of in and n is 0, carbon atoms adjacent to m or n may form a cyclic structure via a direct bond.

13. The method according to claim 12, wherein X is oxygen, m is 1, and n is 0 or 1, with the proviso that when n is 0, carbon atoms adjacent to n form a cyclic structure via a direct bond.

14. The method according to claim 12, wherein the compound is represented by Formula II: 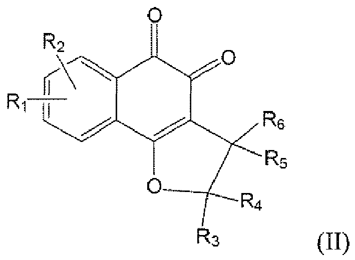 wherein R₁, R₂, R₃, R₄, R₅ and R₆ are the same as defined in Formula I.

15. The method according to claim 12, wherein the compound is represented by Formula III: 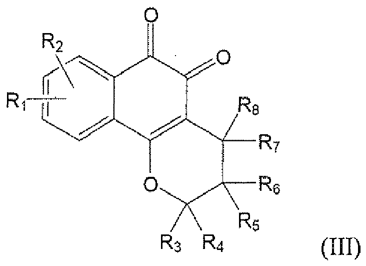 wherein R₁, R₂, R₃, R₄, R₅, R₆, R₇ and R₈ are the same as defined in Formula I.

16. The method according to claim 15, wherein the compound is 2,2-Dimethyl-3,4-dihydro-2*H*-benzo[*h*]chromene-5,6-dione.

17. A method for identifying a compound capable of elevating an NAD(P)⁺/NAD(P)H ratio *in vivo* except a human or *in vitro* via NQO1, comprising:
contacting a candidate compound group with NQO1; and
monitoring an amount or activity of NQO1.

18. The method according to claim 17, wherein the method includes reacting NQO1 with a compound to be screened and NAD(P)H for a predetermined time and quantifying the resulting NAD(P)⁺ or the remaining NAD(P)H.

19. The method according to claim 18, wherein the quantification of the produced NAD(P)⁺ includes measuring absorbance changes by inducement of color development due to changes in an absorption wavelength via reduction of DCPIP used as a hydride (H⁻) acceptor.

20. The method according to claim 18, wherein quantification of the remaining NAD(P)H includes measurement of absorbance changes due to color development of a tetrazolium salt.

21. The method according to claim 17, wherein the method includes reacting NQO1 with a compound to be screened for a predetermined time and quantifying a decrease of NAD(P)H.

22. The method according to claim 21, wherein the method includes reacting NQO1 with a compound to be screened for a predetermined time and quantifying a decrease of an intracellular ATP concentration or an increase of an intracellular AMP concentration.

23. The method according to claim 17, wherein the monitoring step includes observing an increase of an intracellular calcium concentration.

24. The method according to claim 17, wherein the monitoring step includes observing the degree of AMPK phosphorylation and activation.

25. The method according to claim 17, wherein the monitoring step includes observing an increase of ACC phosphorylation and/or a decrease of ACC activity.
